# EUROPEAN PATENT APPLICATION

(11) **EP 4 810 521 A1**
(43) Date of publication of application: **23.09.2026**
(21) Application number: 24890710.7
(22) Date of filing: 13.11.2024
(51) Int. Cl.: C07K 16/46, C07K 16/30, C07K 16/28, A61K 39/395, A61P 35/00

(54) **ANTI-EGFR AND -HER3 BISPECIFIC ANTIBODY-DRUG CONJUGATE AND USE THEREOF**

(30) Priority: 14.11.2023 CN 202311510502; 12.11.2024 CN 202411606421
(71) Applicant: Fortvita Biologics Inc., 1205 Grand Cayman (KY)
(72) Inventor: HE, Kaijie, Suzhou, Jiangsu 215123 (CN); CHEN, Shi, Suzhou, Jiangsu 215123 (CN); ZHANG, Xiao, Suzhou, Jiangsu 215123 (CN); ZHAO, Menghui, Suzhou, Jiangsu 215123 (CN)
(74) Representative: Sagittarius IP
(86) International application number: PCT/CN2024/131744
(87) International publication number: WO 2025/103355

(57) **Abstract**

The present invention relates to a bispecific antibody-drug conjugates targeting EGFR/HER3, a pharmaceutical compositions comprising the same, the use of the same for the treatment or prevention of diseases or conditions associated with EGFR and/or HER3 activity, in particular EGFR and/or HER3 positive tumors, and the use of the same for the manufacture of a medicament for the treatment or prevention of diseases or conditions associated with EGFR and/or HER3 activity, in particular EGFR and/or HER3 positive tumors.

## Description

### Technical Field

The present invention relates to an anti-EGFR/HER3 bispecific antibody-drug conjugate (ADC), a pharmaceutical compositions comprising the same, the use of the same for the treatment or prevention of diseases or conditions associated with EGFR and/or HER3 activity, in particular EGFR and/or HER3 positive tumors, and the use of the same for the manufacture of a medicament for the treatment or prevention of diseases or conditions associated with EGFR and/or HER3 activity, in particular EGFR and/or HER3 positive tumors.

### Background

The human epidermal growth factor receptor (EGFR, also known as ErbB1, HER1) family has four members EGFR, HER2, HER3 and HER4. Deregulation of each member by mutation, amplification and overexpression plays an important role in tumorigenesis and tumor metastasis. Overexpression has been associated with the development of a variety of tumors. Blocking EGFR signaling by blocking the EGFR binding site on the extracellular domain of the receptor or by inhibiting intracellular tyrosine kinase activity can prevent the growth of tumors expressing EGFR and improve the condition of the patient.

HER3 (encoded by ErbB3) is a member of the HER protein receptor family, and HER3 was initially thought to have no kinase activity. However, recent studies indicate that HER3 is capable of binding ATP and promoting the phosphorylation of intracellular domain, but with weaker kinase activity. It has been found HER3 plays an important role in heterodimerization of HER2/HER3 and subsequent activation of the PI3K/AKT cascade signaling, and thus extensive attention and research have been focused on HER3 in recent years. HER3 is overexpressed in a variety of cancers, such as gastric cancer, breast cancer, colorectal cancer and lung cancer, and HER3 expression correlates with poor survival after surgery in patients. The research shows that MET amplification may activate and induce EGFR targeted therapy resistance by maintaining PI3K/AKT signaling mediated by HER3; furthermore, HER3 not only enhances its expression in a variety of cancers, but also has potential functions in mediating drug resistance resulting from targeted therapies, HER3 is therefore a potential and attractive therapeutic target.

A bispecific antibody-drug conjugate ADC (BL-B01D1) targeting both EGFR and HER3 has emerged in the prior art that shows encouraging clinical efficacy against advanced solid tumors. However, the two arms anti-EGFR and anti-HER3 of the bispecific antibody-drug conjugate had very different activity, which is expected to result in a stronger endocytosis-killing activity on tumors with high EGFR expression, and a weaker endocytosis-killing activity on tumors with low EGFR expression but high HER3 expression. This deficiency limits its efficacy in patients harboring tumors with low EGFR expression and high HER3 expression, reduces the potential coverage population, and the advantages of bispecific antibody ADCs could not be fully exerted. In addition, the bispecific antibody-drug conjugate also has off-target toxicity, especially severe blood-related toxicity. The severe blood toxicity can bring pain to a subject patient, and simultaneously, it also limits the further increase of the clinical administration dosage of the bispecific antibody-drug conjugate, so that their efficacy cannot be further enhanced, and the advantages of targeting double antigens of EGFR and HER3 could not be fully reflected.

Therefore, there is still a need to develop more bispecific antibodies and bispecific antibody-drug conjugates targeting EGFR and HER3, which can overcome the above disadvantages and thereby allows for a better treatment or prevention of diseases or conditions associated with EGFR and/or HER3 activity, in particular EGFR and/or HER3 positive tumors or cancers.

The methods for conjugation of ADCs used in majority of the prior art are still random conjugation, including cysteine conjugation and lysine conjugation, etc., which results in a heterogeneity distribution of ADC products, e.g., an ADC with average DAR value of 4 obtained by cysteine conjugation will contain DAR0-DAR8 multiple components, even for the components with the same DAR value, there are still differences in their conjugation site. These components tend to have different properties, such as DAR0 components competitively bind to the target, DAR8 components tend to aggregate due to the large number of conjugated hydrophobic drug and thereby are more easily to be eliminated *in vivo*. The different pharmacodynamic and pharmacokinetic characteristics of these components make PK/PD analysis of heterogeneous ADC mixtures more difficult and higher manufacturing processes is required to produce relatively stable batches of ADC products.

Therefore, there is still a need for a novel antibody engineering technology, and the antibody obtained based on the technology can achieve better stability and stronger efficacy of ADC when used to construct an ADC.

### Disclosure of Invention

The present invention is based on the bispecific antibodies and the bispecific antibody-drug conjugates targeting EGFR and HER3 developed by the inventors, which exhibited a better stability and a stronger antitumor activity relative to the prior art.

In some embodiments, the invention particularly performed a cysteine modification on the constant region of bispecific antibodies targeting EGFR and HER3 , such that the antibody or the antigen binding fragment thereof mutated one or several native amino acids into cysteine on its heavy or light chain. The bispecific antibodies of the present invention comprise cysteine mutations at more cryptic sites of the constant region, thereby giving better stability and/or hydrophilicity to antibody-drug conjugates comprising the same.

Accordingly, the bispecific antibody-drug conjugate targeting EGFR and HER3 of the present invention is particularly suitable for the treatment or prevention of diseases or conditions associated with EGFR and/or HER3 activity, in particular EGFR and/or HER3 positive tumors.

In one aspect, the invention provides an anti-EGFR/HER3 bispecific antibody or an antigen-binding fragment thereof, comprising
a heavy chain variable region and a light chain variable region, the heavy chain variable region comprises HCDR1, HCDR2 and HCDR3 consisting of the amino acid sequences set forth in SEQ ID NO:1, SEQ ID NO:2 and SEQ ID NO:3 respectively, and the light chain variable region comprises LCDR1, LCDR2 and LCDR3 consisting of the amino acid sequences set forth in SEQ ID NO:4, SEQ ID NO:5 and SEQ ID NO:6respectively; and
a heavy chain constant region and a light chain constant region, wherein
the anti-EGFR/HER3 bispecific antibody comprises one or more mutations to cysteine, for example one or more mutations selected from: the mutation to cysteine at position 118 of the heavy chain constant region, the mutation to cysteine at position 239 of the heavy chain constant region, the mutation to cysteine at position 160 of the light chain constant region, and the mutation to cysteine at position 166 of the light chain constant region.

In another aspect, the present invention provides a bispecific antibody-drug conjugate of formula (I), a stereoisomer or pharmaceutically acceptable salt or solvate thereof:

Ab-(L-D)ₙ (I)

wherein,
Ab is an anti-EGFR/HER3 bispecific antibody or an antigen-binding fragment thereof as described in the present invention,
L is a linker,
D is a cytotoxic compound,
n represents a conjugation number, and n is a natural number selected from 1 to 15.

In a further aspect, the present invention provides a pharmaceutical composition comprising the bispecific antibody-drug conjugate, the stereoisomer or pharmaceutically acceptable salt or solvate thereof described in the present invention, and a pharmaceutically acceptable carrier or excipient.

In a further aspect, the invention provides a use of the bispecific antibody-drug conjugate, the stereoisomer or pharmaceutically acceptable salt or solvate thereof as described in the present invention in the manufacture of a medicament for the treatment or prevention of a disease or condition associated with EGFR and/or HER3 activity.

In a further aspect, the invention provides the bispecific antibody-drug conjugate, the stereoisomer or pharmaceutically acceptable salt or solvate thereof as described in the present invention, for use in therapy, e.g., for use in the treatment or prevention of a disease or condition associated with EGFR and/or HER3 activity.

In a further aspect, the invention provides a use of the bispecific antibody-drug conjugate, the stereoisomer or pharmaceutically acceptable salt or solvate thereof as described in the present invention for the treatment or prevention of a disease or condition associated with EGFR and/or HER3 activity.

In a further aspect, the invention provides a method of treating or preventing a disease or condition associated with EGFR and/or HER3 activity, the method comprising administering to a subject an effective amount of the bispecific antibody-drug conjugate, the stereoisomer or pharmaceutically acceptable salt or solvate thereof as described in the present invention.

Compared with the prior art, the bispecific antibody-drug conjugate targeting EGFR and HER3 provided by the invention has the following advantages:
(1) the bispecific antibody-drug conjugate has strong affinity to EGFR and HER3, exhibits endocytosis activity comparable to anti-EGFR and anti-HER3 monoclonal antibody respectively, such that the bispecific antibody ADC molecule exhibits strong endocytosis and killing activity against tumors with different EGFR and HER3 expression levels, overcomes the limitation in high heterogeneity tumor generally existed in the traditional ADC, expands the clinical application range of the EGFR/HER3 bispecific ADC molecule, and exhibits stronger efficacy in tumor patients with low EGFR expression and medium/high HER3 expression;
(2) the homogeneity of ADC molecules and the stability of the linker-payload can be significantly improved by adopting the site-specific conjugation for linker-payload conjugation method; meanwhile, as the number of the linker-payloads on the same molecule decreased, the hydrophobicity of the whole ADC molecule reduced significantly, the *in vivo* PK of the ADC molecule improved significantly, such that stronger efficacy was achieved; the improvement of the stability of the linker-payload brought by the site-specific conjugation will significantly reduce the non-specific release of the small molecular toxin outside the tumor, and the clinical safety of the bispecific ADC molecule is expected to be significantly improved, especially the toxicity related to free small molecules can be reduced, such as blood toxicity;
(3) the Fc moiety of the antibody molecule is designed with IgG1LALA, which destroys its binding capacity with FcYR, reduces the non-tumor-specific binding of ADC molecule, and the clinical safety of bispecific ADC molecule is expected to significantly improved, especially the blood toxicity may be reduced.

### Description of the Drawings

FIG. 1 shows the molecular structure of Duligo-LC1-NT3.
FIG. 2 shows the result of RP-HPLC analysis of Duligo-LC1-NT3.
FIG. 3 shows the results of SEC analysis of Duligo-LC1-NT3.
FIG. 4 shows the molecular structure of SIB001-DXd.
FIG. 5 shows the result of RP-HPLC analysis of SIB001-DXd.
FIG. 6 shows the result of SEC analysis of SIB001-DXd.
FIG. 7 shows the molecular structure of Patritumab-DXd.
FIG. 8 shows the result of RP-HPLC analysis of Patritumab-DXd.
FIG. 9 shows the result of SEC analysis of Patritumab-DXd.
FIG. 10 shows the molecular structure of Duoligo-DXd.
FIG. 11 shows the result of RP-HPLC analysis of Dulgio-DXd.
FIG. 12 shows the result of SEC analysis of Duoligo-DXd.
FIG. 13 shows the molecular structure of IgG1LALA-NT3 (DAR = 8).
FIG. 14 shows the result of RP-HPLC analysis of IgG1LALA-NT3 (DAR = 8).
FIG. 15 shows the result of SEC analysis of IgG1LALA-NT3 (DAR = 8).
FIG. 16 shows the molecular structure of IgG1LALA-NT3 (DAR = 4).
FIG. 17 shows the result of RP-HPLC analysis of IgG1LALA-NT3 (DAR = 4).
FIG. 18 shows the result of SEC analysis of IgG1LALA-NT3 (DAR = 4).
FIG. 19 shows the molecular structure of IgG1LALA-DXd.
FIG. 20 shows the result of RP-HPLC analysis of IgG1LALA-DXd.
FIG. 21 shows the result of SEC analysis of IgG1LALA-DXd.
FIG. 22 shows the stability of Duligo-LC1-NT3 in mouse and monkey plasma, analyzed by RP-HPLC method.
FIG. 23 shows the stability of Duligo-LC1-NT3 in mouse and monkey plasma, analyzed by LC-MS method.
FIG. 24 shows the detection of the expression level of EGFR and HER3 on different tumor cell lines.
FIG. 25 shows Fab-ZAP endocytosis-killing of Duligotuzumab, Cetuximab, Patritumab molecules on human pancreatic cancer cells AsPC1.
FIG. 26 shows Fab-ZAP endocytosis-killing of Duligotuzumab, Cetuximab, Patrituzumab molecules on human gastric cancer cells NUGC4.
FIG. 27 shows Fab-ZAP endocytosis-killing of Duligotuzumab, Cetuximab, Patrituzumab molecules on Chinese hamster ovary cells CHO-HER3.
FIG. 28 shows the *in vitro* killing of Duoligo-DXd and SIB001-DXd on human breast cancer cells MCF 7.
FIG. 29 shows the *in vitro* killing of Duoligo-DXd and SIB001-DXd on human breast cancer cells MDA-MB-453.
FIG. 30 shows the *in vitro* killing of Duligo-DXd and SIB001-DXd on human colorectal cancer cells GP2D.
FIG. 31 shows the *in vitro* killing of Duoligo-DXd and SIB001-DXd on human lung cancer cells H1568.
FIG. 32 shows the *in vitro* killing of Duoligo-DXd and SIB001-DXd on HER3 overexpressing human colorectal cancer cells SW 480.
FIG. 33 shows the *in vitro* killing of Duligo-LC1-NT3 and SIB001-DXd on human breast cancer cells MDA-MB-453.
FIG. 34 shows the *in vitro* killing of Duligo-LC1-NT3 and SIB001-DXd on human lung cancer cells HCC95.
FIG. 35 shows the *in vitro* killing of Duligo-LC1-NT3 and SIB001-DXd on human lung cancer cells H1568.
FIG. 36 shows the *in vitro* killing of Duligo-LC1-NT3 and SIB001-DXd on human colorectal cancer cells H508.
FIG. 37 shows the *in vitro* killing of Duligo-LC1-NT3 and SIB001-DXd on human pancreatic cancer cells AsPC1.
FIG. 38 shows the *in vitro* killing of Duligo-LC1-NT3 and SIB001-DXd on human colorectal cancer cells SW 480 overexpressing HER3.
FIG. 39 shows the *in vitro* killing of Duoligo-LC 1-NT3 and SIB001-DXd on human lung cancer cell H1703 overexpressing HER3.
FIG. 40 shows the affinity of Duligo-LC1-NT3 and SIB001-DXd for EGFR detected by ELISA.
FIG. 41 shows the affinity of Duligo-LC1-NT3 and SIB001-DXd for EGFR detected by ELISA.
FIG. 42 shows the anti-tumor efficacy of EGFR/HER3-ADCs molecules on a H508 tumor-bearing mouse model.
FIG. 43 shows the effect of EGFR/HER3-ADCs molecules on body weight changes in mouse.
FIG. 44 shows the anti-tumor efficacy of EGFR/HER3-ADCs molecules on a NUGC-4 tumor-bearing mouse model.
FIG. 45 shows the body weight change of EGFR/HER3-ADCs molecules on a NUGC-4 tumor-bearing mouse model.
FIG. 46 shows the anti-tumor efficacy of EGFR/HER3-ADCs molecules on a SW620 tumor-bearing mouse model.
FIG. 47 shows the body weight change of EGFR/HER3-ADCs molecules on a SW620 tumor-bearing mouse model.
FIG. 48 shows the anti-tumor efficacy of EGFR/HER3-ADCs molecules on a ASPC1 tumor-bearing mouse model.
FIG. 49 shows the body weight change of EGFR/HER3-ADCs molecules on a ASPC1 tumor-bearing mouse model.
FIG. 50 shows the anti-tumor efficacy of EGFR/HER3-ADCs molecules on a NCI-H1568 tumor-bearing mouse model.
FIG. 51 shows the body weight change of the EGFR/HER3-ADCs molecule on the NCI-H1568 tumor-bearing mouse model.

### Specific Implementation Options

Some of embodiments will now be described in detail, the examples of which are illustrated in the accompanying specific implementation options. While the enumerated embodiments will be described, it will be understood that they are not intended to limit the invention to these embodiments. On the contrary, the invention is intended to cover all alternatives, modifications and equivalents, which may be included within the scope of the present invention as defined by the claims. Those skilled in the art will recognize many methods and materials similar or equivalent to those described herein, which can be used in the practice of the present invention. The present invention is in no way limited to the methods and materials described. If one or more of the incorporated literature and similar materials differ from or contradict the present invention, including but not limited to defined terms, usage of terms, described techniques, etc., the present invention shall prevail.

It is appreciated that certain features of the present invention, for the sake of clarity, are described in the context of an individual embodiment, may also be provided in combination in individual embodiments. On the contrary, for the sake of brevity, various features of the present invention are described in the context of an individual embodiment, may also be provided separately or in any suitable sub-combination.

### I. Definition

Unless otherwise indicated, technical and scientific terms used herein have the same meaning as commonly understood by one of ordinary skill in the art to which this invention belongs.

The term "or" refers to a single element of the recited list of selectable elements unless otherwise the context clearly indicated. The term "and/or" means any one, any two, any three, any more, or all of the recited selectable elements.

As used herein, the term "about" means a value within the range of ± 10% or 5%, 4%, 3%, 2%, or 1% of a given numerical value.

As used herein, the terms "comprising", "including", "having", and similar expressions, mean that the elements not listed are not excluded. These terms also include the situation where only the listed elements are included.

As used herein, the term "HER3" (human epidermal growth factor receptor 3, also known as ErbB3) is a receptor protein tyrosine kinase and belongs to the Epidermal Growth Factor Receptor (EGFR) subfamily of receptor protein tyrosine kinases, which also includes HER1 (also known as EGFR), HER2 and HER4. HER3 is a transmembrane receptor and is composed of an extracellular ligand binding domain (ECD), a dimerization domain within the ECD, a transmembrane domain, and an intracellular protein Tyrosine Kinase Domain (TKD) and a C-terminal phosphorylation domain. HER3 overexpression has been found in several types of cancer (e.g., gastric cancer, breast cancer, colorectal cancer and lung cancer). A correlation between expression of HER2/HER3 and progression from non-invasive stage to invasive stage has been shown.

As used herein, the term "antibody" is used in the broadest sense, and includes immunoglobulins or other types of molecules that comprise one or more antigen binding domains that specifically bind to an antigen, as a protein or polypeptide that exhibits binding specificity for a particular antigen. Specific examples of antibodies may include intact antibodies (e.g., classical four-chain antibody molecules), single chain antibodies, single domain antibodies, multi-specific antibodies, etc. A classical antibody molecule is generally a tetramer consisting of 2 identical heavy chains and 2 identical light chains interconnected by disulfide bonds. The heavy chain and light chain are divided into variable regions (V) at the amino terminus and constant regions (C) at the carboxy terminus, based on conservation differences in amino acid sequence. The variable region is used to recognize and bind antigen, and the constant region (e.g., Fc fragment) is used to initiate downstream effects, such as antibody-dependent cell-mediated cytotoxicity effect (ADCC). Within the variable regions of the heavy and light chains, there are three regions with a higher degree of variation in the amino acid composition and sequence, which are critical positions for binding of the antibody to the antigen, and are therefore also referred to Complementarity Determining Regions (CDRs). Herein, the three heavy chain complementarity determining regions are referred to HCDR1, HCDR2 and HCDR3, respectively, and the three light chain complementarity determining regions are referred to LCDR1, LCDR2 and LCDR3, respectively. The amino acid sequences of the CDRs in an amino acid sequence of given light chain variable region or heavy chain variable region can be readily determined using numbering schemes well-known in the art, such as Kabat, Chothia, IMGT, AbM or Contact, for example, the amino acid sequences of each CDRs in an amino acid sequence of given light chain variable region or heavy chain variable region can be determined by any one of the above-mentioned numbering schemes, or each of them can be determined independently by any one of the above-mentioned numbering schemes, respectively. Unless otherwise indicated, in the present invention, the term "CDR" or "CDR sequence" encompasses CDR sequences determined in any of the ways described above.

Antibodies can be divided into five major different classes based on the amino acid sequence of the heavy chain constant region of the antibody: IgA, IgD, IgE, IgG and IgM. These antibody types can be further divided into subclasses according to the size of the hinge region, the position of interchain disulfide bonds and the difference in molecular weight, for example, IgG1, IgG2a, IgG2b, IgG3, etc. The light chain can be classified into two types, kappa and lambda, depending on the composition and arrangement of amino acids of the light chain constant region of the antibody. The subunit structures and three-dimensional conformations of different types of immunoglobulins are known in the art.

As used herein, when used to refer to amino acid positions in domains other than the variable regions of antibodies (e.g., constant regions, e.g., Fc regions), numbering is according to the EU numbering system (also known as the EU index) as described in Kabat et al, Sequences of Proteins of Immunological interfaces, 5 th edition, Public Health Service, National Institutes of Health, Bethesda, MD, 1991. When a position numbering and/or amino acid residues are assigned to a particular antibody isotype, it is intended to apply to the corresponding position and/or amino acid residue of any other antibody isotype, as is known to those skilled in the art.

General information on a light chain and a heavy chain of the human immunoglobulin is given in Kabat, E.A. et al, Sequences of Proteins of Immunological Interest, 5 th edition, Public Health Service, National Institutes of Health, Bethesda, Md. (1991).

The amino acid position to be mutated to cysteine is generally indicated by "chain type, mutation position". In this context, LLC represents a lambda light chain, LC represents a kappa light chain and HC represents a heavy chain, unless otherwise indicated. Thus, "LLC160" refers to the position 160 of the lambda light chain according to the EU numbering position, e.g., the amino acid at this position is mutated to cysteine. In the present invention, when referring to a heavy chain amino acid position, it refers to amino acid positions according to IgG1 heavy chain numbering, i.e. it encompasses amino acid positions numbering based on IgG1 heavy chain, as well as amino acid positions corresponding to the amino acid positions on other heavy chains, if not clearly indicated. For example, when referring to HC118, it refers to position 118 of the IgG1 heavy chain according to the EU numbering position, and also encompasses positions on IgG isotype other than IgG1 corresponding to amino acid 118 of the IgG1. When a combination of mutations is referred, the combined mutations are linked by a short dash (-). "LLC160-LLC166" indicates the positions 160 and 166 of the lambda light chain according to the EU numbering position, the amino acids in these two positions are mutated to cysteine simultaneously.

As used herein, an "antigen-binding fragment" of an antibody refers to an amino acid fragment of an antibody molecule that involves in antigen-specific binding, e.g., Fab, Fab', and F(ab')₂, and the like. It is known to those skilled in the art how to obtain such antigen-binding fragments. For example, a classical antibody molecule can be digested with papain to yield Fab fragments, can be digested with pepsin to yield F(ab')₂, and can be treated with a reducing agent to break the disulfide bonds between the F(ab')₂ hinge regions and to yield Fab' fragments.

As used herein, the term "single chain fragment variable (scFv)" is composed of an antibody heavy chain variable region and a light chain variable region linked together via a short peptide into a single peptide chain. By proper folding, the variable regions from the heavy chain and light chain form a Fv fragment through a non-covalent bond interaction, such that the scFv is able to retain its affinity activity for the antigen better.

As used herein, the term "single domain antibody (sdAb)", or also referred to "VHH antibody", refers to an antibody molecule having antigen binding capability, which comprises a heavy chain variable region without a light chain. Structurally, single domain antibodies can also be considered as a fragment of classical four-chain antibody molecules. Single domain antibodies were first discovered in camelids, subsequently, more single domain antibodies with antigen binding capability have been discovered by researchers through antibody libraries (e.g., phage display libraries) screening. Single domain antibodies have several advantages over common antibody molecules (e.g., classical antibody molecules), for example, including but are not limited to: smaller molecular weight, so that when used in a human body, a single domain antibody can easily reach tissues or parts which are difficult for common antibody molecules, or can contact with antigen epitopes in the protein or polypeptide which are difficult for common antibody molecules; better stability, and resistance to, for example, variations in temperature and pH and the action of denaturants and proteases.

As used herein, the term "Fc fragment" refers to the stalk region of a Y-shaped classical antibody molecule, i.e. the fragment crystallizable (Fc), comprising the second constant domain and third constant domain of the heavy chain (CH2 and CH3 domains). The antibody Fc region can be obtained by hydrolyzing the antibody molecule with a proteolytic enzyme, such as papain. In some examples, the Fc region may comprise a hinge, CH2, and CH3. When the Fc region contains a hinge, it can mediate dimerization between two Fc-containing polypeptides. The Fc fragment can be derived from IgG, IgM, IgD, IgE or IgA. In some examples, the Fc region is derived from IgG1, IgG2, IgG3, or IgG4. "Fc fragment" also includes variant Fc fragments derived from a native Fc fragment, which have been modified but still retain their effector functions. A "variant Fc fragment" comprises an amino acid sequence having at least one amino acid variation in the amino acid sequence of the native Fc fragment. In some examples, the variant Fc-fragment has at least one amino acid substitutions compared to the parent Fc-fragment (native Fc-fragment), e.g., about 1 to about 10 amino acid substitutions, and preferably about 1 to about 5 amino acid substitutions presented in the parent Fc-fragment. In some examples, the variant Fc fragment Fc region has at least about 80% sequence identity, at least about 90% sequence identity, at least about 95%, at least about 96%, at least about 97%, at least about 98%, or at least about 99% sequence identity to the parent Fc fragment. Effect functions of the "Fc fragment" may include binding to Fc receptors, Clq binding and Complement Dependent Cytotoxicity (CDC), antibody dependent cell mediated cytotoxicity (ADCC), mediated phagocytosis, etc.

As used herein, the term "murine antibody" refers to an antibody in which the variable region and constant region (if any) are derived from the immunoglobulin sequences of mouse or rat. The murine antibody can be conveniently obtained by immunizing a mouse or rat with the corresponding antigen and isolating the objective antibody therefrom. Alternatively, it can be obtained by isolating and culturing cells (e.g., B cells) expressing the objective antibody after immunizing a mouse or rat with the corresponding antigen. Alternatively, after immunizing a mouse or rat with the corresponding antigen, cells expressing the objective antibody are isolated and cultured, and fused with immortalized cells such as myeloma cells to obtain hybridoma cells, culturing hybridoma cells allows the production of the objective antibody (e.g., monoclonal antibody) in a large amount over a long period of time. In some embodiments, the "murine antibody" is a mouse antibody. A "humanized antibody" refers to a chimeric antibody obtained by artificially modifying a non-human antibody, i.e., an antibody in which the variable region and constant region, if any, are not derived from a human immunoglobulin, so that it contains the amino acid sequence of a human antibody. The humanized antibody may comprise a constant region and/or a framework region of a human antibody. The humanized antibody can be obtained by genetic engineering means, for example, by replacing the constant region of a murine antibody with the constant region of a human antibody and/or replacing the framework region of a murine antibody with the framework region of a human antibody. Generally, such humanization modification does not affect the binding specificity of the original antibody to the corresponding antigen, and thus such antigens are also included within the scope of the present invention.

As used herein, the term "monoclonal antibody" refers to a homogeneous antibody that only targets to a specific antigenic epitope. Compared to a polyclonal antibody, which typically include different antibodies that target to different antigenic determinants (epitopes), each monoclonal antibody target to a single antigenic determinant on the antigen. The modifier "monoclonal" indicates a homogeneous characteristic of an antibody and is not interpreted as an antibody requiring to be produced by any particular method. The monoclonal antibody of the present invention may be produced by hybridoma methods or recombinant DNA methods well known in the art, or obtained by screening methods described elsewhere herein.

As used herein, the term "purification tag" refers to an amino acid sequence that facilitates isolation of the objective polypeptide or protein from a cell culture or a supernatant expressing the objective polypeptide or protein. Examples include, but are not limited to, His6 tag, Flag tag, MBP tag, GST tag, SUMO tag, etc.

As for an antibody or antigen-binding fragment thereof, "bind", "target" or "specifically bind" refers to one molecule (e.g., an antibody or an antigen-binding fragment thereof) has a higher binding affinity for another molecule (e.g., an antigen) over the other molecules that are also present in the environment. One molecule may bind, target, or specifically bind to more than one molecule, e.g., a bispecific antibody may have a higher binding affinity for two different antigens over the other molecule. The binding affinity of an antibody to an antigen can be measured by measuring some parameter, such as the EC₅₀ value or KD value of the binding of an antibody and an antigen.

The EC₅₀ (concentration for 50% of maximum effect) refers to the concentration that causes 50% of the maximum effect. When used in an enzyme-linked immunosorbent assay (ELISA) to indicate the binding capacity of an antibody molecule to the corresponding antigen, it may refer to the concentration of the antibody molecule at half of the maximum detected signal (e.g., colorimetric or fluorescent intensity) is produced. The lower the EC₅₀ value, the greater the binding affinity to the antigen.

The KD value can also be used to measure the binding affinity between an antibody and its antigen. The KD value is the equilibrium dissociation constant between an antibody and its antigen, i.e. the ratio of koff/kon. Thus, the lower the KD value (the lower the concentration), the higher the affinity of the antibody.

As used herein, the terms "polypeptide" and "protein" are used interchangeably and refer to a polymer of amino acid residues. Such polymers of amino acid residues may contain natural or unnatural amino acid residues, and include but are not limited to, peptides, oligopeptides, dimers, trimers and multimers composed of amino acid residues. Both full-length proteins and fragments thereof are encompassed by this definition. The term also includes post-expression modifications of the polypeptide, e.g., glycosylation, sialylation, acetylation, phosphorylation, etc. Furthermore, for the purposes of the present invention, "polypeptide" refers to a protein that comprises modifications to the native sequence, such as deletions, additions, and substitutions (which are generally conservative in nature), so long as the protein retains the desired activity. These modifications may be purposeful, such as induced by site-specific mutations; or may be accidental, such as through mutation in the host that produces the protein or error due to PCR amplification.

As used herein, the term "functional variant" refers to a variant molecule obtained after the introduction of one or more amino acid insertions, deletions or substitutions on the parent protein molecule (e.g., a native protein molecule), which still retains at least part of the function of the parent protein molecule (particularly an objective function, e.g., the ability to bind to a corresponding antigen). For example, the functional variant of an antibody molecule may retain at least 20%, 30%, 40%, 50%, 60%, 70%, 80%, 90% of the antigen-binding capacity of its parent molecule, or even have a higher binding capacity than the parent molecule. In some embodiments, the functional variant of an antibody molecule may retain at least 80%, 85%, 90%, 95%, or even 100% or more of the antigen-binding affinity of its parent antibody molecule. For an antibody molecule or an antigen-binding fragment thereof, the functional variant typically contains amino acid alterations in the variable region framework sequences and/or constant regions, but do not exclude one or a few amino acid alterations in the CDR region sequences.

As used herein, the terms "nucleic acid molecule", "nucleic acid" and "polynucleotide" are used interchangeably, and refer to a polymer of nucleotides. Such polymers of nucleotide may contain natural and/or non-natural nucleotides and include, but are not limited to, DNA, RNA, and PNA. "Nucleic acid sequence" refers to the linear sequence of nucleotides contained in the nucleic acid molecule or polynucleotide.

As used herein, the term "vector" refers to a nucleic acid molecule that can be engineered to contain an objective polynucleotide (e.g., a encoding sequence for an objective polypeptide) or a nucleic acid molecule (e.g., a nucleic acid, plasmid, or virus, etc.) can replicate in a host cell. A vector may comprise one or more of the following components: an origin of replication, one or more regulatory sequences (such as a promoter and/or enhancer) that regulate the expression of the polynucleotide of interest, and/or one or more selectable marker genes (such as antibiotic resistance genes and genes useful in colorimetric assays, e.g., β-galactose). The term "expression vector" refers to a vector that is used to express a polypeptide of interest in a host cell.

As used herein, the term "host cell" refers to a cell that can be or has been a recipient of a vector or isolated polynucleotide. The host cell can be a prokaryotic cell or a eukaryotic cell. Exemplary eukaryotic cells include mammalian cells, such as primate or non-primate cells; fungal cells, such as yeast; plant cells; and insect cells. Non-limiting exemplary mammalian cells include, but are not limited to, CHO cells, HEK-293 cells, BHK cells or PER-C6 cells, as well as derived cells thereof, such as 293-6E, CHO-DG44, CHO-K1, CHO-S and CHO-DS cells. Host cells include progeny of a single host cell, and the progeny may not necessarily be completely identical (in morphology or in genomic DNA complementation) to the original parent cell, due to natural, accidental, or deliberate mutation. Host cells can be isolated cells or cell lines, and may also be cells transfected in vivo with a nucleic acid molecules or expression vector provided herein.

When referring to an amino acid or nucleotide sequence, the term "sequence identity" (also known as "sequence consistency") refers to the amount of identity degree between two amino acids or two nucleotide sequences (e.g., a query sequence and a reference sequence), which is typically expressed as a percentage. Typically, prior to calculating the percent identity between two amino acids or nucleotide sequences, the sequence alignment is performed at first and a gap is introduced (gaps), if any. If the amino acid residues or bases in these two sequences are the same at an aligned position, these two sequences are considered to be identical or matched at the position; if the amino acid residues or bases in the two sequences are different, the positions are considered to be inconsistent or mismatched. In some algorithms, the sequence identity is obtained through dividing the number of matched positions by the total number of positions in the alignment window. In other algorithms, the number of gaps and/or the length of the gaps are also taken into account. Commonly used sequence alignment algorithms or software include DANMAN, CLUSTALW, MAFFT, BLAST, MUSCLE, etc. For the purposes of the present invention, the published alignment software BLAST (available from https://www.ncbi.nlm.nih.gov/) can be used to obtain optimal sequence alignments and calculate sequence identity between two amino acid sequences or nucleotide sequences by using the default settings.

As used herein, the term "aliphatic hydrocarbyl" refers to a substituted or unsubstituted straight, branched and/or cyclic, saturated or unsaturated group consisting of only carbon atom and hydrogen atom and attaches to the remainder of the molecule by a single bond, which includes alkyl, alkenyl and alkynyl that are straight, branched, cyclic or a combination thereof. In some embodiments, the term "aliphatic hydrocarbyl" and "aliphatic hydrocarbyl group" may be used interchangeably. In some embodiments, the aliphatic hydrocarbyl comprises one or more, e.g., 1, 2, 3, 4, 5, 6, 7, 8, 9, 10, 11, or 12 unsaturated carbon-carbon double bond (-C=C-), carbon-carbon triple bond (-C≡C-) groups, and/or any combination thereof. Alkyl, alkenyl, alkynyl, cycloalkyl, cycloalkenyl and cycloalkynyl are as defined below. In the present invention, aliphatic hydrocarbyl refers to alkyl, alkenyl, alkynyl, cycloalkyl and/or cycloalkenyl, preferably alkyl and/or cycloalkyl. The hydrogens on the aliphatic hydrocarbyls may be optionally substituted with any suitable group, e.g., halogen, hydroxy, amino, mono-substituted amino, di-substituted amino, alkoxy, heterocyclyl, etc. The term "aliphatic hydrocarbylene" refers to a divalent substituted aliphatic hydrocarbyl formed by substitution of one hydrogen atoms of the aliphatic hydrocarbyl by a bond, and the terms "alkylene," "alkenylene," "alkynylene," "cycloalkylene," "cycloalkenylene," and "cycloalkynylene" have similar meanings.

As used herein, the term "alkyl", as a separate group or a part of another group, refers to a straight or branched group consisting of only carbon atom and hydrogen atom, which contains no unsaturation bond, and attaches to the remainder of the molecule by a single bond. The alkyl may comprise, for example, 1 to 18, preferably 1 to 12, more preferably 1 to 8 carbon atoms. Examples of alkyl include, but are not limited to, methyl, ethyl, propyl, isopropyl, n-butyl, isobutyl, tert-butyl, n-pentyl, 2-pentyl, hexyl, n-hexyl, heptyl, 2-methylhexyl, 3-methylhexyl, octyl, nonyl, and decyl, etc., preferably methyl, ethyl, propyl, isopropyl, n-butyl, and more preferably methyl, ethyl, propyl, and isopropyl. The hydrogens on the alkyl may be optionally substituted with any suitable group, e.g., halogen, hydroxy, amino, mono-substituted amino, di-substituted amino, alkoxy, heterocyclyl, etc.

As used herein, the term "alkenyl" as a separate group or a part of another group, refers to a straight or branched hydrocarbon group consisting of only carbon and hydrogen atoms, which contains at least one double bond, and comprises, for example, from 2 to 18, preferably from 2 to 10, more preferably from 2 to 8 carbon atoms, and attaches to the remainder of the molecule by a single bond, includes but is not limited to vinyl, propenyl, allyl, but-1-enyl, but-2-enyl, pent-1-enyl, pent-2-enyl, pent-1,4-dienyl, etc., preferably vinyl, propenyl. The hydrogens on the alkenyl may be optionally substituted with any suitable group, e.g., halogen, hydroxy, amino, mono-substituted amino, di-substituted amino, alkoxy, heterocyclyl, etc.

As used herein, the term "alkynyl" as a separate group or a part of another group, refers to a straight or branched hydrocarbon chain group consisting of only carbon and hydrogen atoms, which contains at least one triple bond and optionally one or more double bonds, and comprises, for example, from 2 to 18, preferably from 2 to 10, more preferably from 2 to 8 carbon atoms, and attaches to the remainder of the molecule by a single bond. Examples of alkynyl include, but are not limited to, ethynyl, prop-1-ynyl, pent-1-en-4-ynyl, etc. The hydrogens on the alkynyl may be optionally substituted with any suitable group, e.g., halogen, hydroxy, amino, alkoxy, heterocyclyl, etc.

As used herein, the term "cycloalkyl" as a separate group or a part of another group, refers to a stable saturated non-aromatic monocyclic or polycyclic hydrocarbyl consisting of only carbon and hydrogen atoms, which may include fused or bridged ring systems, and comprises, for example, 3 to 15, preferably 3 to 10, more preferably 3 to 8 carbon atoms, e.g., 3 to 6 or 5 to 6 carbon atoms, and attaches to the remainder of the molecule by a single bond via any suitable carbon atom on the ring. Cycloalkyl includes, but is not limited to, cyclopropyl, cyclobutyl, cyclopentyl, cyclohexyl, cycloheptyl, cyclooctyl, bicyclo[2.2.1]heptyl, 7,7-dimethyl-bicyclo[2.2.1]heptyl, bicyclo[2.2.2]octyl, bicyclo[3.1.1]heptyl, bicyclo[3.2.1]octyl, and adamantyl, etc., preferably cyclobutyl, cyclopentyl, cyclohexyl. The hydrogens on the cycloalkyl may be optionally substituted with any suitable group, e.g., halogen, hydroxy, amino, mono-substituted amino, di-substituted amino, alkyl, alkoxy, heterocyclyl, etc.

As used herein, the term "cycloalkenyl" as a separate group or a part of another group, refers to a stable non-aromatic monocyclic or polycyclic hydrocarbyl consisting of only carbon atoms and hydrogen atoms, which contains at least one double bond, which may include fused or bridged ring systems. The cycloalkenyl comprises, for example, from 3 to 15, preferably from 3 to 10, more preferably from 3 to 8 carbon atoms, e.g., from 3 to 6 or from 5 to 6 carbon atoms, and attaches to the remainder of the molecule by a single bond via any suitable carbon atom in the ring. Examples of cycloalkenyl include, but are not limited to, cyclobutenyl, cyclopentenyl, cyclohexenyl, 1,3-cyclohexadiene, 1,4-cyclohexadiene, 1H-indenyl, 2,3-indanyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5-hydro-benzocycloheptenyl, 5,6,7,8,9,10-hexahydro-benzocyclooctenyl, fluorenyl, bicyclo[2.2.1]heptenyl, bicyclo[2.2.2]octyl, bicyclo[2.2.2]octenyl, bicyclo[3.2.1]octenyl, octahydro-4,7-methylene-1-hydro-indenyl, and octahydro-2,5-methylene-pentalenyl, etc. The hydrogens on the cycloalkenyl may be optionally substituted with any suitable group, e.g., halogen, hydroxy, amino, mono-substituted amino, di-substituted amino, alkyl, alkoxy, heterocyclyl, etc.

As used herein, the term "cycloalkynyl" as a separate group or a part of another group, refers to a stable non-aromatic monocyclic or polycyclic hydrocarbyl consisting of only carbon and hydrogen atoms containing at least one triple bond, which may include fused or bridged ring systems. the cycloalkynyl comprises, for example, from 3 to 15, preferably from 3 to 10, more preferably from 3 to 8 carbon atoms, e.g., from 3 to 6 or from 5 to 6 carbon atoms, and attaches to the remainder of the molecule by a single bond via any suitable carbon atom on the ring. Examples of cycloalkynyl include, but are not limited to, cyclobutynyl, cyclopentynyl, cyclohexynyl, etc. The hydrogens on the cycloalkynyl may be optionally substituted with any suitable group, e.g., halogen, hydroxy, amino, mono-substituted amino, di-substituted amino, alkyl, alkoxy, heterocyclyl, etc.

As used herein, the term "aliphatic heterohydrocarbyl" refers to a substituted or unsubstituted straight, branched, and/or cyclic, saturated or unsaturated hydrocarbon group containing a heteroatom selected from N, O and S, which includes alkyl, alkenyl and alkynyl that are straight, branched, cyclic, or a combination thereof. In some embodiments, the term "aliphatic heterohydrocarbyl" and "aliphatic heterohydrocarbyl group"may be used interchangeably. In some embodiments, the heteroatoms included in the aliphatic heterohydrocarbyls may, together with carbon atoms, form the backbone of the aliphatic heterohydrocarbyl groups, for example, but are not limited to, the group structures such as -C-N-C-, -C-O-C, -C-O-O-C, -C-S-C-, -C-S-S-C, or any combination thereof. In some embodiments, the aliphatic heterohydrocarbyl contained in a aliphatic heterohydrocarbyl may be a substituent attached to a carbon atom, for example, but not limited to, a substituted structure such as -C≡N, -C=N-, -C-N=, - C=O, -C-OH, -C=S, -C-SH. In some said embodiments, the heteroatom contained in the aliphatic heterohydrocarbyl may be any combination of the group structures listed above. In some embodiments, the aliphatic heterohydrocarbyl group comprises one or more, e.g., 1, 2, 3, 4, 5, 6, 7, 8, 9, 10, 11, or 12 unsaturated carbon-carbon double bonds (-C=C-), carbon-carbon triple bonds (-C≡C-), , -NH-, - NH₂-, OH, -OR^{m}, -O-, -C(O)-, -C(ORⁿ)-, -C(O)O-, -SH, -SR^{o}, -S-, -C(S)-, -C(SR^{p})-, -C(S)O-, -P(O)-, and/or any combination thereof, wherein R^{m}, Rⁿ, R^{o}, and R^{p} are each independently substituted or unsubstituted C₁-C₁₄ aliphatic hydrocarbyl, e.g., C₁-C₁₂, C₁-C₁₀, C₁-C₈, C₁-C₆, C₁-C₄ aliphatic hydrocarbyl, non-limiting specific examples of substituted or unsubstituted C₁-C₁₄ aliphatic hydrocarbyl include, but are not limited to, methyl, ethyl, n-propyl and isopropyl, n-butyl, sec-butyl, isobutyl, tert-butyl, neopentyl, cyclopropyl, cyclobutyl, cyclopentyl, cyclohexyl, ethylene (vinyl), propenyl, butenyl, pentenyl, 1-methyl-2-buten-1-yl, 5-hexenyl, ethynyl, 1-propynyl, 2-propynyl, etc.

As used herein, the term "aryl" or "aromatic ring" refers to a monocyclic, bicyclic, or polycyclic carbocyclic ring system having at least one aromatic ring. Unless otherwise indicated, aryl may be 6- to 10- membered. In certain embodiments, aryl group may contain 6 ring-forming carbon atoms. All atoms within a carbocyclic aryl group are carbon atoms. Non-limiting examples of aryl groups include phenyl, naphthyl, 1,2-dihydronaphthyl, 1,2,3,4-tetrahydronaphthyl, fluorenyl, indanyl, indenyl, etc. In the context of the present invention, the terms "aryl" and "aromatic ring" are used interchangeably.

As used herein, the term "aliphatic hydrocarbyloxy" refers to the group -O-aliphatic hydrocarbyl, wherein the aliphatic hydrocarbyl has the meaning as defined herein.

As used herein, the term "aliphatic hydrocarbylthio" refers to the group -S-aliphatic hydrocarbyl, wherein the aliphatic hydrocarbyl has the meaning as defined herein.

As used herein, the term "heteroaryl" or "heteroaromatic ring" refers to a monocyclic ring system, or a fused or bridged bicyclic ring system, wherein the ring system contains one, two, three, or four heteroatoms independently selected from the group consisting of nitrogen, oxygen, and sulfur; and at least one ring is aromatic ring. Unless otherwise indicated, heteroaryl may be 5- to 10- membered. In certain embodiments, heteroaryl may be 5- or 6- membered. In certain embodiments, the heteroaryl group may contain one, two or three heteroatoms. In certain embodiments, the heteroaryl group may contain one or two heteroatoms. Non-limiting examples of heteroaryl groups include benzimidazolyl, benzofuranyl, benzothiazolyl, benzothienyl, benzoxazolyl, furanyl, imidazolyl, indolyl, isoindazolyl, isoquinolyl, isothiazolyl, isothiazoleyl, isoxazolyl, oxadiazolyl, oxazolyl, purinyl, pyrrolyl, pyridyl, pyrazinyl, pyrimidinyl, quinolinyl, quinolineyl, thiadiazolyl, thiazolyl, thienyl, triazolyl, tetrazolyl, dihydroquinolinyl, tetrahydroquinolinyl, tetrahydroisoquinolinyl, etc. Heteroaryl groups include at least one ring having at least one heteroatom as described above and at least one aromatic ring. For example, a ring having at least one heteroatom may be fused to one, two or three carbocyclic rings, e.g., an aryl ring, a cyclohexane ring, a cyclohexene ring, a cyclopentane ring, a cyclopentene ring or another monocyclic heterocyclic ring. Non-limiting examples of fused heteroaryl groups include 1,2,3,5,8,8a-hexahydroindolizine, 2,3-dihydrobenzofuran, 2,3-dihydroquinolinyl, 2,3-dihydrobenzothiophene, etc. In the context of the present invention, the terms "heteroaryl" and "heteroaromatic ring" may be used interchangeably.

As used herein, the term "heteroatom" refers to nitrogen (N), oxygen (O), and sulfur (S), and may include any oxidized form of nitrogen and sulfur, as well as any quaternized form of basic nitrogen, unless otherwise indicated.

As used herein, the terms "cancer" and "cancerous" refer to or is used to describe the physiological condition in mammals, in which a population of cells is characterized by unregulated cell growth. As used herein, the terms "cancer cells" and "tumor cells" refer to the total cell number of a tumor, including tumorigenic stem cells (cancer stem cells) that make up the majority of the tumor cell population and non-tumorigenic cells. Examples of cancers include, but are not limited to: carcinoma, lymphoma, blastoma, sarcoma, and leukemia. More specific examples of such cancers include melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer.

As used herein, the term "tumor" refers to any tissue mass caused by excessive growth or proliferation of cells, whether benign (non-cancerous) or malignant (cancerous), including precancerous lesions.

The terms "cancer" and "tumor" are used interchangeably herein and include both solid tumors and hematological tumors.

As used herein, the term "metastasis" refers to the process by which a cancer spreads or metastasizes from its origin site to other areas of the body as similar cancerous lesions occur in new locations. "Metastatic" cells are cells that lose adhesive contact with neighboring cells and migrate from the primary site of disease to invade neighboring body structures through the blood or lymph.

As used herein, the term "subject" refers to any animal (e.g., a mammal), including but is not limited to humans, non-human primates, rodents, etc., that will be the recipient of a particular treatment. Generally, the terms "subject" and "patient" are used interchangeably herein to refer to a human subject.

As used herein, the terms "treating", "treatment", "preventing" and "prevention" refer to: 1) cure, slow, alleviate symptoms and/or stop the progression of the diagnosed pathological condition or disease; 2) a pre-implemented method of preventing, delaying or slowing the progression of a targeted pathological condition or disease. Thus, subjects in need of the treatment include those suffering from the disease; subjects susceptible to such a disease; and subjects in need of the prevention. A subject is successfully "treated" according to the methods of the present invention if the patient exhibits one or more of the following: reduction or absence of cancer cells; reduction in tumor size; inhibition or absence of cancer cell infiltration into peripheral organs (these include cancer cell spread to soft tissue and bone); inhibition or absence of tumor metastasis; inhibition or absence of tumor growth; alleviation of one or more symptoms associated with a particular cancer; reduction of morbidity and mortality; and improvement in quality of life. In the context for cancer, the term "prevention" refers to administering a medical means, such as a drug, to a subject, particularly a subject at risk of cancer, prior to the occurrence of a symptom or pathological condition associated with the cancer.

### II. Antibodies

The invention provides an anti-EGFR/HER3 bispecific antibody or an antigen-binding fragment thereof, comprising
a heavy chain variable region and a light chain variable region, the heavy chain variable region comprises HCDR1, HCDR2 and HCDR3 consisting of the amino acid sequences set forth in SEQ ID NO:1, SEQ ID NO:2 and SEQ ID NO:3 respectively, and the light chain variable region comprises LCDR1, LCDR2 and LCDR3 consisting of the amino acid sequences set forth in SEQ ID NO:4, SEQ ID NO:5 and SEQ ID NO:6 respectively,
the amino acid sequence of HCDR1 is GFTLSGDWIH (SEQ ID NO: 1),
the amino acid sequence of HCDR2 is EISAAGGYTDYADSVKG (SEQ ID NO: 2),
the amino acid sequence of HCDR3 is ESRVSFEAAMDY (SEQ ID NO: 3),
the amino acid sequence of LCDR1 is RASQNIATDVA (SEQ ID NO: 4),
the amino acid sequence of LCDR2 is SASFLYS (SEQ ID NO: 5),
the amino acid sequence of LCDR3 is QQSEPEPYT (SEQ ID NO: 6),
preferably, HCDR1 is defined by the AbM numbering system; HCDR2, HCDR3, LCDR1, LCDR2 and LCDR3 are defined by the Kabat numbering system; and
a heavy chain constant region and a light chain constant region, wherein
the anti-EGFR/HER3 bispecific antibody comprises one or more, e.g. 1,2,3,4 or more mutations to cysteine, e.g. one or more, e.g. 1,2,3,4 or more mutations selected from: the mutation to cysteine at position 118 of the heavy chain constant region, the mutation to cysteine at position 239 of the heavy chain constant region, the mutation to cysteine at position 160 of the light chain constant region, and the mutation to cysteine at position 166 of the light chain constant region.

In some embodiments, in the bispecific antibodies of the present invention, the heavy chain variable region (V_{H})
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:7; or
(ii) comprises or consists of an amino acid sequence set forth in SEQ ID NO:7; or
(iii) comprises or consists of an amino acid sequence having one or more amino acid substitutions, insertions or deletions compared to the amino acid sequence set forth in SEQ ID NO:7, optionally the amino acid substitutions, insertions or deletions do not occur in the CDRs, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids.
wherein the sequence set forth in SEQ ID NO:7 is as follows:



(SEQ ID NO: 7).

The nucleic acid sequence encoding this amino acid sequence is as follows:

GAAGTGCAACTCGTCGAGAGCGGCGGGGGCCTGGTGCAGCCCGGCGGCTCCCTGAGA
CTGAGCTGCGCCGCTAGCGGCTTCACCCTGAGCGGCGACTGGATCCACTGGGTGCGGCAA
GCCCCTGGCAAGGGCCTCGAGTGGGTCGGGGAAATTAGCCCGCTGGCGGCTACACCGAC
TACGCCGACAGCGTGAAGGGCAGATTCACCATCAGCGCCGACACAAGCAAGAACACCGCC
TACCTGCAGATGAACAGCCTGAGAGCCGAGGACACCGCCGTGTACTACTGCGCTAGAGAG
AGCAGAGTGAGCTTCGAGGCCGCCATGGACTACTGGGGCCAAGGCACCCTGGTGACCGTG
AGCAGC (SEQ ID NO: 8).

In some embodiments, in the bispecific antibodies of the present invention, the heavy chain variable region (V_{H})
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence encoded by the nucleic acid sequence set forth in SEQ ID NO:8; or
(ii) comprises or consists of an amino acid sequence encoded by the nucleic acid sequence set forth in SEQ ID NO:8; or
(iii) comprises or consists of an amino acid sequence having one or more amino acid substitutions, insertions or deletions compared to the amino acid sequence encoded by the nucleic acid sequence set forth in SEQ ID NO:8, optionally the amino acid substitutions, insertions or deletions do not occur in the CDRs, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids.

In some embodiments, in the bispecific antibodies of the present invention, the light chain variable region (V_{L})
(i) comprises or consists of an amino acid sequence having at least 90%, 91%, 92%, 93%, 94%, 95%, 96%, 97%, 98% or 99% identity to the amino acid sequence set forth in SEQ ID NO: 9; or
(ii) comprises or consists of an amino acid sequence set forth in SEQ ID NO: 9; or
(iii) comprises or consists of an amino acid sequence having one or more amino acid substitutions, insertions or deletions compared to the amino acid sequence set forth in SEQ ID NO: 9, optionally the amino acid substitutions, insertions or deletions do not occur in the CDRs, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids,
wherein the amino acid sequence set forth in SEQ ID NO: 9 is as follows:


 (SEQ ID NO: 9).

The nucleic acid sequence encoding this amino acid sequence is as follows:

GACATTCAGATGACACAGAGCCCTAGCAGCCTGAGCGCTAGCGTGGGCGACAGAGTG
ACCATCACCTGCAGAGCTAGCCAAAACATCGCCACCGACGTGGCCTGGTATCAGCAGAAGC
CCGGCAAGGCCCCCAAGCTGCTGATCTACAGCGCTAGCTTCCTGTACAGCGGCGTGCCTAG
CAGATTCAGCGGCAGCGGCAGCGGCACCGACTTCACCCTGACCATCAGCAGCCTGCAGCC
CGAGGACTTCGCCACCTACTACTGTCAGCAGAGCGAGCCCGAGCCCTACACCTTCGGCCAA
GGCACCAAGGTGGAGATCAAG (SEQ ID NO: 10).

In some embodiments, in the bispecific antibodies of the present invention, the light chain variable region (V_{L})
(i) comprises or consists of an amino acid sequence having at least 90%, 91%, 92%, 93%, 94%, 95%, 96%, 97%, 98% or 99% identity to the amino acid sequence encoded by the nucleic acid sequence set forth in SEQ ID NO:10; or
(ii) comprises or consists of an amino acid sequence encoded by the nucleic acid sequence set forth in SEQ ID NO: 10; or
(iii) comprises or consists of an amino acid sequence having one or more amino acid substitutions, insertions or deletions compared to the amino acid sequence encoded by the nucleic acid sequence set forth in SEQ ID NO: 10, optionally the amino acid substitutions, insertions or deletions do not occur in the CDRs, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids.

In some embodiments, the anti-EGFR/HER3 bispecific antibodies of the present invention have cysteine mutations at more cryptic sites of the constant region, thereby bring better stability and/or hydrophilicity to an ADC comprising the same. "Cysteine mutation" as defined herein refers to the substitution of an amino acid in a protein that was not originally cysteine by cysteine.

In some embodiments, in the bispecific antibodies of the present invention, the anti-EGFR/HER3 bispecific antibodies comprise any two mutations selected from: the mutation to cysteine at position 118 of the heavy chain constant region, the mutation to cysteine at position 239 of the heavy chain constant region, the mutation to cysteine at position 160 of the light chain constant region, and the mutation to cysteine at position 166 of the light chain constant region.

In some embodiments, in the bispecific antibodies of the present invention, the light chain constant region is a lambda light chain constant region or a kappa light chain constant region.

In some embodiments, the bispecific antibodies of the present invention further comprise mutations to alanine at positions 234 and 235 of the heavy chain constant region (LALA mutation).

In some embodiments, in the bispecific antibody of the present invention, the heavy chain constant region is a heavy chain constant region of human IgG1, IgG2, IgG3, or IgG 4.

In some embodiments, in the bispecific antibodies of the present invention, the light chain constant region is a human lambda light chain constant region or a kappa light chain constant region.

In some embodiments, in the bispecific antibodies of the present invention, the amino acid residues are numbered according to the EU numbering system, e.g., the amino acid residues in constant region are numbered according to the EU numbering system.

In some embodiments, in the bispecific antibodies of the present invention, the mutations of the constant regions are each independently with respect to the parent heavy chain constant region or the parent light chain constant region. The parent heavy chain constant region or the parent light chain constant region may be a native heavy chain constant region or a native light chain constant region, or may be a variant heavy chain constant region or a variant light chain constant region, provided that the amino acid at the position to be mutated is different from the amino acid to be mutated into. For example, in some embodiments, the parent heavy chain constant region is with respect to a native heavy chain constant region of the human IgG1 or the human IgG4 immunoglobulin or a native light chain constant region.

In some embodiments, in the bispecific antibodies of the present invention, the mutations are each independently relative to a native heavy chain constant region of the human IgG1 immunoglobulin or a native light chain constant region.

In some embodiments, the native heavy chain constant region of the human IgG1 is represented by an amino acid sequence set forth in SEQ ID NO:35. In some embodiments, the native human lambda light chain constant region is represented by an amino acid sequence set forth in SEQ ID NO:36. In some embodiments, the native human kappa light chain constant region is represented by an amino acid sequence set forth in SEQ ID NO:17.

In some embodiments, relative to a native light chain constant region and/or a native heavy chain constant region of the human IgG1, the bispecific antibodies of the present invention comprise any of the following combinations of mutations : (1) the mutation to cysteine at position 160 of the lambda light chain constant region and the mutation to cysteine at position 166 of the lambda light chain constant region; (2) the mutation to cysteine at position 118 of the heavy chain constant region, and the mutation to cysteine at position 239 of the heavy chain constant region; (3) the mutation to cysteine at position 160 of the lambda light chain constant region, and the mutation to cysteine at position 118 of the heavy chain constant region; (4) the mutation to cysteine at position 166 of the lambda light chain constant region, and the mutation to cysteine at position 118 of the heavy chain constant region; (5) the mutation to cysteine at position 160 of the lambda light chain constant region, and the mutation to cysteine at position 239 of the heavy chain constant region; and (6) the mutation to cysteine at position 166 of the lambda light chain constant region and the mutation to cysteine at position 239 of the heavy chain constant region.

In some embodiments, when compared to the light chain constant region and/or heavy chain constant region of the native human IgG1, the bispecific antibodies of the present invention comprise any one of the following combinations of cysteine mutations : (1) the mutation to cysteine at position 160 of the lambda light chain constant region, and the mutation to cysteine at position 166 of the lambda light chain constant region; (2) the mutation to cysteine at position 118 of the heavy chain constant region, and the mutation to cysteine at position 239 of the heavy chain constant region; (3) the mutation to cysteine at position 160 of the lambda light chain constant region, and the mutation to cysteine at position 118 of the heavy chain constant region; (4) the mutation to cysteine at position 166 of the lambda light chain constant region, and the mutation to cysteine at position 118 of the heavy chain constant region; (5) the mutation to cysteine at position 160 of the lambda light chain constant region, and the mutation to cysteine at position 239 of the heavy chain constant region; and (6) the mutation to cysteine at position 166 of the lambda light chain constant region and the mutation to cysteine at position 239 of the heavy chain constant region, and the bispecific antibody optionally further comprises a LALA mutation in the heavy chain constant region.

In some embodiments, relative to a native light chain constant region and/or a native heavy chain constant region of the human IgG1, the bispecific antibodies of the present invention comprise the following combinations of the light chain constant region and the heavy chain constant region :
(i) the lambda light chain constant region comprising mutations to cysteine at positions 160 and 166, and the heavy chain constant region without cysteine mutation;
(ii) the kappa light chain constant region without cysteine mutation, and the heavy chain constant region comprisng mutations to cysteine at positions 118 and 239;
(iii) the lambda light chain constant region comprising the mutation to cysteine at position 160 , and the heavy chain constant region comprising the mutation to cysteine at position 118 ;
(iv) the lambda light chain constant region comprising the mutation to cysteine at position 166, and the heavy chain constant region comprising the mutation to cysteine at position 118;
(v) the lambda light chain constant region comprising the mutation to cysteine at position 160, and the heavy chain constant region comprising the mutation to cysteine at position 239;
(vi) the lambda light chain constant region comprising the mutation to cysteine at position 166, and the heavy chain constant region comprising the mutation to cysteine at position 239,
optionally, the heavy chain constant region further comprises a LALA mutation.

In some embodiments, in bispecific antibodies of the present invention, the heavy chain constant region
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:11, 15, 19 and 23; or
(ii) comprises or consists of an amino acid sequence set forth in any one of SEQ ID NO:11, 15, 19 and 23; or
(iii) comprises or consists of an amino acid sequence having one or more amino acid substitutions, insertions or deletions compared to the amino acid sequence set forth in any one of SEQ ID NO:11, 15, 19 and 23, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids. In some embodiments, in a bispecific antibody of the present invention, the heavy chain constant region comprising a LALA mutation comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:11 or an amino acid sequence set forth in SEQ ID NO:11. In some embodiments, the heavy chain constant region comprising a LALA mutation and mutations to cysteine at positions 118 and 239 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:15 or an amino acid sequence set forth in SEQ ID NO:15. In some embodiments, the heavy chain constant region comprising a LALA mutation and the mutation to cysteine at position 118 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:19 or an amino acid sequence set forth in SEQ ID NO:19. In some embodiments, the heavy chain constant region comprising a LALA mutation and the mutation to cysteine at position 239 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:23 or an amino acid sequence set forth in SEQ ID NO:23.

In some embodiments, in bispecific antibodies of the present invention, the light chain constant region
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:13, 17, 21 and 25; or
(ii) comprises or consists of an amino acid sequence set forth in any one of SEQ ID NO:13, 17, 21 and 25; or
(iii) comprises or consists of an amino acid sequence having one or more amino acid substitutions, insertions or deletions compared to the amino acid sequence set forth in any one of SEQ ID NO:13, 17, 21 and 25, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids.

In some embodiments, in the bispecific antibody of the present invention, the lambda light chain constant region comprising mutations to cysteine at positions 160 and 166 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:13 or an amino acid sequence set forth in SEQ ID NO:13. In some embodiments, in the bispecific antibodies of the present invention, the lambda light chain constant region comprising the mutation to cysteine at position 160 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:21 or an amino acid sequence set forth in SEQ ID NO:21. In some embodiments, in the bispecific antibodies of the present invention, the lambda light chain constant region comprising the mutation to cysteine at position 160 comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:25 or an amino acid sequence set forth in SEQ ID NO:25. In some embodiments, in a bispecific antibodies of the present invention, the kappa light chain constant region comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:17 or an amino acid sequence set forth in SEQ ID NO:21.

In some embodiments, a bispecific antibody of the present invention comprises two identical heavy chain constant regions or comprises two different heavy chain constant regions, e.g., a cysteine mutation presents on one heavy chain constant region but no cysteine mutation presents on the other heavy chain constant region. In some embodiments, the bispecific antibody of the present invention comprises two identical light chain constant regions or comprises two different light chain constant regions, e.g., a cysteine mutation presents on one light chain constant region but no cysteine mutation presents on the other light chain constant region. In some embodiments, a bispecific antibody of the present invention comprises two identical heavy chain constant regions and two identical light chain constant regions, e.g., the two heavy chain constant regions comprise identical cysteine mutation, and/or the two light chain constant regions comprise identical cysteine mutation.

In some embodiments, in bispecific antibodies of the present invention,
the heavy chain
   (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:12, 16, 20 and 24; or
   (ii) comprises or consists of an amino acid sequence set forth in any one of SEQ ID NO:12, 16, 20 and 24; or
   (iii) comprises or consists of an amino acid sequence having one or more amino acid substitutions, insertions or deletions compared to the amino acid sequence set forth in any one of SEQ ID NO:12, 16, 20 and 24, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids; and
the light chain
   (i) comprises or consists of an amino acid sequence having at least 85%, 86%, 87%, 88%, 89%, 90%, 91%, 92%, 93%, 94%, 95%, 96%, 97%, 98% or 99% identity to an amino acid sequence selected from any one of SEQ ID NO:14, 18, 22 and 26; or
   (ii) comprises or consists of an amino acid sequence selected from any one of SEQ ID NO:14, 18, 22 and 26; or
   (iii) comprises or consists of an amino acid sequence having one or more amino acid substitutions, insertions or deletions compared to the amino acid selected from any one of SEQ ID NO:14, 18, 22 and 26, wherein the number of amino acid alterations (i.e. substitutions, insertions or deletions) is no more than 10, e.g., no more than 9, 8, 7, 6, 5, 4, 3, 2 or 1, the amino acid substitution is, for example, a conservative substitution of amino acids.

In some embodiments, the bispecific antibody of the present invention is selected from the following groups of antibodies:
Group (I)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:12; or
      (ii) comprises or consists of an amino acid sequence set forth in SEQ ID NO:12; or
      (iii) comprises or consists of an amino acid sequence having one or more amino acid substitutions, insertions or deletions compared to the amino acid sequence set forth in SEQ ID NO:12; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:14; or
      (ii) comprises or consists of an amino acid sequence set forth in SEQ ID NO:14; or
      (iii) comprises or consists of an amino acid sequence having one or more amino acid substitutions, insertions or deletions compared to the amino acid sequence set forth in SEQ ID NO:14.
Group (II)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:16; or
      (ii) comprises or consists of an amino acid sequence set forth in SEQ ID NO:16; or
      (iii) comprises or consists of an amino acid sequence having one or more amino acid substitutions, insertions or deletions compared to the amino acid sequence set forth in SEQ ID NO:16; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:18; or
      (ii) comprises or consists of an amino acid sequence set forth in SEQ ID NO:18; or
      (iii) comprises or consists of an amino acid sequence having one or more amino acid substitutions, insertions or deletions compared to the amino acid sequence set forth in SEQ ID NO:18.
Group (III)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:20; or
      (ii) comprises or consists of an amino acid sequence set forth in SEQ ID NO:20; or
      (iii) comprises or consists of an amino acid sequence having one or more amino acid substitutions, insertions or deletions compared to the amino acid sequence set forth in SEQ ID NO:20; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:22; or
      (ii) comprises or consists of an amino acid sequence set forth in SEQ ID NO:22; or
      (iii) comprises or consists of an amino acid sequence having one or more amino acid substitutions, insertions or deletions compared to the amino acid sequence set forth in SEQ ID NO: 22.
Group (IV)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:20; or
      (ii) comprises or consists of an amino acid sequence set forth in SEQ ID NO:20; or
      (iii) comprises or consists of an amino acid sequence having one or more amino acid replacements, insertions or deletions compared to the amino acid sequence set forth in SEQ ID NO:20; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:26; or
      (ii) comprises or consists of an amino acid sequence set forth in SEQ ID NO:26; or
      (iii) comprises or consists of an amino acid sequence having one or more amino acid substitutions, insertions or deletions compared to the amino acid sequence set forth in SEQ ID NO:26.
Group (V)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:24; or
      (ii) comprises or consists of an amino acid sequence set forth in SEQ ID NO:24; or
      (iii) comprises or consists of an amino acid sequence having one or more amino acid substitutions, insertions or deletions compared to the amino acid sequence set forth in SEQ ID NO:24; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:22; or
      (ii) comprises or consists of an amino acid sequence set forth in SEQ ID NO:22; or
      (iii) comprises or consists of an amino acid sequence having one or more amino acid substitutions, insertions or deletions compared to the amino acid sequence set forth in SEQ ID NO:22.
   And
Group (VI)
   the heavy chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:24; or
      (ii) comprises or consists of an amino acid sequence set forth in SEQ ID NO:24; or
      (iii) comprises or consists of an amino acid sequence having one or more amino acid substitutions, insertions or deletions compared to the amino acid sequence set forth in SEQ ID NO:24; and
   the light chain
      (i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:26; or
      (ii) comprises or consists of an amino acid sequence set forth in SEQ ID NO:26; or
      (iii) comprises or consists of an amino acid sequence having one or more amino acid substitutions, insertions or deletions compared to the amino acid sequence set forth in SEQ ID NO:26.
In some embodiments, the bispecific antibody of the present invention is selected from the following antibodies:
   (1) Duligo-LC1, having a heavy chain represented by an amino acid sequence set forth in SEQ ID NO:12, and a light chain represented by an amino acid sequence set forth in SEQ ID NO:14;
   (2) DU-v-01, having a heavy chain represented by an amino acid sequence set forth in SEQ ID NO:16, and a light chain represented by an amino acid sequence set forth in SEQ ID NO:18;
   (3) DU-v-02, having a heavy chain represented by an amino acid sequence set forth in SEQ ID NO:20, and a light chain represented by an amino acid sequence set forth in SEQ ID NO:22;
   (4) DU-v-03, having a heavy chain represented by an amino acid sequence set forth in SEQ ID NO:20, and a light chain represented by an amino acid sequence set forth in SEQ ID NO:26;
   (5) DU-v-04, having a heavy chain consists of an amino acid sequence set forth in SEQ ID NO:24, and a light chain represented by an amino acid sequence set forth in SEQ ID NO:22; and
   (6) DU-v-05, having a heavy chain consists of an amino acid sequence set forth in SEQ ID NO:24, and a light chain represented by an amino acid sequence set forth in SEQ ID NO:26.

### III. Immunoconjugates

In one aspect, the invention provides an immunoconjugate comprising an anti-EGFR/HER3 bispecific antibody or an antigen-binding fragment thereof as described in the present invention. In some embodiments, the immunoconjugate comprises an anti-EGFR/HER3 bispecific antibody or an antigen-binding fragment thereof as described in the present invention and one or more additional agents conjugated thereto, e.g., a label, a therapeutic agent, or a diagnostic agent. In the immunoconjugate, a linker can be used to covalently link different moieties of the conjugate. For example, the antibody or the antigen-binding fragment thereof is covalently linked to the one or more additional agents using a linker. Suitable linkers for the conjugates include chemical linkers or peptide linkers, and include cleavable linkers and non-cleavable linkers.

In some embodiments, the therapeutic agent includes, but is not limited to, a cytotoxic compound.

In some embodiments, the label or diagnostic agent includes but is not limited to various enzymes, e.g., horseradish peroxidase; prosthetic groups, e.g., streptavidin/biotin and avidin/biotin; fluorescent materials; luminescent materials; radioactive materials, e.g., radiolabeled tracer compounds and positron emitting metals used in various positron emission tomography imaging procedures and nonradioactive paramagnetic metal ions.

In some embodiments, the immunoconjugate is an antibody-drug conjugate.

In one aspect, the present invention provides a bispecific antibody-drug conjugate of formula (I), a stereoisomer or pharmaceutically acceptable salt or solvate thereof:

Ab-(L-D)ₙ (I)

wherein,
Ab is an anti-EGFR/HER3 bispecific antibody or an antigen-binding fragment thereof as described in the present invention,
L is a linker,
D is a biologically active compound moiety, e.g., a cytotoxic compound,
n represents the conjugation number, i.e., the number of -L-D attached to the Ab, and n is a natural number selected from 1 to 15, e.g., a natural number in the range of 1 to 14, 2 to 13, 3 to 12, 4 to 11, 5 to 10, 6 to 9, 7 to 8, 1 to 6, 1 to 5, 1 to 4, 2 to 4, 3 to 4, e.g., a natural number in a numerical range with any two values from 1, 2, 3, 4, 5, 6, 7, 8, 9, 10, 11, 12, 13, 14, and 15 as endpoints, e.g., n is 1, 2, 3, 4, 5, 6, 7, 8, 9, or 10.

As used herein, the term "immunoconjugate" generally refers to a molecule formed by conjugating one or more immunoglobulin-related molecules or fragments thereof (e.g., an antibody or a fragment thereof herein) with one or more other molecules. In some cases, the other molecule may be a proteinaceous molecule, e.g., a peptide, polypeptide, or protein. In some cases, the other molecule may also be a non-proteinaceous molecule. In some embodiments, the other molecule is, for example, a label, a therapeutic agent, or a diagnostic agent. In some cases, the one or more other molecules may be the same or different from each other. For example, the other molecule may be a target binding element and/or an effector element, such as a chemotherapeutic agent, a cytotoxic compound, a radioactive element, a probe or a signaling molecule, etc.

As used herein, the term "antibody-drug conjugate" refers to a targeted biologic drug obtained by linking a biologically active compound fragment (a drug molecule, e.g., a cytotoxic compound) to an antibody or an antigen binding fragment moiety thereof via a linking unit. The linking unit, also known as a linker, can be cleaved in a specific environment (e.g., an intracellular low pH environment) or under a specific action (e.g., the action of a lysosomal protease), thereby the biologically active compound fragment and the antibody or the antigen-binding fragment thereof are separated. The ADC can recognize the surface antigen of the tumor cell by utilizing the high affinity and specificity of the target and the antibody, and deliver a bioactive compound, e.g., a small molecular cytotoxic drug, to the tumor cell through strong intracellular endocytosis thereby achieve a precise and efficient killing of tumor cells. It will be understood that in an antibody-drug conjugate, when referring to a biologically active compound, e.g., a cytotoxic compound, it refers to the biologically active compound moiety/fragment (e.g., cytotoxic compound moiety/fragment) attached to the remainder of the antibody-drug conjugate.

As used herein, the term "linker" refers to a fragment that links a biologically active compound fragment (drug molecule) to a moiety of an antibody or an antigen-binding fragment thereof.

As used herein, the term "biologically active compound fragment" is known in the art, which is in an antibody-drug conjugate (or antibody-conjugated drug, ADC) and is capable of forming a biologically active drug, e.g., a small molecule cytotoxic drug, after cleavage/degradation/enzyme cleavage of a linker between tumor tissues or within tumor cells. It may also be referred to a "biologically active compound moiety".

As used herein, the term "DAR (drug to antibody ratio)" also referred to "a ratio of drug to antibody", refers to the number of drug moieties conjugated to an antibody in an antibody-drug conjugate molecule. The DAR may vary and will be limited by the number of available sites on the antibody. It will be appreciated that for an antibody-drug conjugates of formula (I), DAR is a natural number above 0, i.e. a positive integer. In some embodiments, the antibody-drug conjugate of the present invention has a DAR of 1 to 15, e.g., 1 to 14, 2 to 13, 3 to 12, 4 to 11, 5 to 10, 6 to 9, 7 to 8, 1 to 6, 1 to 5, 1 to 4, 2 to 4, or 3 to 4, e.g., 1, 2, 3, 4, 5, 6, 7, 8, 9, 10, 11, 12, 13, 14, or 15, and for example, a numerical range with any two values from 1, 2, 3, 4, 5, 6, 7, 8, 9, 10, 11, 12, 13, 14, and 15 as endpoints. During the conjugation process, a heterogeneous mixture of different antibody-drug conjugate (ADC) molecules (i.e. ADC molecules with different DAR) is typically produced. Thus, the term "antibody-drug conjugate (ADC)" also refers to such mixture of ADC molecules with different DAR. The term "average DAR" refers to an average value of DAR of ADC molecule population in such mixture, also referred to "average conjugation number" in the present invention. As is well known in the art, DAR and drug load distributions may be determined, for example, using hydrophobic interaction chromatography (HIC), or reverse phase high-performance liquid chromatography (RP-HPLC), wherein HIC is particularly suitable for determining average DAR. In some embodiments, the antibody-drug conjugate of the present invention has an average DAR (average conjugation number) value in the range of 1 to 15, for example a value in the range of 1 to 14, 2 to 13, 3 to 12, 4 to 11, 5 to 10, 6 to 9, 7 to 8, 1 to 6, 1 to 5, 1 to 4, 2 to 4 or 3 to 4, for example a value in a numerical range with any two values from 1, 2, 3, 4, 5, 6, 7, 8, 9, 10, 11, 12, 13, 14 and 15 as endpoints. It should be understood that, for average DAR, the "value" includes both integers and decimals. A value in the range of 1 and 15 refers to any value in the range of 1 and 15, including integers and decimals, and includes endpoints. In some embodiments, the average DAR (average conjugation number) is, for example, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0, or a value in the range of two values selected from the above as endpoints. It should be understood that when referring to an average DAR value, an ADC of the present invention refers to an ADC molecule population or a mixture of ADC molecules comprising ADC molecules with the same and/or different DARs.

"Reactive cysteine" or "free cysteine" refers to a cysteine residue that has a thiol functional group (-SH) (sulfhydryl) and that is not a part of an intramolecular or intermolecular disulfide bridge formed by pairing or other methods.

In some embodiments, in the bispecific antibody-drug conjugate of formula (I) of the present invention, the L is a linker capable of linking an antibody moiety (Ab) and a biologically active compound moiety together.

In some embodiments, in the bispecific antibody-drug conjugate of formula (I) of the present invention, the Ab comprises a cysteine. In some embodiments, in the bispecific antibody-drug conjugate of formula (I) of the present invention, the Ab is linked to a linker through its cysteine, e.g. the linker is conjugated to the Ab through the thiol group of the cysteine. In some embodiments, the Ab comprises a cysteine mutation, such that the linker is conjugated to the antibody via the thiol group of the mutated cysteine. In some embodiments, the Ab comprises the mutation that mutates one or more non-cysteines into cysteines on either its heavy or light chain, such that a linker is conjugated to the cysteine, e.g., site-specific conjugation. The anti-EGFR/HER3 antibodies or the antigen-binding fragments thereof comprising the cysteine mutation suitable for use in ADCs of the present invention are described in detail herein, wherein the cysteine obtained from said mutation is conjugated to a linker, e.g. site-specific conjugation.

In some embodiments, the Ab is conjugated to the linker via the following cysteines obtained from mutations:
(i) the cysteines obtained from cysteine mutations at positions 160 and 166 of one or two lambda light chain constant regions;
(ii) the cysteines obtained from cysteine mutations at positions 118 and 239 of one or two heavy chain constant regions;
(iii) the cysteine obtained from cysteine mutation at position 160 of one or two lambda light chain constant regions and the cysteine obtained from cysteine mutation at position 118 of one or two heavy chain constant regions;
(iv) the cysteine obtained from cysteine mutation at position 166 of one or two lambda light chain constant regions and the cysteine obtained from cysteine mutation at position 118 of one or two heavy chain constant regions;
(v) the cysteine obtained from cysteine mutation at position 160 of one or two lambda light chain constant regions and the cysteine obtained from cysteine mutation at position 239 of one or two heavy chain constant regions;
(vi) the cysteine obtained from cysteine mutation at position 166 of one or two lambda light chain constant regions and the cysteine obtained from cysteine mutation at position 239 of one or two heavy chain constant regions.

In some specific embodiments, the Ab comprising the cysteine mutation comprises two heavy chains and two light chains, and is conjugated to the linker via the following cysteines obtained from mutations:
(i) the cysteines obtained from cysteine mutations at positions 160 and 166 of two lambda light chain constant regions;
(ii) the cysteines obtained from cysteine mutations at positions 118 and 239 of two heavy chain constant regions;
(iii) the cysteines obtained from cysteine mutations at position 160 of two lambda light chain constant regions and the cysteines obtained from cysteine mutations at position 118 of two heavy chain constant regions;
(iv) the cysteines obtained from cysteine mutations at position 166 of two lambda light chain constant regions and the cysteines obtained from cysteine mutations at position 118 of two heavy chain constant regions;
(v) the cysteines obtained from cysteine mutations at position 160 of two lambda light chain constant regions and the cysteines obtained from cysteine mutations at position 239 of two heavy chain constant regions;
(vi) the cysteines obtained from cysteine mutations at position 166 of two lambda light chain constant regions and the cysteines obtained from cysteine mutations at position 239 of two heavy chain constant regions.

Any of the antibodies or the antigen-binding fragments thereof mentioned herein may be used for conjugation with a linker, e.g. site-specific conjugation, via any of the cysteines obtained from cysteine mutation described herein, to obtain an ADC molecule of the present invention.

In some embodiments, the linker L has the structure shown in formula (II):

Q-L' (II),

Q represents a linking moiety conjugated to the Ab through a thioether linkage (-S-);
L' having the following structure represents a linking moiety linking Q to cytotoxic compound D,: 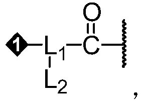
wherein L₁ is a polypeptide residue consisting of 3 to 8 amino acid residues, optionally the polypeptide residue includes at least one amino acid residue with a side chain carboxylic acid, "-C(=O)-" represents the carbonyl group of the C-terminal amino acid residue of the polypeptide residue;
L₂ is absent or is a hydrophilic group linked to the carbonyl group obtained after reacting with the carboxylic acid of the amino acid residue side chain of the polypeptide residue L₁, and L₂ is -NHR_{L2}, R_{L2} is selected from C₁₋₆ alkyl optionally substituted with 1 to 6 hydroxyl groups;
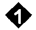 represents the N-terminus of the polypeptide residue covalently linked to the linking moiety Q.

In some embodiments, the linking moiety Q has the following structure 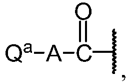 wherein Q^{a} is a functional group conjugated to the Ab;

A is selected from optionally substituted aliphatic hydrocarbylene or optionally substituted aliphatic heterohydrocarbylene, wherein the aliphatic hydrocarbylene and aliphatic heterohydrocarbylene are optionally substituted with 1 to 4 substituents independently selected from the group consisting of halogen, -CN, -OR_{Qa1}, -SR_{Qa1}, -N(R_{Qa1})₂, wherein each R_{Qa1} is independently selected from hydrogen, C₁₋₆ alkyl, C₁₋₆ haloalkyl. In some embodiments, A is selected from optionally substituted C₁₋₁₀ alkylene. In some embodiments, A is selected from optionally substituted C₁₋₁₈ aliphatic heterohydrocarbylene comprising 1 to 6 heteroatoms selected from nitrogen, oxygen, and sulfur.

Preferably, A is optionally substituted C₁₋₁₀ alkylene, C₁₋₈ alkylene, C₁₋₆ alkylene, C₃₋₆ alkylene, or C₄₋₆ alkylene, e.g., methylene, ethylene, propylene, butylene, pentylene, hexylene, heptylene, octylene, nonylene, decylene.

In some embodiments, the functional group Q^{a} is absent or selected from maleimide, iodoacetamide, bromoacetamide, pyrimidine, pyrimidyl sulfide, vinyl pyrimidine, vinyl triazine, vinyl pyridine, disulfide, pyridyl disulfide, haloacetamide, α-haloacetyl, reactive ester such as succinimidyl ester, 4-nitrophenyl ester, pentafluorophenyl ester, tetrafluorophenyl ester, anhydride, acid chloride, sulfonyl chloride, isocyanate, and isothiocyanate, preferably the functional group Q^{a} is selected from maleimide, such as the following structure 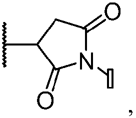 or pyrimidine, such as the following structure: 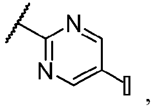
"*" represents a site covalently linked to A;
" 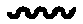 " represents a site covalently linked to the antibody Ab.

In some embodiments, the linking moiety Q has the following structure:

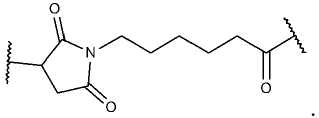

In some embodiments, the linking moiety L₁ has the following structure:

^{NH}-AA1AA²AA³...AA^{p}-^{C(=O)},

wherein each of AA¹, AA², AA³, ...... AA^{p} is independently an optionally substituted amino acid residue, optionally at least one of AA¹, AA², AA³, ...... AA^{p} is an amino acid residue with a side chain carboxylic acid, e.g. Glu or Asp;
p is an integer from 3 to 8, e.g. from 3 to 5;
"NH-" represents the N-terminus of the polypeptide residue;
"-C(=O)" represents the C-terminus of the polypeptide residue.

In some embodiments, each of AA¹, AA², AA³, ...... AA^{p} is independently an optionally substituted amino acid residue selected from the group consisting of Glu, Asp, Pro, Nva, Leu, Ile, Met, Tyr, Trp, Ser, Thr, Cys, Asn, Gln, Arg, Phe, Lys, Val, Ala, Cit, Gly, and N-alkyl amino acids, and at least one of AA¹, AA², AA³, ...... AA^{p} is Glu or Asp.

In some embodiments, the linking moiety L₁ is ^{NH}-Glu-Val-Ala-^{C(=O)}.

In some embodiments, L₂ is selected from the following structure:

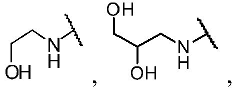

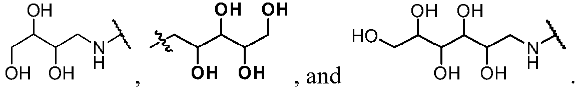

In some embodiments, L₂ has the following structure:

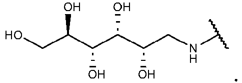

In some embodiments, the linking moiety L' has the following structure:

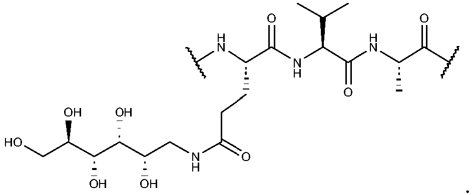

In some embodiments, the linker L has the following structure:

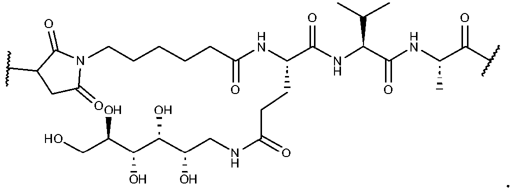

In some embodiments, the linker L is linked to the side chain of cysteine of the antibody by a maleimide connector, for example via an -S- linkage.

In some embodiments, the linker L may be composed of one or more connector components. Exemplary connector components include 6-maleimidocaproyl ("MC"), maleimidopropanoyl ("MP"), valine-citrulline ("val-cit" or "vc"), caproyl-glycine-glycine-phenylalanine-glycine (GGFG), alanine-phenylalanine ("ala-phe" or "af"), p-aminobenzyloxycarbonyl ("PAB"), N-succinimidyl 4-(2-pyridylthio)pentanoate ("SPP"), N-succinimidyl 4-(N-maleimidomethyl)cyclohexane-1 carboxylate ("SMCC"), and N-succinimidyl (4-iodo-acetyl)aminobenzoate ("SIAB"), ethyleneoxy-CH₂CH₂O- as one or more repeating units ("EO" or "PEO").

Exemplary linkers L include, but are not limited to, a maleimido-caproyl-valine-alanine (mc-va) linker, a maleimidobutanoic acid-valine-citrulline (mb-vc) linker, or a maleimido-caproyl-glycine-phenylalanine-glycine (mc-GGFG) linker.

In some embodiments, in the bispecific antibody-drug conjugate of formula (I) of the present invention, D is a biologically active compound, for example, an active compound that have biological effects on tumors (also referred to anti-tumor compound). The anti-tumor compound may be a cytotoxic compound.

In some embodiments, the cytotoxic compound D has the structure of formula (III):

Z-D' (III)

Z is absent or selected from -NH- or -NH-R_{z1}-Y-R_{z2}-, wherein R_{z1} and R_{z2} are each independently absent or selected from optionally substituted C₁₋₈ aliphatic hydrocarbylene, optionally substituted C₀₋₈ aliphatic hydrocarbylene-arylene, optionally substituted C₀₋₈ aliphatic hydrocarbylene-carbonyl (-C(O)-), optionally substituted C₀₋₈ aliphatic hydrocarbylene-oxycarbonyl (-C(O)O- or -OC(O)-), optionally substituted C₀₋₈ aliphatic hydrocarbylene-carbonyl-imino, optionally substituted arylene-carbonyl (-C(O)-), optionally substituted arylene-oxycarbonyl (-C(O)O- or -OC(O)-), optionally substituted arylene-carbonyl-imino, wherein optionally substituted refers to optionally substituted with 1 to 4 substituents independently selected from the group consisting of C₁₋₈ aliphatic hydrocarbyl, halogen, - CN, -OR_{z3}, -SR_{z3}, -N(R_{z3})₂, wherein each R_{z3} is independently selected from hydrogen, C₁₋₆ aliphatic hydrocarbyl, C₁₋₆ haloaliphatic hydrocarbyl, Y is absent or selected from -O- or -S-;
D' is a camptothecin compound, or an analogue or a derivative thereof.

In some embodiments, Z is -NH-CH₂-O-C₃-C₄ alkylene-.

In some embodiments, Z is -NH-CH₂-O-C₃H₆-, -NH-CH₂-O-C₄H₈-.

In some embodiments, Z is -NH-CH₂-O-CH₂-CH₂-CH₂- or -NH-CH₂-O-CH₂-CH₂-CH₂-CH₂-.

In some embodiments, D' is a camptothecin compound having the following structure, or an analogue or an derivative thereof: 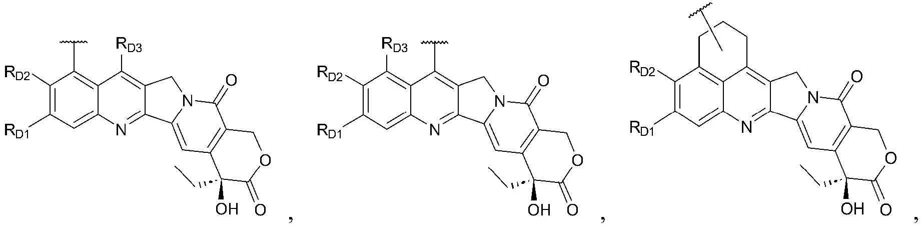 or 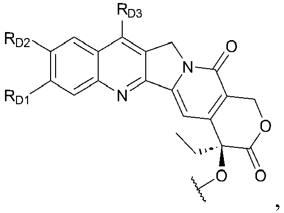 wherein R_{D1}, R_{D2} and R_{D3} are each independently selected from the group consisting of H, hydroxy, cyano, halogen, optionally substituted C₁₋₆ aliphatic hydrocarbyl, optionally substituted C₁₋₆ aliphatic hydrocarbyloxy, optionally substituted C₁₋₆ aliphatic hydrocarbylthio, and optionally substituted C₁₋₆ haloaliphatic hydrocarbyl, or R_{D1} and R_{D2} together with the carbon atom to which they are attached, form a 5-8 membered cyclic or heterocyclic group, wherein optionally substituted refers to optionally substituted with one or more substituents independently selected from the group consisting of C₁₋₈ aliphatic hydrocarbyl, halogen, -CN, -OR_{D'}, -SR_{D'}, -S(O)R_{D'} and-S(O)₂R_{D'}, and - N(R_{D'})₂, wherein each R_{D'} is independently selected from hydrogen, C₁₋₆ aliphatic hydrocarbyl, C₁₋₆ haloaliphatic hydrocarbyl, -SR_{D"}, -S(O)R_{D"} and -S(O)₂R_{D"}, each R_{D"} is independently selected from hydrogen, C₁₋₆ aliphatic hydrocarbyl, C₁₋₆ haloaliphatic hydrocarbyl.

In some embodiments, the cytotoxic compound D is selected from the following compounds:

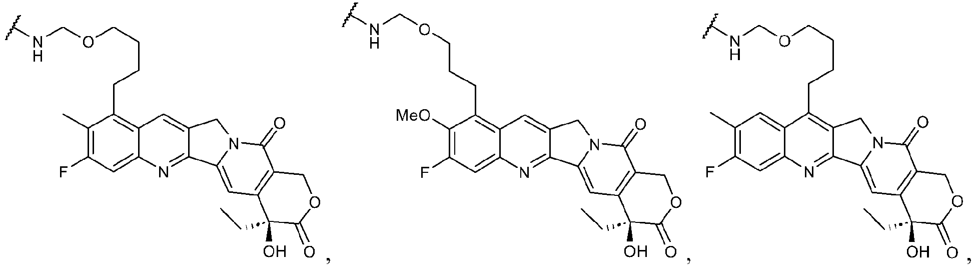

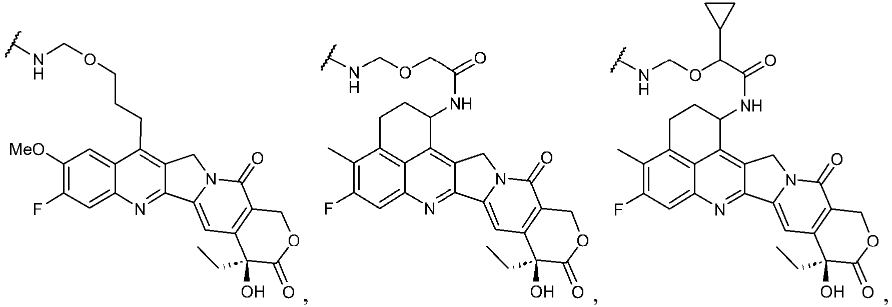

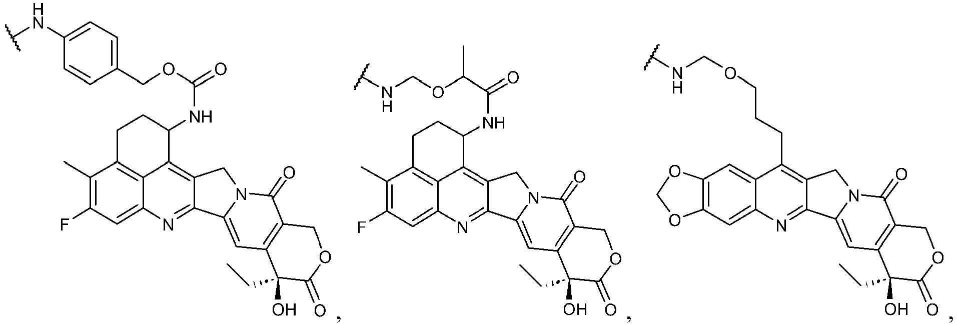

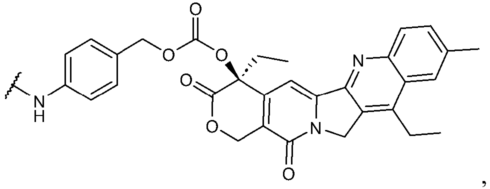

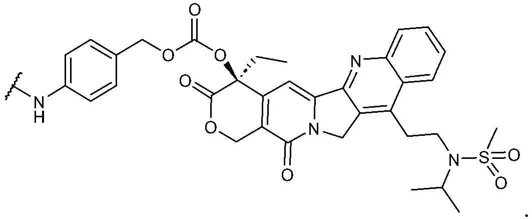

In some embodiments, the chiral carbon with * in the structural fragment 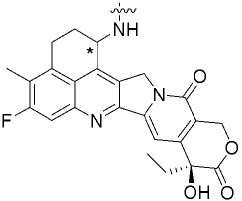 of D is in S configuration.

In some embodiments, the bispecific antibody-drug conjugate of the present invention has the following structure: 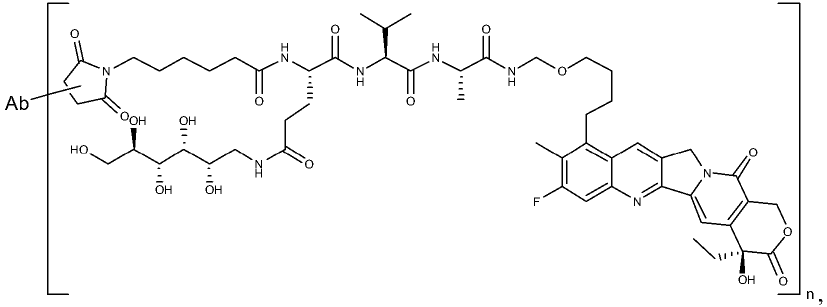 wherein Ab and n are as defined in the present invention. In the present invention, the -L-D moiety of the bispecific antibody-drug conjugate is referred as NT3, that is, the structure shown in square brackets.

In some embodiments, the bispecific antibody-drug conjugate of the present invention has the following structure: 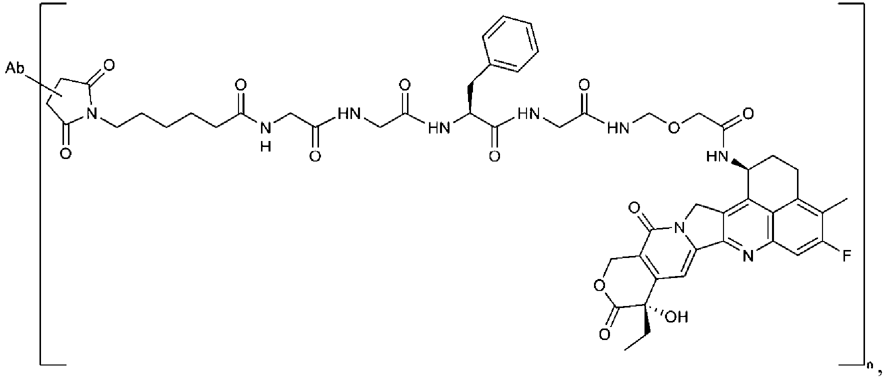 wherein Ab and n are as defined in the present invention. In the present invention, the -L-D moiety of the bispecific antibody-drug conjugate is mc-GGFG-DXd (which may also be furtrher abbreviated as DXd when describing the name of the antibody-drug conjugate), i.e., the structure shown in square brackets.

In some embodiments, the antibody-drug conjugate has an average DAR of 1 to 10, 2 to 8, 3 to 5, 3.0 to 4.0, 3.5 to 4.5, 6 to 10, 7 to 9, or 7.5 to 8.5.

The bispecific antibody-drug conjugates provided herein are described with reference to general formula and specific compounds. Furthermore, the bispecific antibody-drug conjugates of the present invention may exist as a variety of different forms or derivatives, all within the scope of the present invention. These include, for example, pharmaceutically acceptable salts, tautomers, stereoisomers, racemic mixtures, positional isomers, prodrugs, solvated forms, different crystalline forms or polymorphs, and active metabolites, etc.

As used herein, unless otherwise indicated, the term "pharmaceutically acceptable salt" includes salts that retain the biological effectiveness of the free acid/base form of the particular compound and are not undesirable in biological or other aspects. Pharmaceutically acceptable salts may include salts formed with inorganic bases or acids and organic bases or acids. In the case of the bispecific antibody-drug conjugates of the present invention contain one or more acidic or basic groups, the invention further comprises their corresponding pharmaceutically acceptable salts. Accordingly, the bispecific antibody-drug conjugates of the present invention containing an acidic group (e.g. a carboxyl group) may be present in the form of a salt and may be used according to the invention, e.g. an alkali metal salt, an alkaline earth metal salt, an aluminum salt or an ammonium salt. Further non-limiting examples of such salts include lithium, sodium, potassium, calcium, magnesium, barium or salts with ammonia or organic amines (e.g., ethylamine, ethanolamine, diethanolamine, triethanolamine, piperidine, N-methyl glutamine, or amino acids). These salts are readily available, for example, by reacting a compound having an acidic group with a suitable base (e.g., lithium hydroxide, sodium hydroxide, sodium propoxide, potassium hydroxide, potassium ethoxide, magnesium hydroxide, calcium hydroxide, or barium hydroxide). Other base salts of the bispecific antibody-drug conjugates of the present invention include, but are not limited to, copper (I), copper (II), iron (III), manganese (II), and zinc salts. The bispecific antibody-drug conjugates of the present invention contain one or more basic groups, e.g. groups that can be protonated, may be present in the form of salts, and may be used according to the invention in the form of their addition salts with inorganic or organic acids. Examples of suitable acids include hydrogen chloride, hydrogen bromide, hydrogen iodide, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, pamoic acid, mandelic acid, sulfamic acid, phenylpropionic acid, gluconic acid, ascorbic acid, isonicotinic acid, citric acid, adipic acid, taurocholic acid, glutaric acid, stearic acid, glutamic acid, or aspartic acid, and other acids known to those skilled in the art. Salts formed are, in particular, hydrochloride, chloride, hydrobromide, bromide, iodide, sulfate, phosphate, methanesulfonate (mesylate), tosylate, carbonate, bicarbonate, formate, acetate, sulfoacetate, trifluoromethanesulfonate, oxalate, malonate, maleate, succinate, tartrate, malate, pamoate, mandelate, fumarate, lactate, citrate, glutarate, stearate, aspartate and glutamate. Furthermore, the stoichiometry of the salt formed by the bispecific antibody-drug conjugate of the present invention can be an integer multiple or a non-integer multiple of 1.

The bispecific antibody-drug conjugates containing a basic nitrogen-containing group of the present invention can be quaternized using agents such as C₁₋₄ alkyl halides, e.g., methyl, ethyl, isopropyl and tert-butyl chloride, bromide, and iodide; di(C₁₋₄ alkyl) sulfates, such as dimethyl, diethyl, and diamyl sulfates; C₁₀₋₁₈ alkyl halides, such as decyl, dodecyl, lauryl, myristyl and stearyl chlorides, bromides and iodides; and aryl C₁₋₄ alkyl halides, such as benzyl chloride and phenethyl bromide.

If the bispecific antibody-drug conjugate of the present invention contains both an acidic group and a basic group in the molecule, the present invention includes, in addition to the above salt forms, an inner salt or betaine (zwitterion). The corresponding salts can be obtained by conventional methods known to those skilled in the art, for example by contacting them with organic or inorganic acids or bases in solvents or dispersants, or by anion exchange or cation exchange with other salts. The present invention further comprises all salts of the bispecific antibody-drug conjugate of the present invention which are not suitable for directly use as drugs due to low physiological compatibility, but which can be used, for example, as intermediates for chemical reactions or for the preparation of pharmaceutically acceptable salts. For reviews of more suitable salts, see Stahl and Wermuth, Handbook of Pharmaceutical Salts: Properties, Selection, and Use (Wiley-VCH, 2002).

The bispecific antibody-drug conjugate of formula (I) and its pharmaceutically acceptable salts may exist as both non-solvated and solvated forms. As used herein, the term "solvate" refers to a molecular complex comprising a bispecific antibody-drug conjugate of formula (I), a stereoisomer thereof, or a pharmaceutically acceptable salt thereof, as well as one or more pharmaceutically acceptable solvent molecules. For example, the term "hydrate" is used when the solvent is water.

The bispecific antibody-drug conjugate of formula (I) may have one or more chiral (asymmetric) centers. The present invention encompasses all stereoisomeric forms of the bispecific antibody-drug conjugate of formula (I). Centers of asymmetry that are present in the bispecific antibody-drug conjugate of formula (I) may all independently of one another have (R) or (S) configuration. When a bond to a chiral carbon is described as a straight line in the structural formulas of the present invention, or when the compound name is described without an (R) or (S) chiral name designation for a chiral carbon, it is understood that both the (R) and (S) configurations of each such chiral carbon, and thus each enantiomer or diastereomer and mixtures thereof, are encompassed within the formula or by the name. The production of a particular stereoisomer or mixture thereof may be identified in the Examples where such stereoisomers or mixtures were obtained, but this in no way limits the inclusion of all stereoisomers and mixtures thereof from being within the scope of the present invention. When a bond to a chiral carbon is described as a triangular solid or dashed line in the structural formulae of the present invention, or when the compound name is described with an (R) or (S) chiral name designation for a chiral carbon, it is understood that the compound represented by the structural formula or name at this time has a defined stereoconfiguration at the chiral carbon position, and will be distinguished from other stereoisomers or enantiomers or diastereomers or mixtures thereof.

The present invention includes all possible enantiomers and diastereomers as well as mixtures of two or more stereoisomers, for example, mixtures of enantiomers and/or diastereomers in all proportions. Thus, enantiomers are a subject of the present invention in enantiomerically pure form (both as the levorotatory and dextrorotatory enantiomers), in the form of the racemate and in the form of mixtures of the two enantiomers in all ratios. In the case of cis/trans isomerism, the present invention includes both the cis form and the trans form and mixtures of these forms in all ratios. If desired, the preparation of individual stereoisomers may be carried out by separation of a mixture by conventional methods, for example by chromatography or crystallization, by use of stereochemically uniform starting materials or by stereoselective synthesis. Optionally, a derivatization may be carried out prior to separation of stereoisomers. The separation of a mixture of stereoisomers may be carried out in an intermediate step during the synthesis of the bispecific antibody-drug conjugate of formula (I) or it can be done on the final racemic product. Absolute stereochemistry may be determined by the X-ray crystallography of crystalline products or crystalline intermediates which are derivatized, if necessary, with a reagent containing a stereocenter of known configuration. Alternatively, absolute stereochemistry can be determined by Vibrational Circular Dichroism (VCD) spectroscopic analysis.

Unless otherwise indicated, the structures described herein are also intended to include compounds that differ only in the presence of one or more isotopically enriched atoms, in other words, the compounds in which one or more atoms are replaced by atoms having the same atomic number, but an atomic mass or mass number different from the atomic mass or mass number which predominates in nature. Such compounds are referred as "isotopic variants". The present invention is intended to include all pharmaceutically acceptable isotopic variants of the bispecific antibody-drug conjugate of formula (I). Examples of isotopes suitable for inclusion in bispecific antibody-drug conjugate of the present invention include, but are not limited to, isotopes of hydrogen, such as ²H (i.e., D, deuterium) and ³H (i.e., tritium); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O and ¹⁸O; phosphorus, such as ³²P; and sulfur, e.g., ³⁵S. Certain isotopic variants of the bispecific antibody-drug conjugate of formula (I), for example those incorporating a radioactive isotope, may be useful in drug and/or substrate tissue distribution studies. In particular, compounds having the depicted structure that differ only in replacement with heavier isotopes (e.g., the replacement of hydrogen with deuterium (²H or D)) can afford certain therapeutic advantages, for example, resulting from greater metabolic stability, increased *in vivo* half-life or reduced dosage requirements, and thus can be utilized in certain circumstances. Isotopic variants of the bispecific antibody-drug conjugate of formula (I) can generally be prepared by conventional techniques known to those skilled in the art or by processes analogous to those described in the accompanying examples and syntheses using appropriate isotopically labeled reagents in place of the non-labeled reagents previously employed.

When describing the structure of the compounds herein, in the case of a hydrogen atom in the depicted structural formula is not indicated as D (i.e., ²H), it is generally understood the hydrogen at this position exists in the form of the hydrogen isotope "¹hydrogen (¹H)" or in the form of natural isotopic abundance in nature. When a hydrogen atom in the depicted structure is indicated as D (i.e., ²H, deuterium), it is understood that the hydrogen at this position exist in the form of the hydrogen isotope "²hydrogen (²H, D, deuterium)" or where the deuterium exist in a form with greater than the abundance of natural deuterium isotope (e.g., the abundance of deuterium is greater than 50%, greater than 60%, greater than 70%, greater than 80%, greater than 90%, greater than 95%, greater than 96%, greater than 97%, greater than 98%, greater than 99%, greater than 99.5%, or 100%).

Pharmaceutically acceptable solvates according to the present invention may include those in which the solvent of crystallization may be isotopically substituted, such as D₂O, d₆-acetone, d₆-DMSO.

### III. Use and administration

The bispecific antibody-drug conjugate of the present invention (the bispecific antibody-drug conjugate of formula (I), a stereoisomer thereof) -or a pharmaceutically acceptable salt, a solvate thereof, including a mixture thereof in all ratios are useful as medicaments. It was found that they exhibit pharmacological activity targeting EGFR and/or HER3 and thereby killing cancer cells with high expression of EGFR and/or HER3. Through this activity, the bispecific antibody-drug conjugates of the present invention may be used to treat conditions or diseases associated with EGFR and/or HER3 activity, such as EGFR and/or HER3 positive tumors.

Accordingly, the bispecific antibody-drug conjugates of the present invention capable of targeting EGFR and/or HER3 are particularly suitable for the treatment of diseases and conditions associated with EGFR and/or HER3 activity, e.g., cancers and tumors, including but not limited to: lymphoma, blastoma, sarcoma, leukemia, melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer, etc., in particular, breast cancer, lung cancer, pancreatic cancer, colorectal cancer, gastric cancer, head and neck squamous carcinoma, ovarian cancer, melanoma, prostate cancer, bladder cancer, etc.

The bispecific antibody-drug conjugates of the present invention can be administered in an amount effective to treat the diseases or conditions described herein. The bispecific antibody-drug conjugates of the present invention can be administered as the antibody-drug conjugat*e per se*, or optionally, as a pharmaceutically acceptable salt. For the purpose of administration and dosing, the bispecific antibody-drug conjugate of the present invention *per se* (the bispecific antibody-drug conjugate of formula (I), a stereoisomer thereof) or a pharmaceutically acceptable salt, a solvate thereof will be simply referred as the bispecific antibody-drug conjugate of the present invention.

The bispecific antibody-drug conjugates of the present invention are administered via any suitable route and in the form of a pharmaceutical composition suitable for such route, and in a dose expected to be effective for the treatment. The bispecific antibody-drug conjugate of the present invention can be administered intravenously, subcutaneously, intranasally, orally, rectally, vaginally, parenterally or externally, etc.

As used herein, the term "administering", "administered" and "administration" refer to introducing the bispecific antibody-drug conjugate of the present invention or a pharmaceutical composition thereof by absorbing, ingesting, injecting, inhaling, implanting, or otherwise. The term "treating" and "treatment" refers to reversing, alleviating, delaying the onset of, or inhibiting the progression of the "pathological condition" (e.g., a disease, condition or condition, or one or more signs or symptoms thereof) described herein. In certain embodiments, the treatment may be administered after one or more signs or symptoms of the disease or condition have developed or have been observed. In other embodiments, the treatment may be administered without a sign or symptom of the disease or condition. For example, the treatment may be administered in susceptible individuals (e.g., based on a history of symptoms and/or based on genetic or other susceptibility factors) prior to the onset of symptoms. The treatment may also be continued after the regression of the symptoms, e.g., to delay or prevent relapse. As used herein, the terms "disease," "condition", "condition" and "pathological condition" are used interchangeably.

The administered dosage level can be determined by those skilled in the art by conventional experiments. The dosage regimen for the bispecific antibody-drug conjugates of the present invention and/or the compositions comprising the antibody-drug conjugates depends on a variety of factors, including the species, age, weight, sex, and medical condition of the patient; the severity of the condition; the route of administration; and the activity of the particular antibody-drug conjugate used. Thus, the dosage regimen may vary widely.

In some embodiments, the bispecific antibody-drug conjugates of the present invention may be administrated in combination with one or more other therapeutic agents. In some embodiments, non-limiting examples of such other therapeutic agents include chemotherapeutic agents, angiogenesis inhibitors, cytokines, cytotoxic agents, other antibodies, small molecule drugs, immunomodulators, targeted degraders, etc. These therapeutic agents may be administered before, after, or simultaneously or sequentially in any order with the bispecific antibody-drug conjugate of the present invention.

### IV Pharmaceutical compositions

In some aspects, the present invention relates to a pharmaceutical composition comprising the bispecific antibody-drug conjugate of formula (I) as provided herein, a stereoisomer thereof, or a pharmaceutically acceptable salt or a solvate thereof, as well as at least one pharmaceutically acceptable carrier or excipient.

As used herein, the term "pharmaceutically acceptable carrier and/or excipient" refers to a carrier or excipient that can be used in the preparation of a pharmaceutical composition, which is generally safe, non-toxic, and are not undesirable in biological or other aspect, and includes carriers or excipients that are acceptable for veterinary use as well as human pharmaceutical use. The pharmaceutically acceptable carrier or excipient as used herein includes one and more than one such carrier or excipient. The particular carrier or excipient used will depend on the mode and purpose of application of the bispecific antibody-drug conjugate of the present invention. Suitable carriers and excipients are well known to those skilled in the art and are described in detail, for example, in Ansel, Howard C et al., Ansel's Pharmaceutical Dosage Forms and Drug Delivery Systems. Philadelphia: Lippincott, Williams & Wilkins, 2004; Gennaro, Alfonso R. et al., Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. It may also include one or more of buffers, stabilizers, surfactants, wetting agents, lubricants, emulsifiers, suspending agents, preservatives, antioxidants, opaquing agents, glidants, processing aids, colorants, sweeteners, perfuming agents, flavoring agents, diluents, and other known additives to provide a therapeutic performance of the drug (i.e., the bispecific antibody-drug conjugate or pharmaceutical composition provided herein) or aid in the manufacturing of the pharmaceutical product (i.e., medicament).

The compositions of the present invention may be formulated in multiple forms. These include, for example, liquid, semi-solid, and solid dosage forms, such as liquid solutions (e.g., injectable and infusible solutions), dispersions or suspensions, tablets, pills, powders, liposomes, suppositories, etc. The form depends on the intended mode of administration and therapeutic application.

The pharmaceutical compositions of the present invention may be prepared by any of the well-known pharmaceutical techniques, e.g., effective formulations and administration procedures. The above considerations regarding effective formulations and administration procedures are well known in the art and are described in standard textbooks. Formulation of drugs is discussed in, for example, in Hoover, John E., Remington's Pharmaceutical Sciences, Mack Publishing Co., Easton, Pennsylvania, 1975; Liberman et al., editions, Pharmaceutical Dosage Forms, Marcel Decker, New York, N.Y., 1980; and Kibbe et al., editions, Handbook of Pharmaceutical Excipients, 3 rd edition, American Pharmaceutical Association, Washington, 1999.

In yet another aspect, the invention relates to a kit for treating diseases and conditions associated with HER3 activity, e.g., cancer, comprising a bispecific antibody-drug conjugate of formula (I), a stereoisomer thereof, or a pharmaceutically acceptable salt or a solvate thereof, as provided herein, or a pharmaceutical composition comprising the bispecific antibody-drug conjugate of formula (I), the stereoisomer thereof, or the pharmaceutically acceptable salt or the solvate thereof, as provided herein, an optional container and an optional package insert or label instructing treatment.

### V Treatment method

In yet another aspect, the invention relates to a method of treating a disease and condition associated with EGFR and/or HER3 activity, e.g., cancer, in a subject in need thereof, comprising administering to the subject a therapeutically effective amount of the bispecific antibody-drug conjugate of formula (I), the stereoisomer thereof, or the pharmaceutically acceptable salt or the solvate thereof or the pharmaceutical composition of the present invention, as provided herein.

As used herein, the term "subject in need thereof" is a subject suffering from a disease and condition associated with EGFR and/or HER3 activity, e.g., cancer, or a subject having an increased risk of developing a disease or condition associated with EGFR and/or HER3 activity relative to the whole population. In certain embodiments, the subject is a warm-blooded animal. In certain embodiments, the warm-blooded animal is a mammal. In certain embodiments, the warm-blooded animal is a human.

As used herein, the term "therapeutically effective amount" refers to an amount effective to achieve a desired therapeutic result at a required dosage and for a required period of time. The therapeutically effective amount is such an amount in which any toxic or detrimental effect of the antibody drug conjugate or the pharmaceutical composition is inferior to the therapeutically beneficial effects. The "therapeutically effective amount" preferably inhibits a measurable parameter (e.g., tumor volume) by at least about 30%, even more preferably by at least about 40%, 45%, 50%, 55%, 60%, 65%, 70%, 75%, 80%, 85%, 90%, or even 100%, relative to an untreated object.

As used herein, the term "disease and condition associated with EGFR and/or HER3 activity" refers to any pathophysiological condition where inhibition of EGFR and/or HER3 would be beneficial. In certain embodiments, the disease and condition associated with EGFR and/or HER3 activity is a cancer. In some embodiments, the diseases and conditions associated with EGFR and/or HER3 activity is an EGFR and/or HER3 positive tumor, e.g., a tumor with EGFR and/or HER3 overexpression. In some embodiments, the subject (particularly an adult subject) has EGFR and/or HER3 expression, particularly EGFR and/or HER3 overexpression. In some embodiments, the subject has e.g., an elevated level of EGFR and/or HER3, e.g., at nucleic acid or protein or activity level, for example, compared to a healthy subject. In some embodiments, the biological sample (e.g., tumor cells or tumor tissues) of the subject has e.g., an elevated level, (e.g., elevated by greater than 10%, greater than 20%, greater than 50%, greater than 80%, greater than 90%, greater than 1-fold, greater than 2-fold, greater than 3-fold, greater than 5-fold, greater than 10-fold, greater than 50-fold, greater than 100-fold, or a range between these values (e.g., 10% -2 fold)), of EGFR and/or HER3, e.g., at nucleic acid or protein or activity level, for example, compared to a biological sample of a healthy subject (e.g., a corresponding tissue or cell in a healthy subject), or compared to EGFR and/or HER3 in an adjacent healthy tissue or cell of the subject. In certain embodiments, the disease and condition associated with EGFR and/or HER3 activity is a cancer or tumor selected from the group consisting of: lymphoma, blastoma, sarcoma, leukemia, melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer, in particular, breast cancer, lung cancer, pancreatic cancer, colorectal cancer, gastric cancer, head and neck squamous carcinoma, ovarian cancer, melanoma, prostate cancer, and bladder cancer, etc.

In some embodiments, the method of treatment of the present invention further comprises coadministering one or more other therapeutic agents. Non-limiting examples of such other therapeutic agents include chemotherapeutic agents, angiogenesis inhibitors, cytokines, cytotoxic agents, other antibodies, small molecule drugs, immunomodulators, targeted degraders, etc. These therapeutic agents may be administered before, after, simultaneously or sequentially in any order with the bispecific antibody-drug conjugate of the present invention.

In some embodiments, the method further comprises administering to the patient one or more treatment modalities selected from the group consisting of radiation therapy, cell therapy, gene therapy, RNA therapy, surgery, etc.

In yet another aspect, the invention relates to the bispecific antibody-drug conjugate of formula (I), the stereoisomer thereof, or the pharmaceutically acceptable salt or the solvate thereof, or a pharmaceutical composition of the present invention, as provided herein, for use in the treatment of a diseases and conditions associated with EGFR and/or HER3 activity, e.g., cancers or tumors. In some embodiments, the diseases and conditions associated with EGFR and/or HER3 activity are as defined herein.

In yet another aspect, the present invention relates to the use of the bispecific antibody-drug conjugate of formula (I), the stereoisomer thereof, or the pharmaceutically acceptable salt or the solvate thereof, or the pharmaceutical composition of the present invention as provided herein, in the manufacture of a medicament for the treatment of a diseases and conditions associated with EGFR and/or HER3 activity, e.g., cancers or tumors. In some embodiments, the diseases and conditions associated with EGFR and/or HER3 activity are as defined herein.

### Examples

The technical solution of the present invention is further described in detail by the following examples with reference to the accompanying drawings. Unless otherwise indicated, the methods and materials of the examples described below are all conventional products available on the market. It will be appreciated by those skilled in the art that the methods and materials described below are only exemplary, and should not be considered as limiting the scope of the present invention.

The embodiments of the present invention are not limited to the examples described below, and various changes and modifications in form and detail may be made by those skilled in the art without departing from the spirit and scope of the present invention, which is considered to fall within the scope of the present invention.

### Example 1: Preparation of antibody molecules of Duligotuzumab, Duligo-LC1, Patritumab, SIB001, IgG1LALA and Cetuximab

Recombinant antibodies Duligotuzumab (Heavy chain sequence: SEQ ID NO: 29; Light chain sequence: SEQ ID NO: 30), Duligo-LC1 (an antibody resulted from Duligotuzumab by cysteine mutation), Patrituzumab (Heavy chain sequence: SEQ ID NO: 33; Light chain sequence: SEQ ID NO: 34), SIB001 (Heavy chain sequence: SEQ ID NO: 27; Light chain sequence: SEQ ID NO: 28), IgG1LALA (Heavy chain sequence: SEQ ID NO: 31; Light chain sequence: SEQ ID NO: 32) and Cetuximab (Heavy chain sequence: SEQ ID NO: 38; Light chain sequence: SEQ ID NO: 39) were prepared by transient transfection using Expi293F cells (purchased from Gibco, A14527).

The Expi293 cells were passaged according to the desired transfection volume, and the cell density was adjusted to 3×10⁶ cells/ml at the day of transfection. 1/10 transfection final volume of Opti-MEM medium (Gibco, REF # 31985-070) was taken as the transfection buffer, and added to the DNA to be transfected at a ratio of 1mg/L, in which the ratio of the light and heavy chain plasmid pCDNA3.1 was 1:1, mixed uniformly. PEIMax (Polysciences Inc. Cat # 24765-1) was added at a DNA : PEI mass ratio of 1:3, mixed uniformly, incubated at room temperature for 20 min. The mixture was poured into Expi293F cell suspension gently, shaked and turned over, and the cells were cultured in a shaker under the culture conditions of 8% CO₂, 36.5 °C and 120 rpm. After 16-18 h of culture, 2% (volume ratio) of Feed (100 g/L Phytone Peptone + 100 g/L Difco Select Phytone) with a concentration of 200 g/L, a final concentration of 5 g/L glucose solution, and a final concentration of 2.2 mM VPA (Merck, Cat # P4543-100G) were added into the cell suspension, mixed gently, and continuously cultured at 8% CO₂, 36.5 °C and 120 rpm for 7 days.

The cell supernatant was collected for antibody purification. HiTrap MabSelect prism A (GE Healthcare, Cat # 17549853) affinity chromatography column was selected for affinity capture, the affinity chromatography column was treated with 0.1 M NaOH for 2 hours before purification, then the tube and column were washed with 10-20 fold column volume of distilled water, and the packed column was balanced with 5 fold column volume of 1×PBS (Gibco); the filtered cell fluid was passed through the column, and then the packed column was washed with 10 fold column volume of 1×PBS to remove the non-specific binding protein; the padding was washed with 5 fold column volume of eluting buffer (100 mM sodium citrate, pH 3.5), the eluate was collected, ant adjusted with 2 M Tris to pH 6.0, filtered and sterilized, and antibody conjugation was performed after SEC purity detection was qualified (> 98%).

Antibody-drug conjugates comprising NT3 (NT3-ADC) (NT3, i.e., MB3 in example 4 of WO2021173773A1) and antibody-drug conjugates comprising Dxd (DXd-ADC) were synthesized in the following examples respectively, as shown below, it should be understood that, in the following schematic structural diagram, only one drug molecule moiety is specifically shown by way of example, but that these antibody-drug conjugates may have DARs greater than 1, e.g., natural numbers in the range of 1 to 10, 1 to 9, 1 to 8, 1 to 7, 1 to 6, 1 to 5, 1 to 4, 2 to 4, or 3 to 4. The determined DARs for these antibody-drug conjugates prepared in the examples, i.e. the average DARs, are provided in the following section.

The structural schematic diagram of the NT3-ADC is as follow: 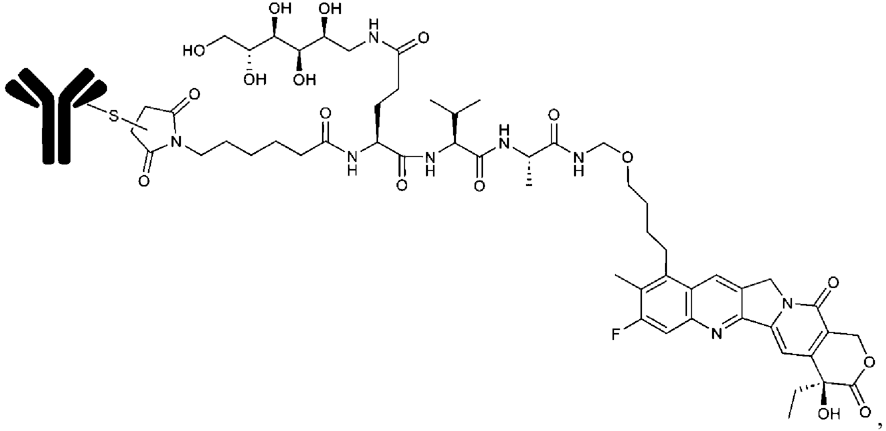 specifically, the antibody moiety is, for example, Duoligo-LC1, DU-v-01, DU-v-02, DU-v-03, DU-v-04, DU-v-05 and IgG1LALA, the amino acid sequences of which are detailed in the Sequence listing. Meanwhile, the antibody moiety may be a monospecific antibody, a bispecific antibody or a multi-specific antibody and an antigen binding fragment, and the schematic diagram here depicted Y-type antibody only by way of example.

The structural schematic diagram of the DXd-ADC is as follow: 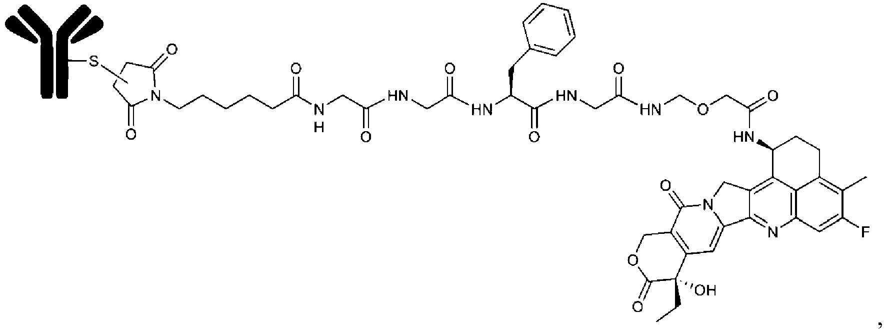 specifically, the antibody moiety is, for example, SIB001, Patritumab, Duligotuzumab (also abbreviated as Duligo), and IgG1LALA, the amino acid sequence set forth in which is detailed in the Sequence listing. Meanwhile, the antibody moiety can be a monospecific antibody, a bispecific antibody or a multi-specific antibody and an antigen binding fragment, and the schematic diagram here depicted Y-type antibody only by way of example.

### Example 2: Synthesis and characterization of Duligo-LC1-NT3

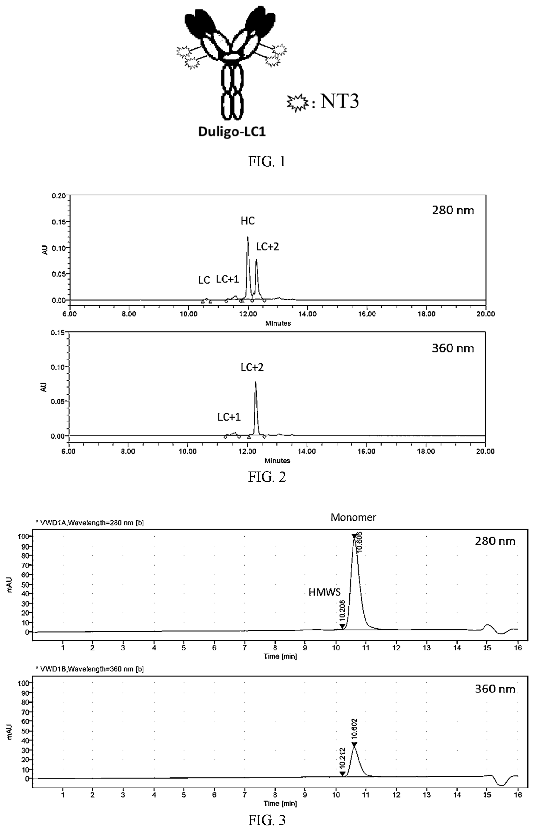

FIG. 1 shows a schematic diagram of the molecular structure of the bispecific antibody-drug conjugate Duoligo-LC1-NT3 of the present invention.

Duligo-LC1 (36.8 mg) was taken and dissolved in 20 mM histidine (8.6 mL, pH = 5.5), 10 mM TCEP solution (513 µL, 20 equiv.) was added, mixed uniformly, then reacted at 37 °C for 3 hours. RP-HPLC confirmed that all interchain disulfide bonds were reduced, the reaction was transferred to a dialysis cassette (Thermo Scientific, Slide-A-Lyzer 20K MWCO) with 20 mM histidine (800 mL, pH = 6.5) as the dialysate, the fresh buffer was replaced at 2 hours, 4 hours sequentially, and dialyzed overnight to remove thiol-containing small molecule impurities and TCEP in the reaction. After the dialysis was completed, 35 mg (95%) of an antibody was obtained.

10 mM dhAA (dehydroascorbic acid, 975 µL, 40 equiv.) was added into the above dialyzed antibody solution (2.55 mg/mL), and mixed uniformly, then reacted at 37 °C for 3 hours. RP-HPLC confirmed that the signal of a free light chain was disappeared, DMSO (164 µL) and NT3 in DMSO (277 µL, 10 mg/mL) were added sequentially, and mixed uniformly, then reacted at 25 °C for 2 hours. After the reaction was completed, small molecule impurities were removed by purification using a desalting column, and the solution was replaced with the 20 mM histidine buffer, pH = 5.5, to obtain the objective ADC product duoligo-LC1-NT3 (32 mg, 90%).

Duligo-LC1-NT3 (50 µg) was taken and diluted to 1 mg/mL by adding H₂O, dithiothreitol (DTT) solution (1 M, 1.0 µL) was added and adjusted to pH = 7.5, and after incubation for 30 minutes at 37 °C, RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 30 min | |

| Time (min) | Mobile phase A% | Mobile phase B% |
|---|---|---|
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The RP-HPLC assay result is shown in FIG. 2, and the calculated average DAR value is 3.64.

Duligo-LC1-NT3 (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25 °C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 3, the proportion of high molecular weight components and monomer ADC were 0.59% and 99.41%, respectively, and no low molecular weight fragment was observed.

Reagent and chromatography column

| **Name** | **Manufacturer, Cat#, Lot#** |
|---|---|
| TFA | Thermo Scientific, Lot# W1334149 |
| Histidine | Sigma, Lot# SLCG1233 |
| TCEP | Aldrich, Lot# SLBZ2552 |
| DMSO | Sigma, Lot# RNBJ3655 |
| Na₂HPO₄ | SCR, Lot# 10039-32-4 |
| NaH₂PO₄ | SCR, Lot# 13472-35-0 |
| NaCl | SCR, Lot# 7647-14-5 |
| (NH₄)₂SO₄ | Aladdin, Lot# H2007071 |
| Isopropanol | Wochem, Lot# 67-63-0 |
| Desalting column | Zeba^{™} Spin Desalting Columns, 40K MWCO, 2/5/10 mL |
| Slide-A-Lyzer dialysis cassette assette | Thermo Scientific, 20K MWCO |
| Amicon 30K | Amicon, Cat# UFC503096, Lot# R0PB38507 |
| SEC column | TSK gel G3000SWXL, 7.8 mm × 30 cm, 5 µm |
| RP column | Waters, BioResolve RP mAb Polyphenyl, 450 Å, 2.7 µm, 2.1 mm × 15 cm |
| HIC column | TSKgel Butyl-NPR, 2.5 µm, 4.6 × 100 mm |

Equipment

| **Analysis** | **Equipment** |
|---|---|
| RP-HPLC | Waters, ACQuity H class UPLC, SZ-A2-DD-HPLC01 |
| SEC purity | Agilent, 1260, AS-146 |
| HIC chromatography | Agilent, 1260, AS-146 |

### Example 3: Preparation of different conjugation site mutants, synthesis and characterization of corresponding site-specific conjugated ADC, and selection of conjugation site

Referring to Example 1, five antibody molecules, DU-v-01, DU-v-02, DU-v-03, DU-v-04 and DU-v-05 (Full-length sequence is shown in the Sequence listing), which respectively have different cysteine mutation sites, were prepared by mutating the heavy chain constant region or the light chain constant region of Duligotuzumab. Referring to example 2, five ADC molecules, DU-v-01-NT3, DU-v-02-NT3, DU-v-03-NT3, DU-v-04-NT3 and DU-v-05-NT3 were synthesized and characterized. The sites, protein expression yields and one-step purification efficiencies, the average DAR values for ADC conjugation and the SEC purities after ADC conjugations of each mutant are summarized in the following table,

| **Molecule** | **Mutation** | **Full-length heavy chain sequence/Full-length light chain sequence** | **Expression level (mg/L)** | **Purity over one step** | **Average DAR of ADC (NT3)** | **Monomer % (NT3)** |
|---|---|---|---|---|---|---|
| **Duligo-LC1** | LLC 160, LLC166 | SEQ ID NO:12/14 | 149.4 | 95.85% | 3.53 | 99.41 |
| **DU-v-01** | HC118, HC239 | SEQ ID NO:16/18 | 214.2 | 98.55% | 3.24 | 88.57 |
| **DU-v-02** | LLC160 (LC1), HC118 | SEQ ID NO:20/22 | 128.2 | 92.23% | 3.54 | 88.38 |
| **DU-v-03** | LLC166 (LC1), HC118 | SEQ ID NO:20/26 | 56.9 | 97.13% | 3.19 | 81.46 |
| **DU-v-04** | LLC160 (LC1), HC239 | SEQ ID NO:24/22 | 90.2 | 86.90% | 3.54 | 98.45 |
| **DU-v-05** | LLC166 (LC1), HC239 | SEQ ID NO:24/26 | 23.5 | 99.32% | 3.50 | 99.16 |

The Duligo-LC1 mutant shows excellent properties in expression yield of antibody, one-step purification efficiency, DAR value and SEC purity (aggregation condition) of ADC molecules after conjugation, and compared with other mutation combinations, the LLC160-LLC166 mutation site combination shows the optimal conjugation effects.

### Example 4: Synthesis and characterization of SIB001-DXd

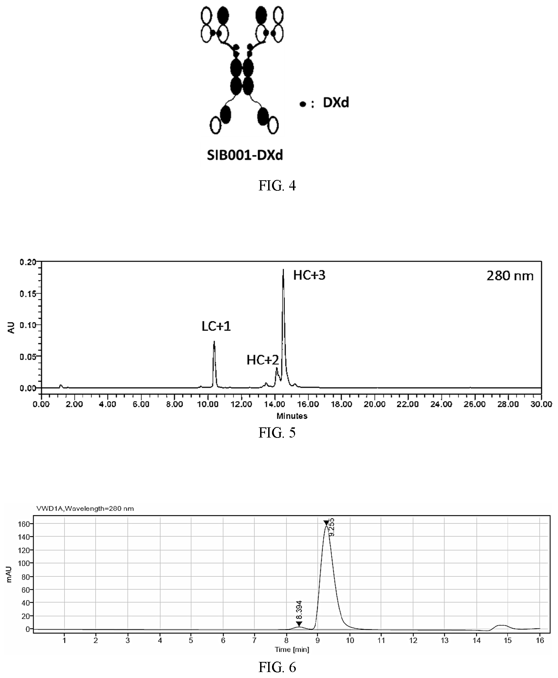

The schematic diagram of molecular structure of SIB001-DXd is shown in FIG. 4.

SIB001 (30.0 mg) was taken and dissolved in 20 mM histidine (7.5 mL, pH = 6.5), 10 mM TCEP solution (228 µL, 15 eq) was added, and mixed well, then reacted at 37 °C for 2 hours. RP-HPLC confirmed that all interchain disulfide bonds were reduced, DMSO (545 µL) and mc-GGFG-DXd in DMSO (205 µL, 10 mg/mL) were added sequentially, and mixed uniformly, then reacted at 25 °C for 1 hour. After the reaction was completed, small-molecule impurities were removed by purification using a desalting column, and the solution was replaced with 20 mM histidine buffer, pH = 6.5, to obtain the objective ADC product SIB001-DXd (28 mg, 91%). Wherein, mc-GGFG-DXd has the following structure:

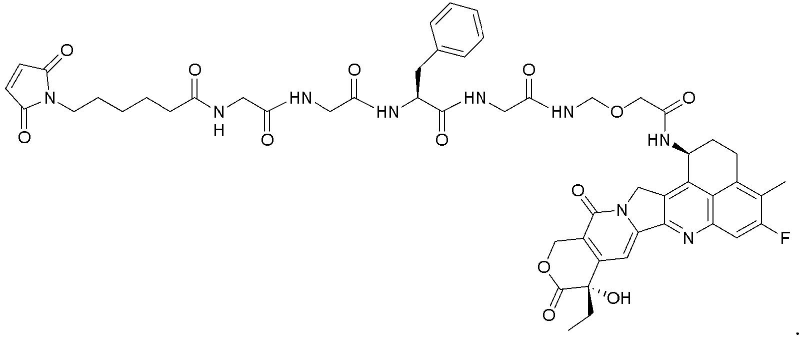

SIB001-DXd (50 µg) was taken and diluted to 1 mg/mL by adding H₂O, and the RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 30 min | |

| Time (min) | Mobile phase A% | Mobile phase B% |
|---|---|---|
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The RP-HPLC assay result is shown in FIG. 5, and the calculated average DAR value is 7.51.

SIB001-DXd (50 µg) was diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 6, the proportion of high molecular weight components and monomer ADC were 2.24% and 97.76%, respectively, and no low molecular weight fragment was observed.

### Example 5: Synthesis and characterization of Patritumab-DXd

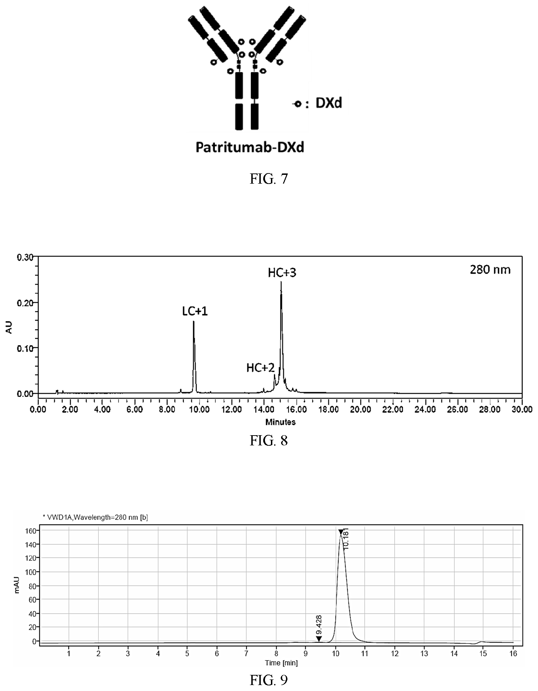

The molecular structure of Patritumab-DXd is shown in FIG. 7.

Patritumab (35.0 mg) was taken and dissolved in 20 mM histidine (7.8 mL, pH = 6.5), 10 mM TCEP solution (477 µL, 20 equiv.) was added, and mixed uniformly, then reacted at 37 °C for 2 hours. RP-HPLC confirmed that all interchain disulfide bonds were reduced, DMSO (457 µL) and mc-GGFG-DXd in DMSO (321 µL, 10 mg/mL) were added sequentially, and mixed uniformly, then reacted at 25 °C for 1 hour. After the reaction was completed, small-molecule impurities were removed by purification using a desalting column, and the solution was replaced with the 20 mM histidine buffer, pH = 6.5, to obtain the objective ADC product Patritumab-DXd (29 mg, 83%).

Patritumab-DXd (50 µg) was taken and diluted to 1 mg/mL by adding H₂O, and the RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |

| Time | 30 min | |
|---|---|---|
| Time (min) | Mobile phase A% | Mobile phase B% |
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The RP-HPLC assay result is shown in FIG. 8, and the calculated average DAR value is 7.75.

Patritumab-DXd (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 9, the proportion of high molecular weight components and monomer ADC were 0.22% and 99.78%, respectively, and no low molecular weight fraction was observed.

### Example 6: Synthesis and characterization of Duligo-DXd

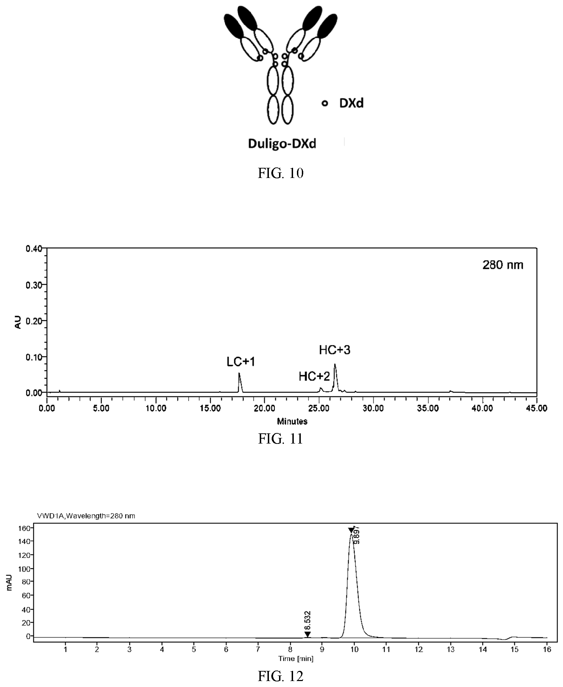

The molecular structure of Duoligo-DXd is shown in FIG. 10.

Duligotuzumab (20.0 mg) was taken and dissolved in 20 mM histidine (6.7 mL, pH = 6.5), 10 mM TCEP solution (276 µL, 20 equiv.) was added, and mixed uniformly, then reacted at 37 °C for 2 hours. RP-HPLC confirmed that all interchain disulfide bonds were reduced, DMSO (481 µL) and mc-GGFG-DXd in DMSO (186 µL, 10 mg/mL) were added sequentially, and mixed uniformly, then reacted at 25 °C for 1 hour. After the reaction was completed, small-molecule impurities were removed by purification using a desalting column, and the solution was replaced with 20 mM histidine buffer, pH = 6.5, to obtain the objective ADC product duoligo-DXd (17.1 mg, 85%).

Duligo-DXd (50 µg) was taken and diluted to 1 mg/mL by adding H₂O, and the RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 45 min | |

| Time (min) | Mobile phase A% | Mobile phase B% |
|---|---|---|
| 0 | 75 | 25 |
| 3 | 75 | 25 |
| 5 | 70 | 30 |
| 35 | 58 | 42 |
| 40 | 10 | 90 |
| 41 | 75 | 25 |
| 45 | 75 | 25 |

The RP-HPLC assay result is shown in FIG. 11, and the calculated average DAR value is 7.70.

Duligo-DXd (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 12, the proportion of high molecular weight components and monomer ADC were 0.80% and 99.20%, respectively, and no low molecular weight fraction was observed.

### Example 7: Synthesis and characterization of IgG1LALA-NT3 (DAR = 8)

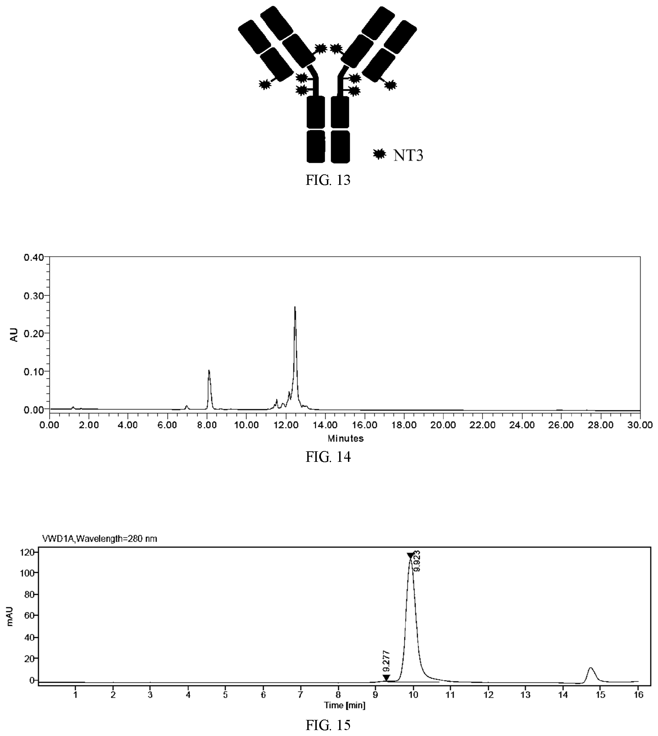

The molecular structure of IgG1LALA-NT3 (DAR = 8) is shown in FIG. 13.

IgG1LALA (20.0 mg) was taken and dissolved in 20 mM histidine (5.0 mL, pH = 6.5), 10 mM TCEP solution (271 µL, 20 equiv.) was added, and mixed uniformly, then reacted at 37 °C for 2 hours. RP-HPLC confirmed that all interchain disulfide bonds were reduced, DMSO (300 µL) and NT3 in DMSO (200 µL, 10 mg/mL) were added sequentially, and mixed uniformly, then reacted at 25 °C for 1 hour. After the reaction was completed, small-molecule impurities were removed by purification using a desalting column, and the solution was replaced with 20 mM histidine buffer, pH = 6.5, to obtain the objective ADC product IgG1LALA-NT3 (DAR = 8) (18.2 mg, 91%).

IgG1LALA-NT3 (DAR = 8) (50 µg) was taken and diluted to 1 mg/mL by adding H₂O, and the RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 30 min | |

| Time (min) | Mobile phase A% | Mobile phase B% |
|---|---|---|
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The RP-HPLC assay result is shown in FIG. 14, and the calculated average DAR value is 7.75.

IgG1LALA-NT3 (DAR = 8) (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 15, the proportion of high molecular weight components and monomer ADC were 1.09% and 98.91%, respectively, and no low molecular weight fraction was observed.

### Example 8: Synthesis and characterization of IgG1LALA-NT3 (DAR = 4)

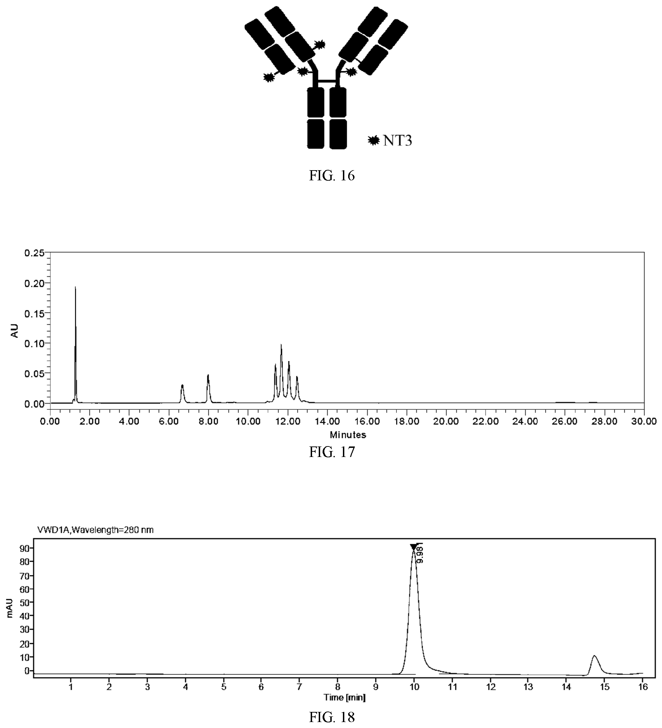

The molecular structure of IgG1LALA-NT3 (DAR = 4) is shown in FIG. 16.

IgG1LALA (1.0 mg) was taken and dissolved in 20 mM histidine (0.25 mL, pH = 6.5), 5 mM TCEP solution (4.06 µL, 3 equiv.) was added, and mixed uniformly, then reacted at 37 °C for 2 hours, DMSO (18.8 µL) and NT3 in DMSO solution (6.2 µL, 10 mg/mL) were added sequentially, and mixed uniformly, then reacted at 25 °C for 1 hour. After the reaction was completed, small-molecule impurities were removed by purification using a desalting column, a sample (50 µg) is taken and diluted to 1 mg/mL by adding H₂O, and the RP-HPLC analysis was performed according to the following method to determine an average DAR.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 45 min | |

| Time (min) | Mobile phase A% | Mobile phase B% |
|---|---|---|
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The above steps were repeated, the DAR of the prepared ADC under the condition that TCEP equivalent is 4.0 and 5.0 were measured respectively, linear relation between the DAR and the TCEP equivalent was fitted, and it is determined that when the TCEP equivalent is 2.5, the theoretical DAR is 4.0. IgG1LALA (40.0 mg) was taken and dissolved in 20 mM histidine (10.0 mL, pH = 6.5), 5 mM TCEP solution (135 µL, 2.5 equiv.) was added, and mixed uniformly, then reacted at 37 °C for 2 hours, DMSO (754 µL) and NT3 in DMSO (246 µL, 10 mg/mL) were added sequentially after cooling to room temperature, and mixed uniformly, then reacted at 25 °C for 1 hour. After the reaction was completed, small-molecule impurities were removed by purification using a desalting column, and the solution was replaced with 20 mM histidine buffer, pH = 6.5, to obtain the objective ADC product IgG1LALA-NT3 (DAR = 4) (35.2 mg, 88%).

The RP-HPLC determination was performed according to the above method, the result is shown in FIG. 17, and the calculated average DAR value is 3.89.

IgG1LALA-NT3 (DAR = 4) (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 18, the proportion of monomer ADC was 100%, and no high molecular weight component and no low molecular weight fraction were observed.

### Example 9: Synthesis and characterization of IgG1LALA-DXd

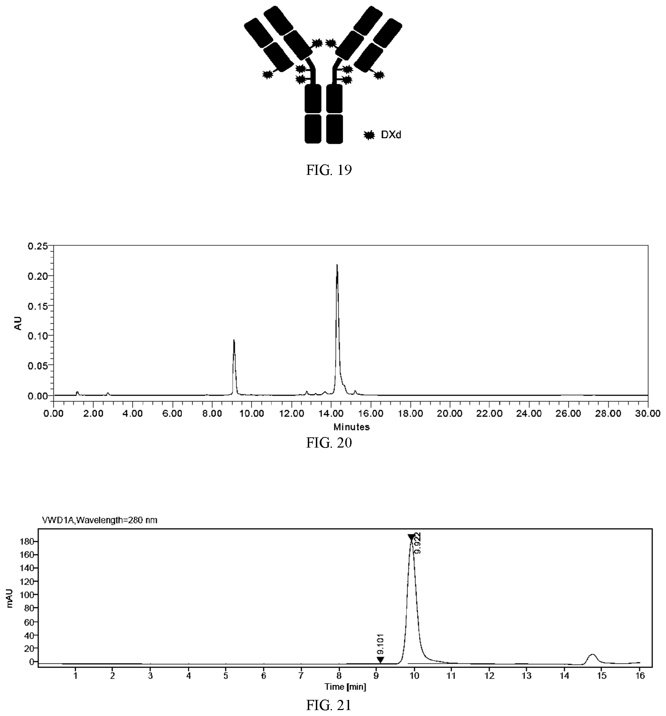

The molecular structure of IgG1LALA-DXd is shown in FIG. 19.

IgG1LALA (15.0 mg) was dissolved in 20 mM histidine (5.0 mL, pH = 6.5), 10 mM TCEP solution (196 µL, 20 equiv.) was added, and mixed uniformly, then reacted at 37 °C for 2 hours. RP-HPLC confirmed that all interchain disulfide bonds were reduced, DMSO (378 µL) and DXd in DMSO solution (122 µL, 10 mg/mL) were added sequentially, and mixed uniformly, then reacted at 25 °C for 1 hour. After the reaction was completed, small-molecule impurities were removed by purification using a desalting column, and the solution was replaced with 20 mM histidine buffer, pH = 6.5, to obtain the objective ADC product IgG1LALA-DXd (DAR = 8) (13.4 mg, 89%).

IgG1LALA-DXd (DAR = 8) (50 µg) was taken and diluted to 1 mg/mL by adding H₂O, and the RP-HPLC analysis was performed according to the following method.

| | | |
|---|---|---|
| Equipment | Waters, ACQuity H class UPLC, SZ-A2-DD-HPLC01 | |
| Column | Waters, BioResolve RP mAb Polyphenyl, 450 Å, 2.7 µm, 2.1 mm × 15 cm | |
| Column temperature | 70°C | |
| Mobile phase | A: 0.1% TFA in H₂O, B: 0.1% TFA in ACN. | |
| Flow rate | 0.3 mL/min | |
| Injection amount | 10 µg | |
| Detecting wavelength | 280 nm; 360 nm | |
| Gradient | Gradient | |
| Time | 30 min | |

| Time (min) | Mobile phase A% | Mobile phase B% |
|---|---|---|
| 0 | 70 | 30 |
| 3 | 70 | 30 |
| 23 | 50 | 50 |
| 24 | 10 | 90 |
| 25 | 10 | 90 |
| 26 | 70 | 30 |
| 30 | 70 | 30 |

The RP-HPLC assay result is shown in FIG. 20, and the calculated DAR value is 7.97.

IgG1LALA-DXd (DAR = 8) (50 µg) was taken and diluted to 3 mg/mL by adding H₂O, and the SEC analysis was performed according to the following method.

| | |
|---|---|
| Equipment | Agilent, 1260, AS-146 |
| Column | TSK gel G3000SWXL, 7.8 mm × 30 cm, 5 µm |
| Column temperature | 25°C |
| Mobile phase | 0.2 M PB, 0.25 M KCl, 15% (v/v) IPA, pH 7.0 |
| Flow rate | 0.75 mL/min |
| Injection amount | 30 µg |
| Detecting wavelength | 280 nm, 360 nm |
| Gradient | Isocratic |
| Time | 16 min |

The SEC assay result is shown in FIG. 21, the proportions of high molecular weight components and monomer ADC were 0.20% and 99.80%, respectively, and no low molecular weight fraction was observed.

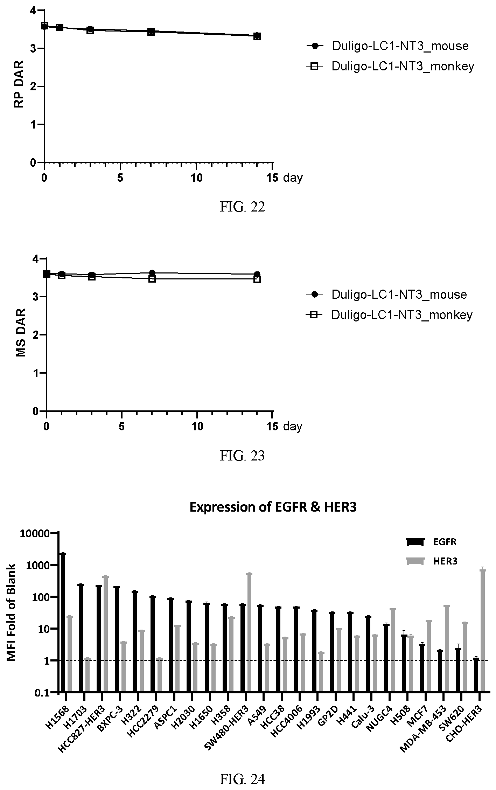

**Example 10: Plasma stability assay of Duligo-LC1-NT3 in mice and monkeys** Duligo-LC1-NT3 (100 µg) was taken and added into mouse plasma or monkey plasma and diluted to 100 µL (5 tubes of samples were prepared for these two types of plasma, respectively), incubated at 37 °C for 0, 1, 3, 7 and 14 days respectively, ADC samples were purified by affinity chromatography for RP-HPLC and LC-MS analysis, and the change trend of the DAR value over time was determined. The results are shown in FIG. 22 and FIG. 23, indicating that Duoligo-LC1-NT3 was very stable in both plasma and no significant drug releasing was observed.

### RP-HPLC analysis

| **Sample** | **Duligo-LC1-NT3** | |
|---|---|---|
| Blood plasma | Mouse plasma | Monkey plasma |
| Day 0 | 3.57 | 3.59 |
| Day 1 | 3.54 | 3.55 |
| Day 3 | 3.51 | 3.47 |
| Day 7 | 3.46 | 3.43 |
| Day 14 | 3.34 | 3.32 |

**LC-MS analysis**

| **Sample** | **Duligo-LC1-NT3** | |
|---|---|---|
| Blood plasma | Mouse plasma | Monkey plasma |
| Day 0 | 3.61 | 3.60 |
| Day 1 | 3.60 | 3.56 |
| Day 3 | 3.58 | 3.53 |
| Day 7 | 3.63 | 3.47 |
| Day 14 | 3.59 | 3.46 |

### Example 11: Detection of the expression level of EGFR & HER3 in different tumor cell lines Detection method:

After 2-3 passages of the resurrected cells, the culture medium was poured out firstly, rinsed with 10 mL PBS (catalogue number: 10010-023, manufacture: Gibco) once, then the cells was digested with 4mL trypsin (catalogue number: 25200-072, manufacturer: Gibco), the culture medium was terminated, then centrifuged at 400xg for 3min, the supernatant was discarded, 2% of FBS (catalogue number: 10091-148, manufacture: Gibco) were pre-configured as FACS Buffers by adding them to PBS, resuspended with FACS Buffer for counting, and the cell density was adjusted to 4x10⁶/mL, 50 µL per well was added into a circular bottom 96-well plate at, centrifuged at 400xg for 5 minutes;

The antibodies PE anti-human EGFR (catalogue number: 352904, manufacturer: Bio Legend) and PE anti-human erbB3/HER-3 (catalogue number:324706, manufacturer: Bio Legend) were diluted with FACS Buffer in a ratio of 1:100, each antibody for two wells, cells in which were resuspended with 50 µL antibody per well, simultaneously, two wells were resuspended with FACS Buffer as a control, and incubated at 4 °C in a dark for 1 h; 150 µL FACS buffer was added into each well, and centrifuged at 400xg for 5min; each well was resuspended with 200 µL FACS buffer followed by centrifuging at 400xg for 5min, finally the cells were resuspended with 100 µL FACS Buffer in each well, the flow detection was carried out, 20000 data collection points were collected in a PE channel for each sample.

The MFI value of the PE channel was analyzed and exported by using software, and then the average was taken, the value of the each well in which the cells were stained with the antibody was divided by the value of the control well, to obtain the corresponding value respectively, and finally plotted.

### Detection results of expression level of EGFR & HER3 in different tumor cell lines:

The diverse expression profiles of EGFR and HER3 in different tumor cell lines were verified by the determination of expression levels of EGFR and HER3 in a series of cell lines, the results are shown in FIG. 24.

### Example 12: Fab-ZAP endocytosis-killing experiment of antibody molecules

### Detection method:

After 2-3 passages of the resurrected AspC1 cells, the culture medium was poured out firstly, rinsed with 10 mL PBS once, then the cells were digested with 4mL trypsin, the culture medium was terminated, then centrifuged at 400xg for 3min, the supernatant was discarded, and the culture medium was further resuspended for counting. The cell density was adjusted to 3E4/mL, 50 µL of which per well was added into a flat-bottom 96-well plate. The antibody to be detected was diluted to 160 nM (the highest using concentration was 40 nM) with a complete culture medium, Fab-ZAP was diluted to 640nM (the highest using concentration was 160nM, Fab-ZAP: antibody = 4:1), then an equal volume of antibody was taken and mixed with Fab-ZAP in a ratio of 1:1, which was used as the initial concentration, after gradient diluted 10 wells in a ratio of 1/4, incubated at room temperature for 30 min, 50 µL of which per well was added into the cells, mixed uniformly, cultured at 37 °C for 5 days, then the 96-well plate was taken out, the plate was balanced to room temperature, 100 µL of CTG detection solution was added into each well, placed at a shaker at 300 rpm for 20 min, 100 µL of which was absorbed and placed into the white bottom 96-well plate, and the luminescence value was detected by using a multifunctional microplate reader.

### Results of Fab-ZAP endocytosis experiment:

Fab-ZAP as a chemical conjugate of a mAb and ribosome inactivation protein saporin, recognized the Fc terminus of the target antibody through its F(ab) fragment, and conjugated with the antibody, then the ZAP compound was guided by the antibody to reach the target cells, and followed by combined with the target cells and endocytosis, the ZAP compound entered into cytoplasm and then released saporin upon enzymolysis, normal function of ribosome was influenced so as the cells were kill, and finally the endocytosis rate of the antibody was calculated by detecting the killing efficiency of the antibody compound on the cells.

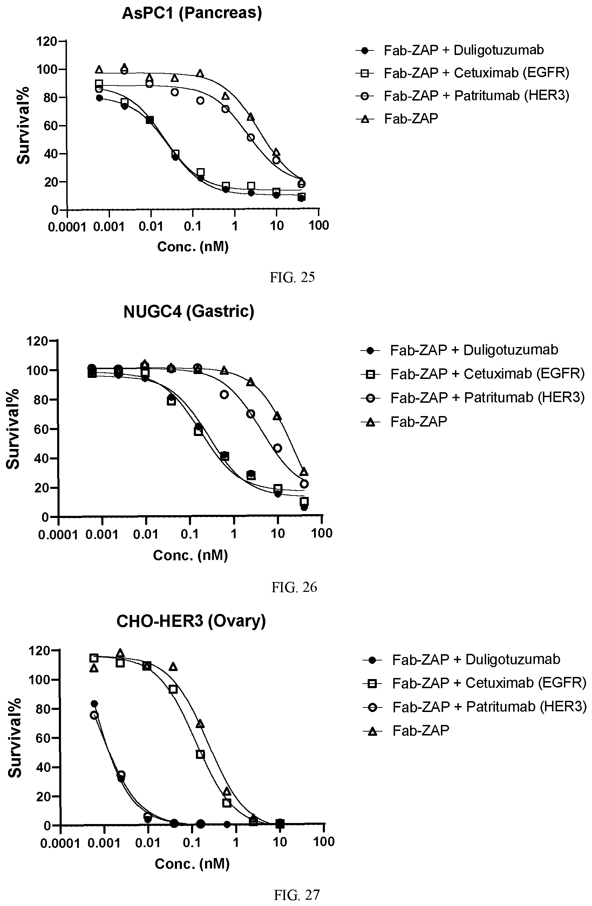

Duligutemab and Cetuximab exhibited a stronger endocytosis effect than Patritumab on human pancreatic cancer cells AsPC1 with higher expression of both EGFR and HER3 (MFI-fold (EGFR) = 186, MFI-fold (HER3) =12.4), with approximately 80-fold reduction in IC₅₀ (the IC₅₀ of duligutemab is 0.028 nM, the IC₅₀ of Cetuximab is 0.021 nM, the IC₅₀ of Patritumab is 2.127 nM), as shown in FIG. 25.

Duligutemab and Cetuximab exhibited a stronger endocytosis effect than Patritumab on the human gastric cancer cells NUGC4 with moderate expression of both EGFR and HER3 (MFI-fold (EGFR) = 14.7, MFI-fold (HER3) =44.9), as shown in FIG. 26; Duligotuzumab and Patritumab exhibited a stronger endocytosis effect than Cetuximab on chinese hamster ovary cells CHO-HER3 with no expression of EGFR and overexpression of HER3, as shown in FIG. 27.

### Example 13: The in vitro cell killing assay of antibody drug conjugates Duligo-DXd and SIB001-DXd

### Detection method:

After 2-3 passages of the resurrected MCF7 cells, the culture medium was poured out firstly, rinsed with 10 mL PBS once, then the cells were digested with 4mL trypsin, the culture medium was terminated before centrifuged at 400xg for 3min, the supernatant was discarded, and the culture medium was further resuspended for counting. The cell density was adjusted to 3E4/mL, 50 µL of which per well was added into a round bottom low adsorption 96-well plate. The antibody to be detected was diluted in a gradient manner, then 50 µL of which per well was added into the corresponding cells, cultured at 37 °C for 5 days, then the 96-well plate was taken out, the plate was balanced to room temperature, 100 µL of CTG detection solution was added into each well, placed at a shaker at 300 rpm for 20 min, 100 µL of which was absorbed and placed into the white bottom 96-well plate, and the luminescence value was detected by using a multifunctional microplate reader.

### Experiment results of the in vitro cell killing:

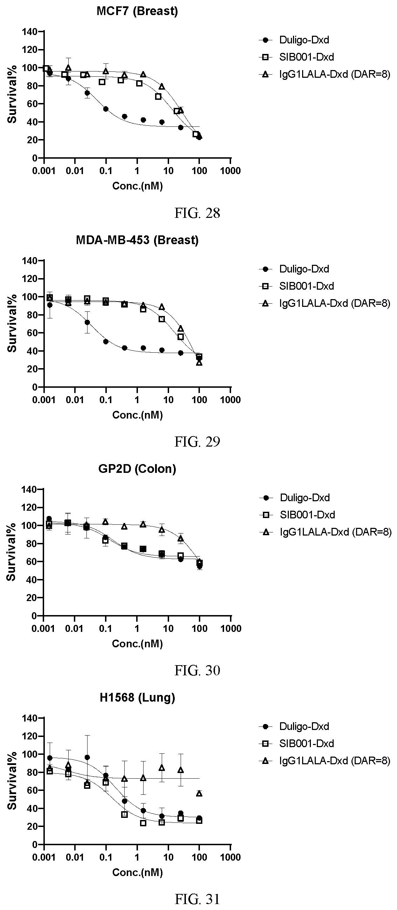

Duligo-DXd exhibited a significantly stronger killing effect than SIB001-DXd on the human breast cancer cells MCF7 with lower expression of EGFR and higher expression of HER3 (MFI-fold (EGFR) = 3.3, MFI-fold (HER3) = 19.1), with approximately 250-fold reduction in IC₅₀ (the IC₅₀ of Duligo-DXd is 0.049 nM, the IC₅₀ of SIB001-DXd is 13.23 nM), as shown in FIG. 28.

Duligo-DXd exhibited a significantly stronger killing effect than SIB001-DXd on the human breast cancer such as MDA-MB-453 cells, as shown in FIG. 29; Duligo-DXd exhibited a similar *in **vitro*** killing effects with SIB001-DXd on human colorectal cancer cells GP2D, human lung cancer cells H1568 and human colorectal cancer cells SW480 with overexpression of HER3, as shown in FIGS. 30-32.

### Example 14: The in vitro cell killing assay of antibody drug conjugates Duligo-LC1-NT3 and SIB001-DXd

### Detection method:

After 2-3 passages of the resurrected MDA-MB-453 cells, the culture medium was poured out firstly, rinsed with 10 mL PBS once, then the cells were digested with 4mL trypsin, the culture medium was terminated, then centrifuged at 400xg for 3min, the supernatant was discarded, and the culture medium was further resuspended for counting. The cell density was adjusted to 3E4/mL, 50 µL of which per well was added into a round bottom low adsorption 96-well plate; The antibody to be detected was diluted in a gradient manner, then 50 µL of which per well was added into the corresponding cells, cultured at 37 °C for 5 days, then the 96-well plate was taken out, the plate was balanced to room temperature, 100 µL of CTG detection solution was added into each well, placed at a shaker at 300 rpm for 20 min, 100 µL of which was absorbed and placed into the white bottom 96-well plate, and the luminescence value was detected by using a multifunctional microplate reader.

### Experiment results of the in vitro cell killing:

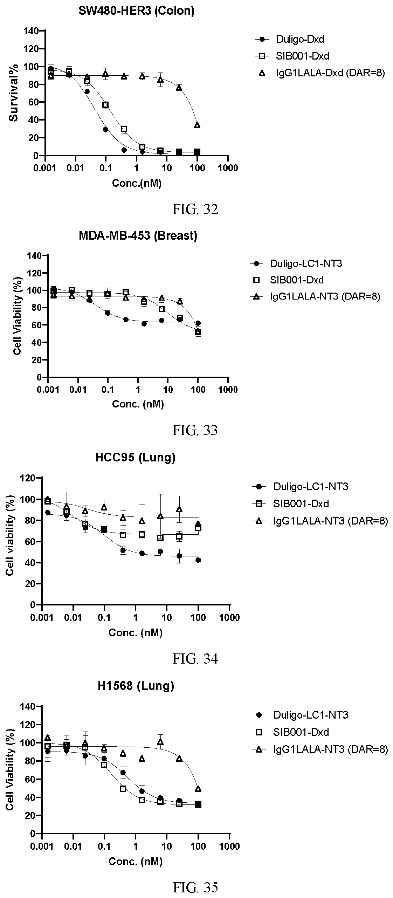

Duligo-LC1-NT3 exhibited a significantly killing effect than SIB001-DXd on the human breast cancer cells MDA-MB-453 with lower expression of EGFR and higher expression of HER3 (MFI-fold (EGFR) = 2.2, MFI-fold (HER3) =55.2), with approximately 250-fold reduction in IC₅₀ (the IC₅₀ of Duligo-LC1-NT3 is0.037 nM, the IC₅₀ of SIB001-DXd is 9.36 nM), as shown in FIG. 33.

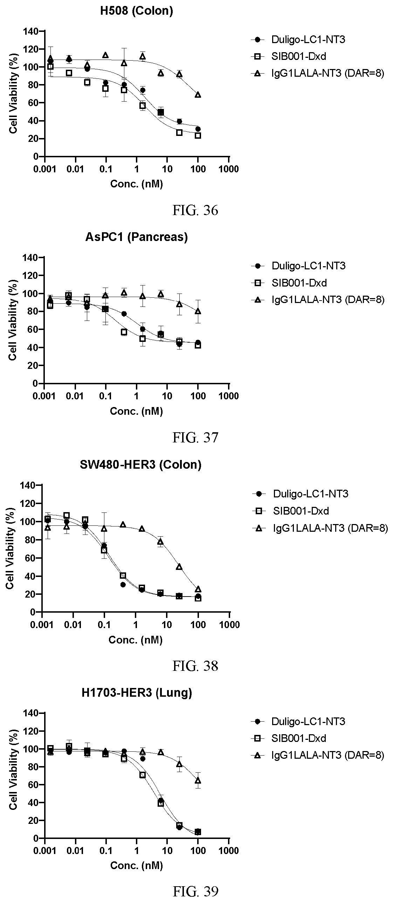

Duligo-LC1-NT3 (DAR = 4) and SIB001-DXd (DAR = 8) exhibited a similar *in vitro* killing activity on lung cancer, colorectal cancer, pancreatic cancer cell lines, as well as some cell lines with overexpression of HER3, as shown in FIGS. 34-39.

### Example 15: ELISA detection of the affinity of Duligo-LC1-NT3 and SIB001-DXd on human EGFR and human HER3

### Affinity detection method:

A 96-well enzyme label plate was coated one day in advance, EGFR or HER3 antigen was diluted to 0.25 µg/mL by using coating solution, then 100 µL of which was added into each well, the plate was sealed by a plate film, and placed at 4 °C overnight; the pre-coating solution in the 96-well enzyme label plate was poured out, patted dry on absorbent paper, 230 µL of washing solution was added into each well, and washed for 3 times repeatedly after patting dry the washing solution; 200 µL of blocking solution was added into each well by the multichannel pipette gun, the plate was sealed by a microplate sealer, blocked at 37 °C for 2h, then the plate was washed; the sample to be detected was diluted at the maximum concentration of 100 nM and a ratio of 1:4 into 12 wells, and then 100 µL of dilutions was added into each well, incubated at room temperature for 2 hours in the dark, the plate was washed, 100µL of Goat anti-Human IgG Fc-HRP diluting at 1:80000 was added into each well, incubated at room temperature for 1 hour in the dark, and the plate was washed; 100 µL of TMB substrate was added into a 96-well enzyme label plate, developed for 2-5 minutes at room temperature in a dark, then 50 µL of ELISA stop solution was added into each well, and the values of OD₄₅₀ₙₘ and OD₆₂₀ₙₘ were read within 30 minutes.

### Experiment result of the affinity:

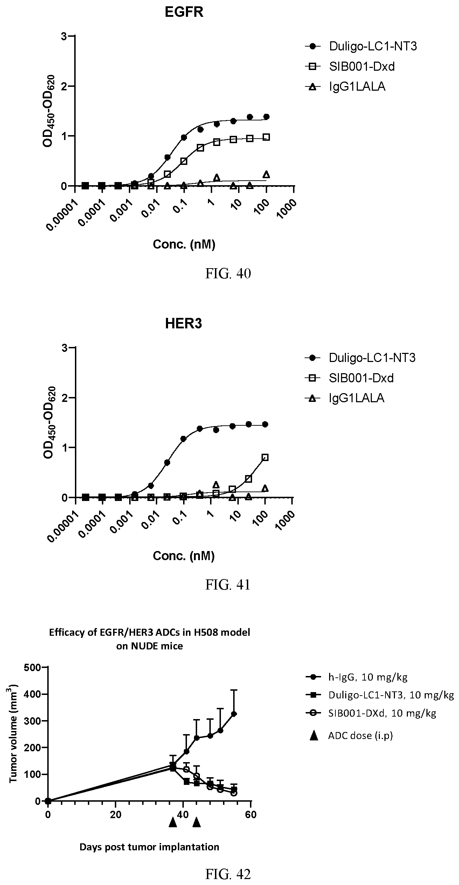

Duligo-LC1-NT3 exhibited a stronger affinity for EGFR than SIB001-DXd, with an approximately 2.5-fold decrease in Kd (Kd (Duligo-LC1-NT3) = 0.03517 nM, Kd (SIB001-DXd) = 0.08833 nM), as shown in FIG. 40; Duligo-LC1-NT3 exhibited a stronger affinity for HER3 (Kd (Duligo-LC1-NT3) = 0.02574 nM), SIB001-DXd exhibited a weak binding to HER3, as shown in FIG. 41.

### Example 16: The therapeutic effect of EGFR/HER3-ADCs in the H508 model

In the present experiment, the BALB/c NUDE mice inoculated with H508 cells were used for determining the anti-tumor effect of the EGFR/HER3-ADCs.

### BALB/c NUDE mice:

The female BALB/c NUDE background mice were purchased from Beijing Vital River Laboratory Animal Technology Co., Ltd, with the grade of SPF, the quality control department is Beijing Vital River Laboratory Animal Technology Co., Ltd, and the certificate number was NO. 110011230103807467. The mice were domesticated for 3 days after arrival, and then the study began.

### Cells:

H508 cells were purchased from Nanjing Cobioer (CAT#: CBP60795) and routinely subcultured in strict accordance with the instructions for subsequent *in vivo* experiments. The cells were collected by centrifugation, resuspended in sterile PBS and adjusted to a cell density of 25×10⁶ cells/ml. A H508 tumor-bearing mouse model was established by subcutaneous inoculation of 0.2 ml of cell suspension into the right abdominal region of BALB/c nude mice on day 0.

### Administration:

Tumor volume of each mouse was measured 37 days after tumor cell inoculation, the mice with tumor volumes between about 120-140 mm³ were selected and grouped on average according to their tumor volume (6 mice per group). The dosages and modes of administration are shown in Table 1, h-IgG (purchased from EQUITECH-BIO, SLH56-0001) was used as a negative control. Administrations were conducted on day 37 and day 44 after inoculation respectively, and the tumor volume and body weight of mice were monitored twice a week. Body weight and tumor volume were measured before each dose, and the relative tumor growth inhibition (TGI%) was calculated on day 55 after inoculation, the calculating formula was as follows: TGI% =100% * (tumor volume of control group -tumor volume of treatment group)/ (tumor volume of control group-tumor volume of control group before administration). Measurement of tumor volume: the maximum long axis (L) and maximum wide axis (W) of the tumor were measured using a vernier caliper, and the tumor volume was calculated as follows: V=L×W²/2. Body weight was measured using an electronic balance.

**Table 1. Experimental design**

| Groups | Administration Dosage | Administration frequency | Administration mode |
|---|---|---|---|
| h-IgG, 10 mg/kg | 10 mg/kg | QWx2 | Intraperitoneal injection |
| Duligo-LC1-NT3 | 10 mg/kg | QWx2 | Intraperitoneal injection |
| SIB001-DXd | 10 mg/kg | QWx2 | Intraperitoneal injection |

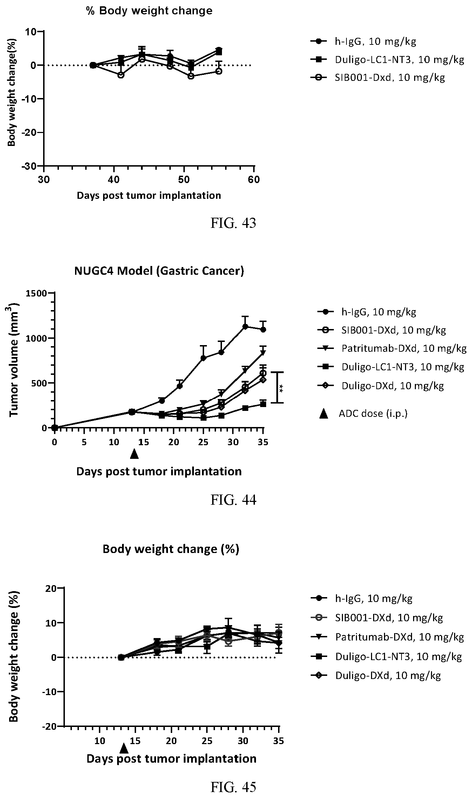

The tumor growth inhibition of Duligo-LC1-NT3 group was 147%, and the tumor growth inhibition of SIB001-DXd group was 155%. The tumor growth inhibition of SIB001-DXd group was similar to that of Duligo-LC1-NT3 group, both of which were stronger than the tumor growth inhibition of h-IgG alone group. The result of efficacy is shown in FIG. 42. Meanwhile, the body weight of the mice was monitored, and the result is shown in FIG. 43, and there was no significant difference in the body weights of the mice. Therefore, the EGFR/HER3-ADCs medicament exhibited a significant inhibitory effect on tumors.

**Table 2. Tumor growth inhibition on day 55**

| Groups | Tumor volume (mm³) | TGI (%) |
|---|---|---|
| h-IgG, 10 mg/kg | 326 | N/A |
| Duligo-LC1-NT3, 10 mg/kg | 44 | 147 |
| SIB001-DXd, 10 mg/kg | 30 | 155 |

### Example 17: The therapeutic effect of EGFR-HER3 ADCs in NUGC-4 model

In the present experiment, the BALB/c NUDE mice inoculated with NUGC-4 cells were used for determining the anti-tumor effect of the EGFR/HER3-ADCs.

### BALB/c-NUDE mice:

The female BALB/c-NUDE mice were purchased from Beijing Vital River Laboratory Animal Technology Co., Ltd, with the grade of SPF, the quality control department is Beijing Vital River Laboratory Animal Technology Co., Ltd, and the certificate number was NO.110011230103807533. The mice were domesticated for 3 days after arrival, and then the study began.

### Cells:

NUGC-4 cells were purchased from NIBIOHN (CAT #: JCRB0834) and routinely subcultured in strict accordance with the instructions for subsequent *in vivo* experiments. The cells were collected by centrifugation, resuspended in sterile PBS and adjusted to a cell density of 20×10⁶ cells/ml. A NUGC-4 tumor-bearing mouse model was established by subcutaneous inoculation of 0.2 ml of cell suspension into the right abdominal region of BALB/c-NUDE mice on day 0.

### Administration:

Tumor volume of each mouse was measured 13 days after tumor cell inoculation, the mice with tumor volumes about 200 mm³ were selected and grouped on average according to their tumor volume (7 mice per group). The dosages and modes of administration are shown in Table 3, h-IgG (purchased from EQUITECH-BIO, SLH56-0001) was used as a negative control. Administrations were conducted on day 13 after inoculation respectively, and the tumor volume and body weight of mice were monitored twice a week. Body weight and tumor volume were measured before each dose, and the relative tumor growth inhibition (TGI%) was calculated on day 32 after inoculation, the calculating formula was as follows: TGI% =100% * (tumor volume of control group - tumor volume of treatment group)/ (tumor volume of control group - tumor volume of control group before administration). Measurement of tumor volume: the maximum long axis (L) and maximum wide axis (W) of the tumor were measured using a vernier caliper, and the tumor volume was calculated as follows: V=L×W²/2. Body weight was measured using an electronic balance.

**Table 3. Experimental design**

| Groups | Administration dosage | Administration frequency | Administration mode |
|---|---|---|---|
| h-IgG | 10 mg/kg | Single dose | Intraperitoneal injection |
| SIB001-DXd | 10 mg/kg | Single dose | Intraperitoneal injection |
| Patritumab-DXd | 10 mg/kg | Single dose | Intraperitoneal injection |
| Duligo-LC1-NT3 | 10 mg/kg | Single dose | Intraperitoneal injection |
| Duligo-DXd | 10 mg/kg | Single dose | Intraperitoneal injection |

Results of tumor growth inhibition are shown in FIG. 44 and Table 4: on day 32 after inoculation, the tumor growth inhibition of Patritumab-DXd/ SIB001-DXd/ Duligo-LC1-NT3/Duligo-DXd were: 51% / 70% / 95% / 75%, respectively. Duligo-LC1-NT3 was superior in efficacy than SIB001-DXd and Duligo-DXd with statistical difference. Meanwhile, the body weights of the mice were measured, and the result is shown in FIG. 45, and there was no significant difference in the body weights of the mice. Therefore, EGFR/HER3 ADCs exhibited a significant inhibitory effect on NUGC-4 tumors.

**Table 4. Tumor growth inhibition on day 32**

| Groups | Tumor volume (mm³) | TGI (%) |
|---|---|---|
| h-IgG, 10 mg/kg | 1127 | / |
| SIB001-DXd, 10 mg/kg | 457 | 70 |
| Patritumab-DXd, 10 mg/kg | 636 | 51 |
| Duligo-LC1-NT3, 10 mg/kg | 220 | 95 |
| Duligo-DXd, 10 mg/kg | 414 | 75 |

### Example 18: The therapeutic effect of EGFR-HER3 ADCs in SW620 model

In the present experiment, the BALB/c NUDE mice inoculated with SW620 cells were used for determining the anti-tumor effect of the EGFR/HER3-ADCs.

### BALB/c-NUDE mice:

The female BALB/c-NUDE mice were purchased from Beijing Vital River Laboratory Animal Technology Co., Ltd, with the grade of SPF, the quality control department is Beijing Vital River Laboratory Animal Technology Co., Ltd, and the certificate number was 20230614Abzz0619000190. The mice were domesticated for 3 days after arrival, and then the study began.

### Cells:

SW620 cells were obtained Nanjing Cobioer Biotechnology Co., Ltd. The cells were collected by centrifugation, resuspended in sterile PBS and adjusted to a cell density of 10×10⁶ cells/ml. A SW620 tumor-bearing mouse model was established by subcutaneous inoculation of 0.2 ml of cell suspension into the right abdominal region of BALB/c-NUDE mice on day 0.

### Administration:

Tumor volume of each mouse was measured 14 days after tumor cell inoculation, the mice with tumor volumes about 200 mm³ were selected and grouped on average according to their tumor volume (7 mice per group). The dosages and modes of administration are shown in Table 5, h-IgG (purchased from EQUITECH-BIO, SLH56-0001) was used as a negative control. Administrations were conducted on day 14, 21 and 28 after inoculation respectively, and tumor volume and body weight of mice were monitored twice a week. Body weight and tumor volume were measured before each dose, and the relative tumor growth inhibition (TGI%) was calculated on day 42 after inoculation, the calculating formula was as follows: TGI% =100% * (tumor volume of control group - tumor volume of treatment group)/ (tumor volume of control group -tumor volume control group before administration). Measurement of tumor volume: the maximum long axis (L) and maximum wide axis (W) of the tumor were measured using a vernier caliper, and the tumor volume was calculated as follows: V=L×W²/2. Body weight was measured using an electronic balance.

**Table 5. Experimental design**

| Groups | Administration dosage | Administratio n frequency | Administration mode |
|---|---|---|---|
| h-IgG | 10 mg/kg | QWx3 | Intraperitoneal injection |
| IgG1LALA-NT3 (DAR=4) | 10 mg/kg | QWx3 | Intraperitoneal injection |
| SIB001-DXd | 10 mg/kg | QWx3 | Intraperitoneal injection |
| Duligo-LC1-NT3 | 10 mg/kg | QWx3 | Intraperitoneal injection |

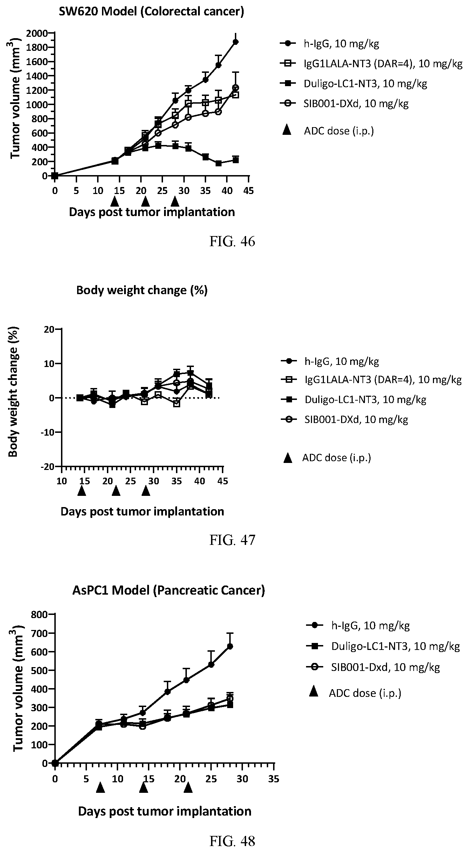

Results of tumor growth inhibition are shown in FIG. 46 and Table 6: on day 42 after inoculation, the tumor growth inhibition of IgG1LALA-NT3 (DAR=4) / SIB001-DXd / Duligo-LC1-NT3 were: 45% / 39% / 100%, respectively. Duligo-LC1-NT3 was superior in efficacy than SIB001-DXd. Meanwhile, the body weights of the mice were measured, and the result is shown in FIG. 47, and there was no significant difference in the body weights of the mice. Therefore, EGFR/HER3 ADCs exhibited a significant inhibitory effect on SW620 tumors.

**Table 6. Tumor growth inhibition on day 42**

| Groups | Tumor volume (mm³) | TGI (%) |
|---|---|---|
| h-IgG, 10 mg/kg | 1877 | / |
| IgG1LALA-NT3 (DAR=4), 10 mg/kg | 1134 | 45 |
| Duligo-LC1-NT3, 10 mg/kg | 220 | 100 |
| SIB001-DXd, 10 mg/kg | 1235 | 39 |

### Example 19: The therapeutic effect of EGFR-HER3 ADCs in ASPC1 model

In the present experiment, the BALB/c NUDE mice inoculated with ASPC1 cells were used for determining the anti-tumor effect of the EGFR/HER3-ADCs.

### BALB/c-NUDE mice:

The female BALB/c-NUDE mice were purchased from Beijing Vital River Laboratory Animal Technology Co., Ltd, with the grade of SPF, the quality control department is Beijing Vital River Laboratory Animal Technology Co., Ltd, and the certificate number was 20230531Abzz0619000787. The mice purchased from Vital River laboratory mice were domesticated for 3 days after arrival, and then the study began.

### Cells:

ASPC1 cells were obtained Nanjing Cobioer Biotechnology Co., Ltd, and routinely subcultured in strict accordance with the instructions for subsequent *in vivo* experiments. The cells were collected by centrifugation, resuspended in sterile PBS and adjusted to a cell density of 25×10⁶ cells/ml. A ASPC1 tumor-bearing mouse model was established by subcutaneous inoculation of 0.2 ml of cell suspension into the right abdominal region of BALB/c-NUDE mice on day 0.

### Administration:

Tumor volume of each mouse was measured 7 days after tumor cell inoculation, the mice with tumor volumes about 200 mm³ were selected and grouped on average according to their tumor volume (7 mice per group). The dosages and modes of administration are shown in Table 7, h-IgG (purchased from EQUITECH-BIO, SLH56-0001) was used as a negative control. Administrations were conducted on day 7, 14 and 21 after inoculation respectively, and the tumor volume and body weight of mice were monitored twice a week. Body weight and tumor volume were measured before each dose, and the relative tumor growth inhibition (TGI%) was calculated on day 28 after inoculation, the calculating formula was as follows: TGI% =100% * (tumor volume of control group - tumor volume of treatment group)/ (tumor volume of control group - tumor volume of control group before administration). Measurement of tumor volume: the maximum long axis (L) and maximum wide axis (W) of the tumor were measured using a vernier caliper, and the tumor volume was calculated as follows: V=L×W²/2. Body weight was measured using an electronic balance.

**Table 7. Experimental design**

| Groups | Administration dosage | Administration frequency | Administration mode |
|---|---|---|---|
| h-IgG | 10 mg/kg | QWx3 | Intraperitoneal injection |
| SIB001-DXd | 10 mg/kg | QWx3 | Intraperitoneal injection |
| Duligo-LC1-NT3 | 10 mg/kg | QWx3 | Intraperitoneal injection |

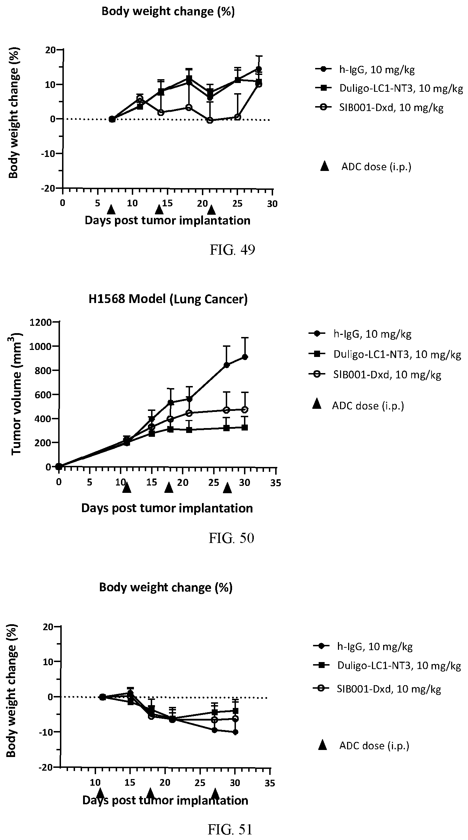

Results of tumor growth inhibition are shown in FIG. 48 and Table 8: on day 28 after inoculation, the tumor growth inhibition of SIB001-DXd / Duligo-LC1-NT3 were: 67% / 75%, respectively. Duligo-LC1-NT3 was superior in efficacy than SIB001-DXd. Meanwhile, the body weights of the mice were measured, and the result is shown in FIG. 49, and there was no significant difference in the body weights of the mice. Therefore, EGFR/HER3 ADCs exhibited a significant inhibitory effect on ASPC1 tumors.

**Table 8. Tumor growth inhibition on day 28**

| Groups | Tumor volume (mm³) | TGI (%) |
|---|---|---|
| h-IgG, 10 mg/kg | 629 | / |
| SIB001-DXd, 10 mg/kg | 347 | 67 |
| Duligo-LC1-NT3, 10 mg/kg | 314 | 75 |

### Example 20: The therapeutic effect of EGFR-HER3 ADCs in NCI-H1568 model

In the present experiment, the BALB/c NUDE mice inoculated with NCI-H1568 cells were used for determining the anti-tumor effect of the EGFR/HER3-ADCs.

### BALB/c-NUDE mice:

The female BALB/c-NUDE mice were purchased from Beijing Vital River Laboratory Animal Technology Co., Ltd, with the grade of SPF, the quality control department is Beijing Vital River Laboratory Animal Technology Co., Ltd, and the certificate number was NO. 110011230103807467. The mice were domesticated for 3 days after arrival, and then the study began.

### Cells:

NCI-H1568 cells were purchased from Nanjing Cobioer Biotechnology Co., Ltd. and routinely subcultured in strict accordance with the instructions for subsequent *in vivo* experiments. The cells were collected by centrifugation, resuspended in sterile PBS and adjusted to a cell density of 25×10⁶ cells/ml. An NCI-H1568 tumor-bearing mouse model was established by subcutaneous inoculation of 0.2 ml of cell suspension into the right abdominal region of BALB/c-NUDE mice on day 0.

### Administration:

Tumor volume of each mouse was measured 11 days after tumor cell inoculation, the mice with tumor volumes about 200 mm³ were selected and grouped on average according to their tumor volume (7 mice per group). The dosages and modes of administration shown in Table 9, h-IgG (purchased from EQUITECH-BIO, SLH56-0001) was used as a negative control. Administrations were conducted on day 11, 18 and 27 after inoculation respectively, and tumor volume and body weight of mice were monitored twice a week. Body weight and tumor volume were measured before each dose, and the relative tumor growth inhibition (TGI%) was calculated on day 30 after inoculation, the calculating formula was as follows: TGI% =100% * (tumor volume of control group - tumor volume of treatment group)/ (tumor volume of control group - tumor volume of control group before administration). Measurement of tumor volume: the maximum long axis (L) and maximum wide axis (W) of the tumor were measured using a vernier caliper, and the tumor volume was calculated as follows: V=L×W²/2. Body weight was measured using an electronic balance.

**TABLE 9. Experimental design**

| Groups | Administration dosage | Administration frequency | Administration mode |
|---|---|---|---|
| h-IgG | 10 mg/kg | QWx3 | Intraperitoneal injection |
| SIB001-DXd | 10 mg/kg | QWx3 | Intraperitoneal injection |
| Duligo-LC1-NT3 | 10 mg/kg | QWx3 | Intraperitoneal injection |

Results of tumor growth inhibition are shown in FIG. 50 and table 10: on day 30 after inoculation, the tumor growth inhibition of SIB001-DXd / Duligo-LC1-NT3 were: 61% / 81%, respectively. Duligo-LC1-NT3 was superior in efficacy than SIB001-DXd. Meanwhile, the body weights of the mice were measured, and the result is shown in FIG. 51, and the body weights of the mice in the administration group were slightly reduced. Therefore, EGFR/HER3 ADCs exhibited a significant inhibitory effect on NCI-H1568 tumors.

**Table 10. Tumor growth inhibition on day 30**

| Groups | Tumor volume (mm³) | TGI (%) |
|---|---|---|
| h-IgG, 10 mg/kg | 917 | / |
| SIB001-DXd, 10 mg/kg | 480 | 61 |
| Duligo-LC1-NT3, 10 mg/kg | 332 | 81 |

The amino acid sequences of the antibody molecules referred herein, as well as the nucleic acid sequences encoding the antibody molecules, are as follows.
**EGFR/HER3 bispecific antibody HCDR1 amino acid sequence SEQ ID NO: 1**
   GFTLSGDWIH
**EGFR/HER3 bispecific antibody HCDR2 amino acid sequence SEQ ID NO: 2**
   EISAAGGYTDYADSVKG
**EGFR/HER3 bispecific antibody HCDR3 amino acid sequence SEQ ID NO: 3**
   ESRVSFEAAMDY
**EGFR/HER3 bispecific antibody LCDR1 amino acid sequence SEQ ID NO: 4**
   RASQNIATDVA
**EGFR/HER3 bispecific antibody LCDR2 amino acid sequence SEQ ID NO: 5**
   SASFLYS
**EGFR/HER3 bispecific antibody LCDR3 amino acid sequence SEQ ID NO: 6**
   QQSEPEPYT
**EGFR/HER3 bispecific antibody heavy chain variable region amino acid sequence SEQ ID NO: 7**
**EGFR/HER3 bispecific antibody heavy chain variable region nucleotide coding sequence SEQ ID NO: 8** 
GAAGTGCAACTCGTCGAGAGCGGCGGGGGCCTGGTGCAGCCGGCGGCTCCCTGAGA
CTGAGCTGCGCCGCTAGCGGCTTCACCCTGAGCGGCGACTGGATCCACTGGGTGCGGCAA
GCCCCTGGCAAGGGCCTCGAGTGGGTCGGGGAAATTAGCGCCGCTGGCGGCTACACCGAC
TACGCCGACAGCGTGAAGGGCAGATTCACCATCAGCGCCGACACAAGCAAGAACACCGCC
 
TACCTGCAGATGAACAGCCTGAGAGCCGAGGACACCGCCGTGTACTACTGCGCTAGAGAG
AGCAGAGTGAGCTTCGAGGCCGCCATGGACTACTGGGGCCAAGGCACCCTGGTGACCGTG
AGCAGC
**EGFR/HER3 bispecific antibody light chain variable region amino acid sequence SEQ ID NO: 9**
**EGFR/HER3 bispecific antibody light chain variable region nucleotide coding sequence SEQ ID NO**: **10** 
GACATTCAGATGACACAGAGCCCTAGCAGCCTGAGCGCTAGCGTGGGCGACAGAGTG
ACCATCACCTGCAGAGCTAGCCAAAACATCGCCACCGACGTGGCCTGGTATCAGCAGAAGC
CCGGCAAGGCCCCCAAGCTGCTGATCTACAGCGCTAGCTTCCTGTACAGCGGCGTGCCTAG
CAGATTCAGCGGCAGCGGCAGCGGCACCGACTTCACCCTGACCATCAGCAGCCTGCAGCC
CGAGGACTTCGCCACCTACTACTGTCAGCAGAGCGAGCCCGAGCCCTACACCTTCGGCCAA
GGCACCAAGGTGGAGATCAAG
**EGFR/HER3 bispecific antibody heavy chain constant region amino acid sequence SEQ ID NO: 11 (only LALA mutation, no cysteine mutation)** 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPEAAGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMHE
ALHNHYTQKSLSLSPGK
**EGFR/HER3 bispecific antibody heavy chain amino acid sequence SEQ ID NO:12** 
EVQLVESGGGLVQPGGSLRLSCAASGFTLSGDWIHWVRQAPGKGLEWVGEISAAGGYTD
YADSVKGRFTISADTSKNTAYLQMNSLRAEDTAVYYCARESRVSFEAAMDYWGQGTLVTVSS
ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPEAAGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHN
 HYTQKSLSLSPGK
**EGFR/HER3 bispecific antibody light chain constant region amino acid sequence SEQ ID NO: 13 (LLC160-LLC166)** 
GQPKAAPSVTLFPPSSEELQANKATLVCLISDFYPGAVTVAWKADSSPVKAGVCTTTPSCQ
SNNKYAASSYLSLTPEQWKSHRSYSCQVTHEGSTVEKTVAPTECS
**EGFR/HER3 bispecific antibody light chain amino acid sequence SEQ ID NO:14** 
DIQMTQSPSSLSASVGDRVTITCRASQNIATDVAWYQQKPGKAPKLLIYSASFLYSGVPSRF
SGSGSGTDFTLTISSLQPEDFATYYCQQSEPEPYTFGQGTKVEIKGQPKAAPSVTLFPPSSEELQA
NKATLVCLISDFYPGAVTVAWKADSSPVKAGVCTTTPSCQSNNKYAASSYLSLTPEQWKSHRS
YSCQVTHEGSTVEKTVAPTECS
**EGFR/HER3 bispecific antibody heavy chain constant region amino acid sequence SEQ ID NO:15 (HC118 and HC239 and LALA mutations)** 
CSTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPCVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMHEA
LHNHYTQKSLSLSPGK
**EGFR/HER3 bispecific antibody heavy chain amino acid sequence SEQ ID NO:16** 
EVQLVESGGGLVQPGGSLRLSCAASGFTLSGDWIHWVRQAPGKGLEWVGEISAAGGYTD
YADSVKGRFTISADTSKNTAYLQMNSLRAEDTAVYYCARESRVSFEAAMDYWGQGTLVTVSS
CSTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPEAAGGPCVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHN
HYTQKSLSLSPGK
**EGFR/HER3 bispecific antibody light chain constant region amino acid sequence SEQ ID NO:17(Kappa light chain constant region, no mutation)**
**EGFR/HER3 bispecific antibody light chain amino acid sequence SEQ ID NO:18** 
DIQMTQSPSSLSASVGDRVTITCRASQNIATDVAWYQQKPGKAPKLLIYSASFLYSGVPSRF
SGSGSGTDFTLTISSLQPEDFATYYCQQSEPEPYTFGQGTKVEIKRTVAAPSVFIFPPSDEQLKSGT
ASVVCLLNNFYPREAKVQWKVDNALQSGNSQESVTEQDSKDSTYSLSSTLTLSKADYEKHKV
YACEVTHQGLSSPVTKSFNRGEC
**EGFR/HER3 bispecific antibody heavy chain constant region amino acid sequence SEQ ID NO:19 (HC118 mutation, LALA mutation)** 
CSTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMHEA
LHNHYTQKSLSLSPGK
**EGFR/HER3 bispecific antibody heavy chain amino acid sequence SEQ ID NO:20** 
EVQLVESGGGLVQPGGSLRLSCAASGFTLSGDWIHWVRQAPGKGLEWVGEISAAGGYTD
YADSVKGRFTISADTSKNTAYLQMNSLRAEDTAVYYCARESRVSFEAAMDYWGQGTLVTVSS
CSTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPEAAGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHN
HYTQKSLSLSPGK
**EGFR/HER3 bispecific antibody light chain constant region amino acid sequence SEQ ID NO:21 (LLC160)** 
GQPKAAPSVTLFPPSSEELQANKATLVCLISDFYPGAVTVAWKADSSPVKAGVCTTTPSKQ
SNNKYAASSYLSLTPEQWKSHRSYSCQVTHEGSTVEKTVAPTECS
**EGFR/HER3 bispecific antibody light chain amino acid sequence SEQ ID NO:22** DIQMTQSPSSLSASVGDRVTITCRASQNIATDVAWYQQKPGKAPKLLIYSASFLYSGVPSRF 
SGSGSGTDFTLTISSLQPEDFATYYCQQSEPEPYTFGQGTKVEIKGQPKAAPSVTLFPPSSEELQA
NKATLVCLISDFYPGAVTVAWKADSSPVKAGVCTTTPSKQSNNKYAASSYLSLTPEQWKSHRS
YSCQVTHEGSTVEKTVAPTECS
**EGFR/HER3 bispecific antibody heavy chain constant region amino acid sequence SEQ ID NO: 23(HC239, LALA mutation)** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPEAAGGPCVFLFPPKPKDTLMISRTPEVTCV
VVDVSHEDPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK
**EGFR/HER3 bispecific antibody heavy chain amino acid sequence SEQ ID NO:24** 
EVQLVESGGGLVQPGGSLRLSCAASGFTLSGDWIHWVRQAPGKGLEWVGEISAAGGYTD
YADSVKGRFTISADTSKNTAYLQMNSLRAEDTAVYYCARESRVSFEAAMDYWGQGTLVTVSS
ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPEAAGGPCVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHN
HYTQKSLSLSPGK
**EGFR/HER3 bispecific antibody light chain constant region amino acid sequence SEQ ID NO:25 (LLC166 mutation)** 
GQPKAAPSVTLFPPSSEELQANKATLVCLISDFYPGAVTVAWKADSSPVKAGVETTTPSCQ
SNNKYAASSYLSLTPEQWKSHRSYSCQVTHEGSTVEKTVAPTECS
**EGFR/HER3 bispecific antibody light chain amino acid sequence SEQ ID NO:26** 
DIQMTQSPSSLSASVGDRVTITCRASQNIATDVAWYQQKPGKAPKLLIYSASFLYSGVPSRF
SGSGSGTDFTLTISSLQPEDFATYYCQQSEPEPYTFGQGTKVEIKGQPKAAPSVTLFPPSSEELQA
NKATLVCLISDFYPGAVTVAWKADSSPVKAGVETTTPSCQSNNKYAASSYLSLTPEQWKSHRSY
SCQVTHEGSTVEKTVAPTECS
**SIB001 antibody heavy chain amino acid sequence SEQ ID NO:27** QVQLKQSGPGLVQPSQSLSITCTVSGFSLTNYGVHWVRQSPGKGLEWLGVIWSGGNTDY 
NTPFTSRLSINKDNSKSQVFFKMNSLQSNDTAIYYCARALTYYDYEFAYWGQGTLVTVSSAST
KGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYT
QKSLSLSPGGGGGSGGGGSQVQLQESGGGLVKPGGSLRLSCAASGFTFSSYWMSWVRQAPGK
GLEWVANINRDGSASYYVDSVKGRFTISRDDAKNSLYLQMNSLRAEDTAVYYCARDRGVGYF
DLWGRGTLVTVSSGGGGSGGGGSGGGGSQSALTQPASVSGSPGQSITISCTGTSSDVGGYNFVS
WYQQHPGKAPKLMIYDVSDRPSGVSDRFSGSKSGNTASLIISGLQADDEADYYCSSYGSSSTH
VIFGGGTKVTVL
**SIB001 antibody light chain amino acid sequence SEQ ID NO:28** 
DILLTQSPVILSVSPGERVSFSCRASQSIGTNIHWYQQRTNGSPRLLIKYASESISGIPSRFSGS
GSGTDFTLSINSVESEDIADYYCQQNNNWPTTFGAGTKLELKRTVAAPSVFIFPPSDEQLKSGTA
SVVCLLNNFYPREAKVQWKVDNALQSGNSQESVTEQDSKDSTYSLSSTLTLSKADYEKHKVY
ACEVTHQGLSSPVTKSFNRGEC
**Duligotuzumab antibody heavy chain amino acid sequence SEQ ID NO:29** 
EVQLVESGGGLVQPGGSLRLSCAASGFTLSGDWIHWVRQAPGKGLEWVGEISAAGGYTD
YADSVKGRFTISADTSKNTAYLQMNSLRAEDTAVYYCARESRVSFEAAMDYWGQGTLVTVSS
ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALHNH
YTQKSLSLSPGK
**Duligotuzumab antibody light chain amino acid sequence SEQ ID NO:30** 
DIQMTQSPSSLSASVGDRVTITCRASQNIATDVAWYQQKPGKAPKLLIYSASFLYSGVPSRF
SGSGSGTDFTLTISSLQPEDFATYYCQQSEPEPYTFGQGTKVEIKRTVAAPSVFIFPPSDEQLKSGT
ASVVCLLNNFYPREAKVQWKVDNALQSGNSQESVTEQDSKDSTYSLSSTLTLSKADYEKHKV
YACEVTHQGLSSPVTKSFNRGEC
**IgG1LALA antibody heavy chain amino acid sequence SEQ ID NO:31** 
QVQLVQSGAEVKKPGASVKVSCQASGYRFSNFVIHWVRQAPGQRFEWMGWINPYNGNK
EFSAKFQDRVTFTADTSANTAYMELRSLRSADTAVYYCARVGPYSWDDSPQDNYYMDVWGK
GTTVIVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEAAGGP
SVFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPG
**IgG1LALA antibody light chain amino acid sequence SEQ ID NO:32** 
EIVLTQSPGTLSLSPGERATFSCRSSHSIRSRRVAWYQHKPGQAPRLVIHGVSNRASGISDRF
SGSGSGTDFTLTITRVEPEDFALYYCQVYGASSYTFGQGTKLERKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC
**Patritumab antibody heavy chain amino acid sequence SEQ ID NO:33** 
QVQLQQWGAGLLKPSETLSLTCAVYGGSFSGYYWSWIRQPPGKGLEWIGEINHSGSTNY
NPSLKSRVTISVETSKNQFSLKLSSVTAADTAVYYCARDKWTWYFDLWGRGTLVTVSSASTKG
PSVFPLAPSSKSTSGGTAALGCLVKDYFPEPVTVSWNSGALTSGVHTFPAVLQSSGLYSLSSVVT
VPSSSLGTQTYICNVNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYT
QKSLSLSPGK
**Patritumab antibody light chain amino acid sequence SEQ ID NO:34** 
DIEMTQSPDSLAVSLGERATINCRSSQSVLYSSSNRNYLAWYQQNPGQPPKLLIYWASTRE
SGVPDRFSGSGSGTDFTLTISSLQAEDVAVYYCQQYYSTPRTFGQGTKVEIKRTVAAPSVFIFPPS
DEQLKSGTASVVCLLNNFYPREAKVQWKVDNALQSGNSQESVTEQDSKDSTYSLSSTLTLSKA
DYEKHKVYACEVTHQGLSSPVTKSFNRGEC
**Human IgG1 heavy chain constant region amino acid sequence SEQ ID NO:35** ASTKGPSVFPLAPSSKSTSGGTAALGCLVKDYFPEPVTVSWNSGALTSGVHTFPAVLQSSG 
LYSLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMHEA
LHNHYTQKSLSLSPGK
**Human lambda light chain constant region amino acid sequence SEQ ID NO:36** 
GQPKAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS
**Cetuximab antibody heavy chain amino acid sequence SEQ ID NO:38** 
QVQLKQSGPGLVQPSQSLSITCTVSGFSLTNYGVHWVRQSPGKGLEWLGVIWSGGNTDY
NTPFTSRLSINKDNSKSQVFFKMNSLQSNDTAIYYCARALTYYDYEFAYWGQGTLVTVSAAST
KGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYT
QKSLSLSPGK
**Cetuximab antibody light chain amino acid sequence SEQ ID NO:39**

DILLTQSPVILSVSPGERVSFSCRASQSIGTNIHWYQQRTNGSPRLLIKYASESISGIPSRFSGS
GSGTDFTLSINSVESEDIADYYCQQNNNWPTTFGAGTKLELKRTVAAPSVFIFPPSDEQLKSGTA
SVVCLLNNFYPREAKVQWKVDNALQSGNSQESVTEQDSKDSTYSLSSTLTLSKADYEKHKVY
ACEVTHQGLSSPVTKSFNRGEC

## Claims

1. An anti-EGFR/HER3 bispecific antibody or an antigen-binding fragment thereof, comprising
a heavy chain variable region and a light chain variable region, the heavy chain variable region comprises HCDR1, HCDR2 and HCDR3 consisting of the amino acid sequences set forth in SEQ ID NO:1, SEQ ID NO:2 and SEQ ID NO:3 respectively, and the light chain variable region comprises LCDR1, LCDR2 and LCDR3 consisting of the amino acid sequences set forth in SEQ ID NO:4, SEQ ID NO:5 and SEQ ID NO:6 respectively;
a heavy chain constant region and a light chain constant region, wherein the anti-EGFR/HER3 bispecific antibody comprises one or more mutations to cysteine, for example one or more mutations selected from: the mutation to cysteine at position 118 of the heavy chain constant region, the mutation to cysteine at position 239 of the heavy chain constant region, the mutation to cysteine at position 160 of the light chain constant region, and the mutation to cysteine at position 166 of the light chain constant region; wherein the amino acid residues are numbered according to EU numbering system.

2. The anti-EGFR/HER3 bispecific antibody or the antigen-binding fragment thereof according to claim 1, wherein the heavy chain variable region
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:7; or
(ii) comprises or consists of an amino acid sequence set forth in SEQ ID NO:7; or
(iii) comprises or consists of an amino acid sequence having one or more amino acid substitutions, insertions or deletions compared to the amino acid sequence set forth in SEQ ID NO:7, optionally the amino acid substitutions, insertions or deletions do not occur in the CDRs.

3. The anti-EGFR/HER3 bispecific antibody or the antigen-binding fragment thereof according to claim 1 or 2, wherein the light chain variable region
(i) comprises or consists of an amino acid sequence having at least 90%, 91%, 92%, 93%, 94%, 95%, 96%, 97%, 98% or 99% identity to the amino acid sequence set forth in SEQ ID NO: 9; or
(ii) comprises or consists of an amino acid sequence set forth in SEQ ID NO: 9; or
(iii) comprises or consists of an amino acid sequence having one or more amino acid substitutions, insertions or deletions compared to the amino acid sequence set forth in SEQ ID NO: 9, optionally the amino acid substitutions, insertions or deletions do not occur in the CDRs.

4. The anti-EGFR/HER3 bispecific antibody or the antigen-binding fragment thereof according to any one of claims 1-3, wherein the anti-EGFR/HER3 bispecific antibody comprises any two mutations selected from: the mutation to cysteine at position 118 of the heavy chain constant region, the mutation to cysteine at position 239 of the heavy chain constant region, the mutation to cysteine at position 160 of the light chain constant region, and the mutation to cysteine at position 166 of the light chain constant region.

5. The anti-EGFR/HER3 bispecific antibody or the antigen-binding fragment thereof according to any one of claims 1-4, wherein the light chain constant region is a lambda light chain constant region or a kappa light chain constant region.

6. The anti-EGFR/HER3 bispecific antibody or the antigen-binding fragment thereof according to any one of claims 1-5, wherein the anti-EGFR/HER3 bispecific antibody further comprises the mutations to alanine at positions 234 and 235 of the heavy chain constant region.

7. The anti-EGFR/HER3 bispecific antibody or the antigen-binding fragment thereof according to any one of claims 1-6, wherein the heavy chain constant region is a heavy chain constant region of human IgG1.

8. The anti-EGFR/HER3 bispecific antibody or the antigen-binding fragment thereof according to any one of claims 1-7, wherein the light chain constant region is a human lambda light chain constant region or a human kappa light chain constant region.

9. The anti-EGFR/HER3 bispecific antibody or the antigen-binding fragment thereof according to any one of claims 1-8, wherein the mutations are each independently relative to a native heavy chain constant region of the human IgG1 immunoglobulin or a native light chain constant region.

10. The anti-EGFR/HER3 bispecific antibody or the antigen-binding fragment thereof according to any one of claims 1-9, wherein the anti-EGFR/HER3 bispecific antibody comprising anyone of the following combinations of mutations to cysteine : (1) the mutation to cysteine at position 160 of the lambda light chain constant region, and the mutation to cysteine at position 166 of the lambda light chain constant region; (2) the mutation to cysteine at position 118 of the heavy chain constant region, and the mutation to cysteine at position 239 of the heavy chain constant region; (3) the mutation to cysteine at position 160 of the lambda light chain constant region, and the mutation to cysteine at position 118 of the heavy chain constant region; (4) the mutation to cysteine at position 166 of the lambda light chain constant region, and the mutation to cysteine at position 118 of the heavy chain constant region; (5) the mutation to cysteine at position 160 of the lambda light chain constant region, and the mutation to cysteine at position 239 of the heavy chain constant region; and (6) the mutation to cysteine at position 166 of the lambda light chain constant region and the mutation to cysteine at position 239 of the heavy chain constant region, and the bispecific antibody optionally further comprises a LALA mutation in the heavy chain constant region.

11. The anti-EGFR/HER3 bispecific antibody or the antigen-binding fragment thereof according to any one of claims 1-10, wherein the heavy chain constant region of the anti-EGFR/HER3 bispecific antibody
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:11, 15, 19 and 23; or
(ii) comprises or consists of an amino acid sequence set forth in any one of SEQ ID NO:11, 15, 19 and 23; or
(iii) comprises or consists of an amino acid sequence having one or more amino acid substitutions, insertions or deletions compared to the amino acid sequence set forth in any one of SEQ ID NO:11, 15, 19 and 23.

12. The anti-EGFR/HER3 bispecific antibody or the antigen-binding fragment thereof according to any one of claims 1-11, wherein the light chain constant region of the anti-EGFR/HER3 bispecific antibody
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:13, 17, 21 and 25; or
(ii) comprises or consists of an amino acid sequence set forth in any one of SEQ ID NO:13, 17, 21 and 25; or
(iii) comprises or consists of an amino acid sequence having one or more amino acid substitutions, insertions or deletions compared to the amino acid sequence set forth in any one of SEQ ID NO:13, 17, 21 and 25.

13. The anti-EGFR/HER3 bispecific antibody or the antigen-binding fragment thereof according to any one of claims 1-12, wherein the anti-EGFR/HER3 bispecific antibody comprises a heavy chain and a light chain, wherein
the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:12, 16, 20 and 24; or
(ii) comprises or consists of an amino acid sequence set forth in any one of SEQ ID NO:12, 16, 20 and 24; or
(iii) comprises or consists of an amino acid sequence having one or more amino acid substitutions, insertions or deletions compared to the amino acid sequence set forth in any one of SEQ ID NO:12, 16, 20 and 24; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in any one of SEQ ID NO:14, 18, 22 and 26; or
(ii) comprises or consists of the amino acid sequence set forth in any one of SEQ ID NO:14, 18, 22 and 26; or
(iii) comprises or consists of an amino acid sequence having one or more amino acid substitutions, insertions or deletions compared to the amino acid sequence set forth in any one of SEQ ID NO:14, 18, 22 and 26.

14. The anti-EGFR/HER3 bispecific antibody or the antigen-binding fragment thereof according to any one of claims 1-13, wherein the anti-EGFR/HER3 bispecific antibody comprises a heavy chain and a light chain, wherein
(I) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:12; or
(ii) comprises or consists of the amino acid sequence set forth in SEQ ID NO:12; or
(iii) comprises or consists of an amino acid sequence having one or more amino acid substitutions, insertions or deletions compared to the amino acid sequence set forth in SEQ ID NO:12; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:14; or
(ii) comprises or consists of the amino acid sequence set forth in SEQ ID NO:14; or
(iii) comprises or consists of an amino acid sequence having one or more amino acid substitutions, insertions or deletions compared to the amino acid sequence set forth in SEQ ID NO:14;
(II) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:16; or
(ii) comprises or consists of the amino acid sequence set forth in SEQ ID NO:16; or
(iii) comprises or consists of an amino acid sequence having one or more amino acid substitutions, insertions or deletions compared to the amino acid sequence set forth in SEQ ID NO:16; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:18; or
(ii) comprises or consists of the amino acid sequence set forth in SEQ ID NO:18; or
(iii) comprises or consists of an amino acid sequence having one or more amino acid substitutions, insertions or deletions compared to the amino acid sequence set forth in SEQ ID No: 18;
(III) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:20; or
(ii) comprises or consists of the amino acid sequence set forth in SEQ ID NO:20; or
(iii) comprises or consists of an amino acid sequence having one or more amino acid substitutions, insertions or deletions compared to the amino acid sequence set forth in SEQ ID NO:20; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:22; or
(ii) comprises or consists of the amino acid sequence set forth in SEQ ID NO:22; or
(iii) comprises or consists of an amino acid sequence having one or more amino acid substitutions, insertions or deletions compared to the amino acid sequence set forth in SEQ ID NO:22;
(IV) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:20; or
(ii) comprises or consists of the amino acid sequence set forth in SEQ ID NO:20; or
(iii) comprises or consists of an amino acid sequence having one or more amino acid substitutions, insertions or deletions compared to the amino acid sequence set forth in SEQ ID NO:20; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:26; or
(ii) comprises or consists of the amino acid sequence set forth in SEQ ID NO:26; or
(iii) comprises or consists of an amino acid sequence having one or more amino acid substitutions, insertions or deletions compared to the amino acid sequence set forth in SEQ ID NO:26;
(V) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:24; or
(ii) comprises or consists of the amino acid sequence set forth in SEQ ID NO:24; or
(iii) comprises or consists of an amino acid sequence having one or more amino acid substitutions, insertions or deletions compared to the amino acid sequence set forth in SEQ ID NO:24; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:22; or
(ii) comprises or consists of the amino acid sequence set forth in SEQ ID NO:22; or
(iii) comprises or consists of an amino acid sequence having one or more amino acid substitutions, insertions or deletions compared to the amino acid sequence set forth in SEQ ID NO:22;
or
(VI) the heavy chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:24; or
(ii) comprises or consists of the amino acid sequence set forth in SEQ ID NO:24; or
(iii) comprises or consists of an amino acid sequence having one or more amino acid substitutions, insertions or deletions compared to the amino acid sequence set forth in SEQ ID NO:24; and
the light chain
(i) comprises or consists of an amino acid sequence having at least 85%, 86%, 87%, 88%, 89%, 90%, 91%, 92%, 93%, 94%, 95%, 96%, 97%, 98% or 99% identity to the amino acid sequence set forth in SEQ ID NO:26; or
(ii) comprises or consists of the amino acid sequence set forth in SEQ ID NO:26; or
(iii) comprises or consists of an amino acid sequence having one or more amino acid substitutions, insertions or deletions compared to the amino acid sequence set forth in SEQ ID NO:26.

15. An immunoconjugate comprising the anti-EGFR/HER3 bispecific antibody or the antigen-binding fragment thereof according to any one of claims 1-14.

16. A bispecific antibody-drug conjugate of formula (I), a stereoisomer or a pharmaceutically acceptable salt or solvate thereof:
Ab-(L-D)ₙ (I)
wherein,
Ab is the anti-EGFR/HER3 bispecific antibody or the antigen-binding fragment thereof according to any one of claims 1-14,
L is a linker,
D is a cytotoxic compound,
n represents a conjugation number, and n is a natural number selected from 1 to 15;
preferably, the L is conjugated to a mutated cysteine in the Ab.

17. The bispecific antibody-drug conjugate, the stereoisomer or pharmaceutically acceptable salt or solvate thereof according to claim 16, wherein the linker L has the structure of formula (II):
Q-L' (II),
Q represents a linking moiety conjugated to the Ab through a thioether linkage (-S-);
L' represents a linking moiety linking Q to the cytotoxic compound D, having the following structure: 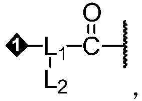
wherein L₁ is a polypeptide residue consisting of 3 to 8 amino acid residues, optionally the polypeptide residue includes at least one amino acid residue with a side chain carboxylic acid, "-C(=O)-" represents the carbonyl group of the C-terminal amino acid residue of the polypeptide residue;
L₂ is absent or is a hydrophilic group linked to the carbonyl group obtained after reacting with the carboxylic acid of the amino acid residue side chain of the polypeptide residue L₁, and L₂ is -NHR_{L2}, R_{L2} is selected from C₁₋₆ alkyl optionally substituted with 1 to 6 hydroxyl groups;
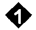 represents the N-terminus of the polypeptide residue covalently linked to the linking moiety Q.

18. The bispecific antibody-drug conjugate, the stereoisomer or pharmaceutically acceptable salt or solvate thereof according to claim 17, wherein the linking moiety Q has the following structure: 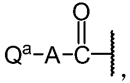
wherein Q^{a} is a functional group conjugated to the Ab;
A is selected from optionally substituted aliphatic hydrocarbylene or optionally substituted aliphatic heterohydrocarbylene, wherein the aliphatic hydrocarbylene and aliphatic heterohydrocarbylene are optionally substituted with 1 to 4 substituents independently selected from the group consisting of halogen, -CN, -OR_{Qa1}, -SR_{Qa1}, -N(R_{Qa1})₂, wherein each R_{Qa1} is independently selected from hydrogen, C₁₋₆ alkyl, C₁₋₆ haloalkyl; and
" 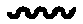 " represents a site covalently linked to the linking moiety L.

19. The bispecific antibody-drug conjugate, the stereoisomer or pharmaceutically acceptable salt or solvate thereof according to claim 18, wherein the functional group Q^{a} is absent or selected from maleimide, iodoacetamide, bromoacetamide, pyrimidine, pyrimidyl sulfide, vinyl pyrimidine, vinyl triazine, vinyl pyridine, disulfide, pyridyl disulfide, haloacetamide, α-haloacetyl, reactive ester, preferably maleimide of the following structure: 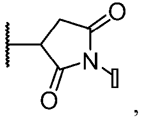
"*" represents a site covalently linked to A;
" 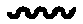 " represents a site covalently linked to the antibody Ab.

20. The bispecific antibody-drug conjugate, the stereoisomer or pharmaceutically acceptable salt or solvate thereof according to any one of claims 17-19, wherein the linking moiety Q has the following structure:

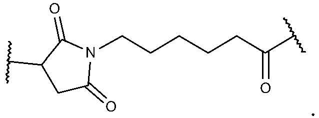

21. The bispecific antibody-drug conjugate, the stereoisomer or pharmaceutically acceptable salt or solvate thereof according to any one of claims 17-20, wherein the linking moiety L₁ has the following structure:
^{NH}-AA¹AA²AA³...AA^{p}-^{C(=O)},
wherein each of AA¹, AA², AA³, ...... AA^{p} is independently an optionally substituted amino acid residue, optionally at least one of AA¹, AA², AA³, ...... AA^{P} is an amino acid residue with a side chain carboxylic acid, e.g. Glu or Asp;
p is an integer from 3 to 8, e.g. from 3 to 5;
"NH-" represents the N-terminus of the polypeptide residue;
"-C(=O)" represents the C-terminus of the polypeptide residue.

22. The bispecific antibody-drug conjugate, the stereoisomer or pharmaceutically acceptable salt or solvate thereof according to claim 21, wherein each of AA¹, AA², AA³, ...... AA^{p} is independently an optionally substituted amino acid residue selected from the group consisting of Glu, Asp, Pro, Nva, Leu, Ile, Met, Tyr, Trp, Ser, Thr, Cys, Asn, Gln, Arg, Phe, Lys, Val, Ala, Cit, Gly, and N-alkyl amino acids, and at least one of AA¹, AA², AA³, ...... AA^{p} is Glu or Asp.

23. The bispecific antibody-drug conjugate, the stereoisomer or pharmaceutically acceptable salt or solvate thereof according to claim 21 or 22, wherein the linking moiety L₁ is ^{NH}-Glu-Val-Ala-^{C(=O)}.

24. The bispecific antibody-drug conjugate, the stereoisomer or pharmaceutically acceptable salt or solvate thereof according to any one of claims 17-23, wherein L₂ has the following structure:

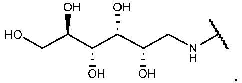

25. The bispecific antibody-drug conjugate, the stereoisomer or pharmaceutically acceptable salt or solvate thereof according to any one of claims 17-24, wherein the linking moiety L' has the following structure:

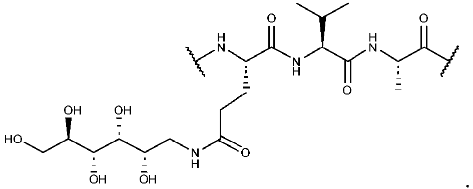

26. The bispecific antibody-drug conjugate, the stereoisomer or pharmaceutically acceptable salt or solvate thereof according to any one of claims 17-25, wherein the linker L has the following structure:

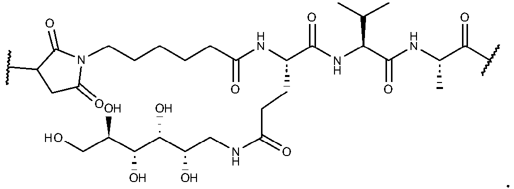

27. The bispecific antibody-drug conjugate, the stereoisomer or pharmaceutically acceptable salt or solvate thereof according to any one of claims 16-26, wherein the linker L is linked to the Ab through a side chain of cysteine residue.

28. The bispecific antibody-drug conjugate, the stereoisomer or pharmaceutically acceptable salt or solvate thereof according to any one of claims 16-27, wherein the cytotoxic compound D has the structure of formula (III):
Z-D' (III)
Z is absent or selected from -NH- or -NH-R_{z1}-Y-R_{z2}-, wherein R_{z1} and R_{z2} are each independently absent or selected from optionally substituted C₁₋₈ aliphatic hydrocarbylene, optionally substituted C₀₋₈ aliphatic hydrocarbylene-arylene, optionally substituted C₀₋₈ aliphatic hydrocarbylene-carbonyl (-C(O)-), optionally substituted C₀₋₈ aliphatic hydrocarbylene-oxycarbonyl (-C(O)O- or -OC(O)-), optionally substituted C₀₋₈ aliphatic hydrocarbylene-carbonyl-imino, optionally substituted arylene-carbonyl (-C(O)-), optionally substituted arylene-oxycarbonyl (-C(O)O- or -OC(O)-), optionally substituted arylene-carbonyl-imino, wherein optionally substituted refers to optionally substituted with 1 to 4 substituents independently selected from the group consisting of C₁₋₈ aliphatic hydrocarbyl, halogen, - CN, -OR_{z3}, -SR_{z3}, -N(R_{z3})₂, wherein each R_{z3} is independently selected from hydrogen, C₁₋₆ aliphatic hydrocarbyl, C₁₋₆ haloaliphatic hydrocarbyl, Y is absent or selected from -O- or -S-;
D' is a camptothecin compound, or an analogue or derivative thereof.

29. The bispecific antibody-drug conjugate, the stereoisomer or pharmaceutically acceptable salt or solvate thereof according to claim 28, wherein D' is the camptothecin compound, or the analogue or derivative thereof, having the following structure: 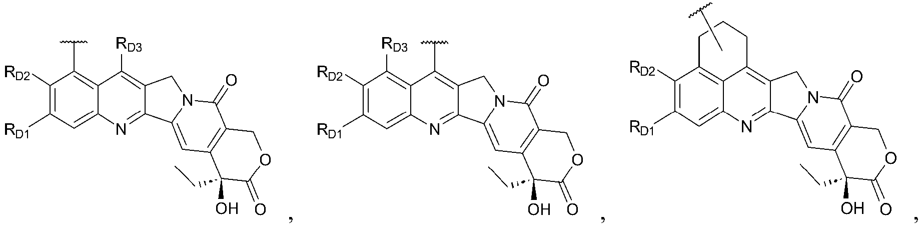 or 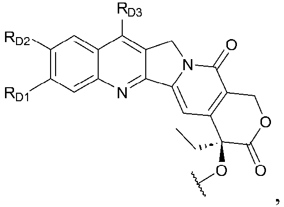 wherein R_{D1}, R_{D2} and R_{D3} are each independently selected from the group consisting of H, hydroxy, cyano, halogen, C₁₋₆ alkyl, C₁₋₆ alkoxy, C₁₋₆ alkylthio, and C₁₋₆ haloalkyl, or R_{D1} and R_{D2} together with the carbon atom to which they are attached form a 5-8 membered cyclic or heterocyclic group, wherein optionally substituted refers to optionally substituted with one or more substituents independently selected from the group consisting of C₁₋₈ aliphatic hydrocarbyl, halogen, -CN, -OR_{D'}, - SR_{D'}, -S(O)R_{D'} and -S(O)₂R_{D'}, and -N(R_{D'})₂, wherein each R_{D'} is independently selected from hydrogen, C₁₋₆ aliphatic hydrocarbyl, C₁₋₆ haloaliphatic hydrocarbyl, -SR_{D"}, -S(O)R_{D"} and -S(O)₂R_{D"}, each R_{D"} is independently selected from hydrogen, C₁₋₆ aliphatic hydrocarbyl, C₁₋₆ haloaliphatic hydrocarbyl.

30. The bispecific antibody-drug conjugate, the stereoisomer or pharmaceutically acceptable salt or solvate thereof according to any one of claims 16-29, wherein the cytotoxic compound D is selected from the following compounds:

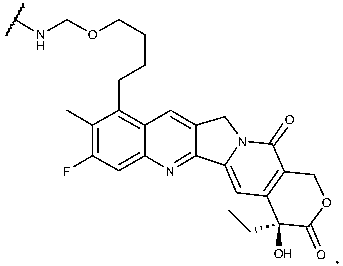

31. The bispecific antibody-drug conjugate, the stereoisomer or pharmaceutically acceptable salt or solvate thereof according to claim 16, wherein the antibody-drug conjugate has the following structure: 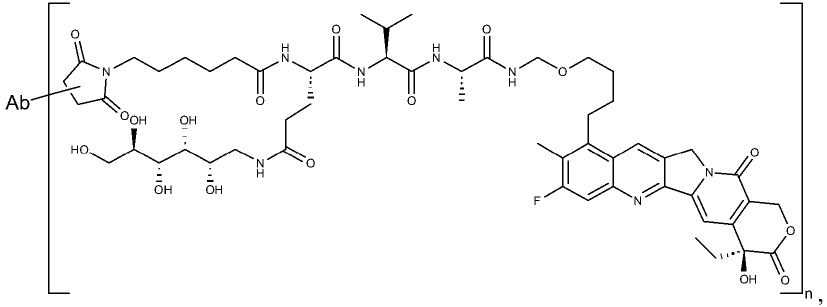 wherein Ab and n are as defined in claim 16.

32. The bispecific antibody-drug conjugate, the stereoisomer or pharmaceutically acceptable salt or solvate thereof according to any one of claims 16-31, having an average DAR of 1 to 10, e.g. 3 to 5 or 7 to 9.

33. A pharmaceutical composition comprising the bispecific antibody-drug conjugate, the stereoisomer or pharmaceutically acceptable salt or solvate thereof according to any one of claims 16-32, and a pharmaceutically acceptable carrier or excipient.

34. Use of the bispecific antibody-drug conjugate, the stereoisomer or pharmaceutically acceptable salt or solvate thereof according to any one of claims 16-32 in the manufacture of a medicament for the treatment or prevention of a disease or condition associated with EGFR and/or HER3 activity.

35. The use according to claim 34, wherein the disease or condition associated with EGFR and/or HER3 activity is an EGFR and/or HER3 positive tumor.

36. The use according to claim 35, wherein the EGFR and/or HER3 positive tumor is selected from the group consisting of lymphoma, blastoma, sarcoma, leukemia, melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer.

37. The bispecific antibody-drug conjugate, the stereoisomer or pharmaceutically acceptable salt or solvate thereof according to any one of claims 16-32, for use in the treatment or prevention of a disease or condition associated with EGFR and/or HER3 activity.

38. The bispecific antibody-drug conjugate, the stereoisomer or pharmaceutically acceptable salt or solvate thereof for use in the treatment or prevention of a disease or condition associated with EGFR and/or HER3 activity according to claim 37, wherein the disease or condition associated with EGFR and/or HER3 activity is an EGFR and/or HER3 positive tumor.

39. The bispecific antibody-drug conjugate, the stereoisomer or pharmaceutically acceptable salt or solvate thereof for use in the treatment or prevention of a disease or condition associated with EGFR and/or HER3 activity, according to claim 38, wherein the EGFR and/or HER3 positive tumor is selected from the group consisting of lymphoma, blastoma, sarcoma, leukemia, melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer.

40. A method of treating or preventing a disease or condition associated with EGFR and/or HER3 activity, comprising administering to a subject an effective amount of the bispecific antibody-drug conjugate, the stereoisomer or pharmaceutically acceptable salt or solvate thereof according to any one of claims 16-32.

41. The method according to claim 40, wherein the disease or condition associated with EGFR and/or HER3 activity is an EGFR and/or HER3 positive tumor.

42. The method according to claim 41, wherein the EGFR and/or HER3 positive tumor is selected from the group consisting of lymphoma, blastoma, sarcoma, leukemia, melanoma, squamous cell carcinoma, lung cancer, small cell lung cancer, non-small cell lung cancer, lung adenocarcinoma, squamous cell lung cancer, peritoneal cancer, hepatocellular cancer, gastrointestinal cancer, pancreatic cancer, glioblastoma, cervical cancer, ovarian cancer, liver cancer, bladder cancer, breast cancer, colon cancer, colorectal cancer, endometrial carcinoma or uterine carcinoma, salivary gland carcinoma, kidney cancer, prostate cancer, vulval cancer, thyroid cancer, gastric cancer, head and neck squamous carcinoma, as well as various types of head and neck cancer.

43. The method according to any one of claims 40-42, wherein the method further comprises coadministering one or more therapeutic agents selected from the group consisting of chemotherapeutic agents, angiogenesis inhibitors, cytokines, cytotoxic agents, other antibodies, small molecule drugs, immunomodulators and targeted degraders.

44. The method according to claim 43, wherein the antibody-drug conjugate, the stereoisomer or pharmaceutically acceptable salt or solvate thereof is administered simultaneously or sequentially in any order with one or more therapeutic agents.

45. The method according to claims 40-44, wherein the method further comprises administering to the patient one or more treatment modalities selected from the group consisting of radiation therapy, cell therapy, gene therapy, RNA therapy and surgery, etc.
